# EUROPÄISCHE PATENTSCHRIFT

(11) **EP 3 956 281 B1**
(45) Veröffentlichungstag und Bekanntmachung des Hinweises auf die Patenterteilung: **07.01.2026**
(21) Anmeldenummer: 19731647.4
(22) Anmeldetag: 12.06.2019
(51) Int. Cl.: C07C 67/03, C07C 69/72, A61P 3/00, A61K 31/22

(54) **VERFAHREN ZUR HERSTELLUNG VON POLYOLBASIERTEN ESTERN VON KETOCARBONSÄUREN**
PROCESS FOR THE PREPARATION OF POLYOL-BASED KETOCARBOXYLIC ESTERS
PROCÉDÉ DE PRÉPARATION D'ESTERS CÉTOCARBOXYLIQUE À BASE DE POLYOL

(43) Veröffentlichungstag der Anmeldung: 23.02.2022
(73) Patentinhaber: KetoLipix Therapeutics GmbH, 20459 Hamburg (DE)
(72) Erfinder: LOCHMANN, Dirk, 58453 Witten (DE); REYER, Sebastian, 58453 Witten (DE); STEHR, Michael, 58453 Witten (DE)
(74) Vertreter: Strehlke, Ingo Kurt
(86) Internationale Anmeldenummer: PCT/EP2019/065268
(87) Internationale Veröffentlichungsnummer: WO 2020/249195

(56) Entgegenhaltungen:
- EP-A1- 0 022 978
- WO-A1-2008/005818
- WO-A1-90/02549
- WO-A1-95/09146
- GB-A- 2 046 091
- JP-A- 2007 277 441
- US-A- 3 907 994
- US-A1- 2003 064 954
- US-A1- 2009 137 710
- US-A1- 2009 253 781
- MICHAEL J. HAAS ET AL: "Lipase-catalyzed synthesis of partial acylglycerols of acetoacetate", EUROPEAN JOURNAL OF LIPID SCIENCE AND TECHNOLOGY., vol. 113, no. 2, 1 February 2011 (2011-02-01), DE, pages 168 - 179, XP055655581, ISSN: 1438-7697, DOI: 10.1002/ejlt.201000477
- SANJIB KUMAR KARMEE ET AL: "Rapid and Simple Method of Monoacylation of Polyols by [beta]-Ketoesters Using Microwave Irradiation", SYNTHETIC COMMUNICATIONS, vol. 35, no. 9, 1 May 2005 (2005-05-01), PHILADELPHIA, PA; US, pages 1151 - 1160, XP055655592, ISSN: 0039-7911, DOI: 10.1081/SCC-200054750

## Beschreibung

Die vorliegende Erfindung betrifft das Gebiet der Ketokörper und des damit zusammenhängenden Stoffwechsels sowie die Therapie von damit im Zusammenhang stehenden Erkrankungen.

Insbesondere betrifft die vorliegende Erfindung ein Verfahren zur Herstellung von Polyglycerinestern der 3-Oxobuttersäure (synonym auch als "3-Oxobutansäure", "beta-Oxobuttersäure", "beta-Oxobutansäure" etc. bezeichnet) sowie die auf diese Weise erhältlichen bzw. hergestellten Reaktionsprodukte (d. h. Polyglycerinester der 3-Oxobuttersäure) und deren funktionalisierte Derivate.

Des Weiteren betrifft die vorliegende Erfindung pharmazeutische Zusammensetzungen, insbesondere Arzneimittel oder Medikamente, welche die nach dem erfindungsgemäßen Herstellungsverfahren erhältlichen bzw. hergestellten Reaktionsprodukte (d. h. Polyglycerinester der 3-Oxobuttersäure) bzw. deren funktionalisierte Derivate umfassen, sowie deren Anwendungen bzw. Verwendungen.

Schließlich betrifft die vorliegende Erfindung Nahrungsmittel- und/oder Lebensmittelerzeugnisse, insbesondere Nahrungsergänzungsmittel, funktionelle Lebensmittel *(Functional Food), Novel Food,* Lebensmittelzusatzstoffe, Nahrungszusätze, diätetische Lebensmittel, Power-Snacks, Appetitzügler und Kraft- und/oder Ausdauersport-Supplements, welche die nach dem erfindungsgemäßen Herstellungsverfahren erhältlichen bzw. hergestellten Reaktionsprodukte (d. h. Polyglycerinester der 3-Oxobuttersäure) bzw. deren funktionalisierte Derivate umfassen, sowie deren Anwendungen bzw. Verwendungen.

Im menschlichen Energiestoffwechsel ist Glucose der kurzfristig zur Verfügung stehende Energieträger, welcher in den Mitochondrien unter Freisetzung von Wasser und Kohlendioxid zu Energie verstoffwechselt wird. Bereits durch die Schlafperiode während der Nacht sind aber die Glykogenspeicher der Leber geleert. Jedoch benötigen vor allem das menschliche zentrale Nervensystem (ZNS) und das Herz eine permanente Energieversorgung.

Die physiologische Alternative zu Glucose, welche vor allem dem zentralen Nervensystem zur Verfügung steht, sind die sogenannten Ketokörper (synonym auch als "Ketonkörper" oder Englisch auch als *"Keton Bodies"* bezeichnet).

Der Begriff der Ketokörper ist insbesondere eine Sammelbezeichnung für drei Verbindungen, welche vor allem in katabolen Stoffwechsellagen (wie z. B. bei Hunger, Reduktionsdiät oder kohlenhydratarmer Ernährung) gebildet werden und unter Umständen zu einer Ketose führen. Unter dem Begriff der Ketokörper fasst man insbesondere die drei Verbindungen Acetoacetat (synonym auch Acetacetat oder 3-Oxobutyrat genannt) und Aceton sowie 3-Hydroxybuttersäure (nachfolgend synonym auch als beta-Hydroxybuttersäure oder BHB oder 3-BHB bezeichnet) bzw. deren Salz (d. h. 3-Hydroxybutyrat oder beta-Hydroxybutyrat) zusammen.

Diese Ketokörper werden auch in großer Zahl beim Fasten oder Hungern physiologisch aus im Körper eingelagerten Lipiden durch Lipolyse bereitgestellt und ersetzen den Energieträger Glucose fast vollständig.

Die Ketokörper werden in der Leber aus Acetyl-Coenzym A (= Acetyl-CoA) gebildet, welches aus der beta-Oxidation stammt; sie stellen eine transportable Form des Acetyl-Coenzyms A im menschliche Körper dar. Zur Verwertung der Ketokörper müssen sich Gehirn und Muskeln aber zunächst umstellen, indem sie Enzyme exprimieren, welche zur Rückwandlung von Ketokörpern in Acetyl-Coenzym A benötigt werden. Insbesondere in Hungerzeiten tragen die Ketokörper einen beträchtlichen Anteil zur Energiegewinnung bei. So ist es beispielsweise dem Gehirn nach einiger Zeit möglich, mit nur einem Drittel der Tagesmenge an Glucose auszukommen.

Physiologisch erfolgt die Synthese der Ketokörper aus zwei Molekülen aktivierter Essigsäure in Form von Acetyl-Coenzym A, dem normalen Zwischenprodukt des Fettsäureabbaus, wobei zunächst mit Hilfe der Acetyl-Coenzym A-Acetyltransferase das Acetoacetyl-Coenzym A gebildet wird, welches unter Verwendung einer weiteren Acetyl-Coenzym A-Einheit und des Enzyms HMG-CoA-Synthase zum Zwischenprodukt 3-Hydroxy-3-methyl-glutaryl-CoA (HMG-CoA) verlängert wird, wobei schließlich die HMG-CoA-Lyase das Acetoacetat abspaltet. Diese drei Schritte finden ausschließlich in den Mitochondrien der Leber statt (Lynenzyklus), wobei schließlich im Zytosol aus Acetoacetat durch das Enzym D-beta-Hydroxybutyrat-Dehydrogenase 3-Hydroxybutyrat entsteht. HMG-CoA ist außerdem ein Endprodukt beim Abbau der Aminosäure Leucin, während Acetoacetat beim Abbau der Aminosäuren Phenylalanin und Tyrosin entsteht.

Acetoacetat wird also unter physiologischen Bedingungen reduktiv in die physiologisch relevante Form der 3-Hydroxybuttersäure bzw. des 3-Hydroxybutyrats überführt.

3-Hydroxybuttersäure selbst wird derzeit im Bereich des Kraftsports als Natrium-, Magnesium- oder Calcium-Salz eingesetzt und in den Handel gebracht.

Jedoch ist 3-Hydroxybuttersäure selbst für den Menschen evolutionär nicht oder in nur sehr geringer Menge bekannt, da Pflanzen keine 3-Hydroxybuttersäure produzieren und 3-Hydroxybuttersäure im tierischen Organismus nur bei toten ausgezehrten Tieren in der Ketose vorkommt, so dass 3-Hydroxybuttersäure bei peroraler Verabreichung Brechreiz auslöst. 3-Hydroxybuttersäure in Form der freien Säure sowie deren Salzen schmeckt zudem stark bitter und kann schweres Erbrechen und Übelkeit hervorrufen.

Zudem können Patienten, vor allem Neugeborene, aber auch Erwachsene größere Mengen an Salzen der 3-Hydroxybuttersäure nicht permanent verkraften, da diese Verbindungen nierenschädigend wirken können.

Außerdem ist die Plasmahalbwertszeit von 3-Hydroxybuttersäure und deren Salzen derart gering, dass selbst bei Einnahme von mehreren Gramm die Ketose nur für ca. drei bis vier Stunden vorhält, d. h. Patienten können insbesondere während der Nacht daher nicht von einer Therapie mit 3-Hydroxybuttersäure oder deren Salzen kontinuierlich profitieren. Bei Stoffwechselerkrankungen kann dies zu lebensbedrohlichen Situationen führen.

Daher werden im Fall der Therapie derartiger Stoffwechselerkrankungen heute sogenannte mittelkettige Triglyceride, sogenannte MCTs, für die ketogene Therapie eingesetzt, d. h. es wird die metabolische Umwandlung von Capron-, Capryl- und Caprinsäure (d. h. von gesättigten linearen C₆-, C₈- und C₁₀-Fettsäuren) aus den korrespondierenden Triglyceriden beabsichtigt.

Grundsätzlich stellt aber aus pharmazeutischer und klinischer Hinsicht Acetoacetat als physiologischer Vorläufer der 3-Hydroxybuttersäure demgegenüber ein wirksameres pharmazeutisch-pharmakologisches Zielmolekül dar, welches nach den Erkenntnissen des Standes der Technik prinzipiell für die Therapie einer Vielzahl von Erkrankungen zum Einsatz kommen könnte (z. B. bei Erkrankungen im Zusammenhang mit einer Störung des Energiestoffwechsels, insbesondere Ketokörperstoffwechsels, oder neurodegenerativen Erkrankungen wie Demenz, Morbus Alzheimer, Morbus Parkinson etc., Fettstoffwechselerkrankungen usw.), aber aufgrund seiner mangelnden physiologischen Kompatibilität bzw. Verfügbarkeit dort nicht zum Einsatz kommen kann.

Die nachfolgende Tabelle veranschaulicht rein beispielhaft, aber keinesfalls beschränkend, potentielle Therapiemöglichkeiten bzw. mögliche Indikationen für den Wirkstoff Acetoacetat bzw. die physiologisch durch Reduktion von Acetoacetat erhältliche 3-Hydroxybuttersäure bzw. dessen Salz (d. h. 3-Hydroxybutyrat).

| **Indikation** | **Therapeutischer Effekt** |
|---|---|
| Schädel-Hirn-Trauma | Senkung der Apoptose und Nekrose-Rate von Nervenzellen |
| Schlaganfall | Senkung der Apoptose und Nekrose-Rate von Nervenzellen |
| Refeeding-Syndrom | Bei Anorexie, Absetzen enteraler oder parenteraler Ernährung und nach langen Hungerperioden kann der Konsum von Stärke oder Glucose zum Tod führen (siehe auch WHO-Schema Erdnusspaste). Acetoacetat bzw. physiologisch hieraus generiertes BHB kann hier als Therapeutikum zum schnelleren Erlangen einer normalen Nahrungsaufnahme eingesetzt werden. |
| Appetitzügler | Unterdrückung des Hungergefühls im Zentralnervensystem (ZNS) |
| Epilepsie | Herkömmliche ketogene Diät zur signifikanten Reduzierung der Häufigkeit von Krampfanfällen hat extrem schlechte Patienten-Verträglichkeit. Acetoacetat bzw. physiologisch hieraus generiertes BHB bietet hier eine unmittelbar wirksame Alternative. |
| Morbus Alzheimer, Demenz | Verbesserung der kognitive Leistung von Patienten Acetoacetat bzw. physiologisch hieraus generiertes BHB ist auch für die Prävention von neurodegenerativen Erkrankungen wirksam. |
| Störungen der Fettsäureoxidation (z. B. Electron-Transfer-Protein Defekt) | Ausgleich eines Nährstoffmangels bei Defekt im Energiestoffwechsel. |

Daher ist es aus pharmazeutischer und klinischer Hinsicht wünschenswert, wirksame Präkursoren oder Metabolite auffinden zu können, welche physiologisch einen direkten oder indirekten Zugang zu Acetoacetat (und damit physiologisch auch zu 3-Hydroxybuttersäure oder deren Salzen) ermöglichen, insbesondere im physiologischen Stoffwechsel des menschlichen oder tierischen Körpers.

Folglich hat es im Stand der Technik nicht an Versuchen gefehlt, physiologisch geeignete Präkursoren oder Metaboliten für 3-Hydroxybuttersäure bzw. deren Salze aufzufinden. Bislang wurden im Stand der Technik jedoch keine effizienten diesbezüglichen Verbindungen aufgefunden. Auch ist ein diesbezüglicher Zugang zu solchen Verbindungen nach dem Stand der Technik bislang nicht bzw. nicht ohne Weiteres möglich.

Die WO 2008/005818 A1 betrifft alternative Quellen für Ketokörper zur Verringerung oder Beseitigung der Symptome von Parkinson, amyotropher Lateralsklerose (ALS bzw. auch Lou-Gehrig genannt), Alzheimer, Huntington, Epilepsie und anderen Krankheiten oder Störungen, welche durch einen gestörten Glykosestoffwechsel gekennzeichnet sind, wobei die alternativen Quellen für Ketokörper Mono-, Di- oder Triglyceridester von Acetoacetat und Gemischen davon bzw. Mono-, Di- oder Triglyceridester von 3-Hydroxybutyrat und deren Mischungen umfassen, wobei die Glyceridester oral als Nahrungsergänzungsmittel oder in einer Nährstoffzusammensetzung verabreicht werden können.

Darüber hinaus betrifft die wissenschaftliche Publikation Michael J. Haas et al., European Journal of Lipid Science and Technology, Bd. 113, Nr. 2, 1. Februar 2011, Seiten 168-179 die Verwendung von immobilisierter *Rhizomucor-miehei-*Lipase bei der Synthese von partiellen Acylglycerinen von Acetoacetat, wobei aus der Veresterung von Glycerin mit Ethylacetoacetat sowohl 1(3)-*sn-*Monoacetoacetylglycerin (MAcG) als auch 1,3-sn-Diacetoacetylglycerin (DAcG) resultieren, wobei verschiedene Reaktionsbedingungen zur vollständigen Umsetzung des Glycerins zu einer Mischung aus Mono- und Diacetoacetylesten analysiert werden.

Weiterhin betrifft die wissenschaftliche Publikation Sanjib Kumar Karmee et al., Synthetic Communications, Bd. 35, Nr. 9, 1. Mai 2005, Seiten 1151-1160 die selektive Umesterung von Polyolen mit Ketoestern bei Mikrowellenbestrahlung unter lösemittelfreien und katalysatorfreien Bedingungen unter Bildung der entsprechenden Monoester.

Ferner betrifft die US 3 907 994 A Alkylester von Chinoxalin-di-N-oxid-2-carbonsäure, welche am Alkylrest des Esters durch Hydroxy-, Acyloxy-, N-Alkylcarbamyloxy-, Dialkylaminoacyloxy-, Carboxyacyloxy-, Alkoxycarbonyloxy-, Halogenacyloxy- und Amino- sowie mono- und disubstituierte Amino-Reste substituiert sind, wobei diese Alkylester als antibakterielle Mittel und zur Förderung des Wachstums und zur Verbesserung der Futtereffizienz von Tieren im Allgemeinen einsetzbar sein sollen.

Die Druckschrift GB 2 046 091 A betrifft pharmazeutische Zusammensetzungen zur oralen oder rektalen Verabreichung, welche (1) eine spezifische β-Diketonverbindung; und (2) eine pharmakologisch aktive Substanz (z.B. Insulin, β-Laktam-Antibiotika) umfassen, wobei die β-Diketone gemäß (1) die Förderung der Absorption des pharmakologisch aktiven Stoffes über den Darmtrakt fördern sollen.

Darüber hinaus betrifft die Druckschrift JP 2007 277441 A die Bereitstellung eines Weichmachers für ein biologisch abbaubares Harz mit geringem Geruch und Flexibilität sowie die Bereitstellung einer biologisch abbaubaren Harzzusammensetzung mit geringem Geruch und geringer Flexibilität, wobei der Weichmacher eine Verbindung mit mindestens einer Acetessigsäureestergruppe umfasst.

Weiterhin betrifft die US 2009/253781 A1 Zusammensetzungen, welche Ketokörper bzw. ihre metabolischen Vorläufer umfassen, welche zur Verabreichung an Menschen oder Tieren geeignet sind und welche unter anderem die folgenden Eigenschaften haben (i) Erhöhung der Herzeffizienz, insbesondere der Effizienz bei der Verwendung von Glucose; (ii) Bereitstellung einer Energiequelle, insbesondere bei Diabetes- und Insulinresistenzzuständen; und (iii) Behandlung von Störungen, die durch Schädigung von Gehirnzellen verursacht werden, insbesondere durch Verzögerung oder Verhinderung von Hirnschäden in gedächtnis-assoziierten Hirnregionen, wie sie bei Alzheimer oder ähnlichen Erkrankungen auftreten. US 2009/253781 A1 betrifft weiterhin die Verwendung dieser Zusammensetzungen als Ernährungshilfen, beispielsweise für Sportler, oder zur Behandlung von Erkrankungen, insbesondere solchen, die mit einer schlechten Herzleistung, Insulinresistenz und neuronalen Schäden verbunden sind.

Ferner betrifft die WO 95/09146 A1 Zusammensetzungen, welche als Nährstoffe nützlich sein sollen, wobei die Zusammensetzungen sterile wässrige Lösungen mit einer wirksamen Menge mindestens eines Glycerinbisacetoacetats umfassen, wobei die Zusammensetzungen als Ersatz für Glycose bei der intravenösen Nahrungszufuhr nützlich sein sollen.

Die Druckschrift WO 90/02549 A1 betrifft einen Nährstoff für Menschen oder Tiere, welcher mindestens ein nicht leicht wasserlösliches Glycerid von mindestens einer Ketosäure oder Hydroxysäure enthält, wobei der Nährstoff mit der Nahrung oder in Form einer Emulsion des Glycerids verabreicht werden kann.

Darüber hinaus betrifft US 2009/137710 A1 eine Organosiliziumverbindung mit einer β-Ketoester-Struktur und eine Haftklebstoffzusammensetzung für eine Flüssigkristallelement, welches die Organosiliziumverbindung enthält.

Weiterhin betrifft EP 0 022 978 A1 ungesättigte Acetessigsäureester, welche sich als Comonomere bei der Polymerisation von ungesättigten Verbindungen in wässrigem Medium eignen, wobei insbesondere Acetylacetoxy-alkyl-allyl-ether besonders geeignet sein sollen, wobei die Allylether-Derivate insbesondere durch Umsetzung eines Allylalkohols mit einem Epoxid und anschließende Reaktion des erhaltenen Hydroxyalkyl-allyl-ethers mit Diketen hergestellt werden, wobei beide Reaktionsschritte bei einer Temperatur von 0 bis 100 °C, vorzugsweise in Gegenwart eines Katalysators, durchgeführt werden und die erhältlichen Allylether-Derivate beispielsweise mit Vinylverbindungen, wie Vinylestern, Acrylsäureestern, Olefinen, Vinylhallogeniden und Vinylaromaten, copolymerisierbar sein sollen.

Schließlich betrifft US 2003/064954 A1 amphiphile 1,4-Dihydropyridin-Derivate, welche zur Herstellung einer Zusammensetzung zur Abgabe von nukleotidhaltigen Verbindungen in eine Zielzelle bzw. ihren Kern nützlich sein sollen, wobei die Zusammensetzung 1,4-Dihydropyridin-Derivate mit DNA-Kondensationskapazität und der Fähigkeit zur Selbstassoziation umfasst. Weiterhin betrifft US 2003/064954 A1 Zusammensetzungen, welche die Derivate umfassen, welche mit Nukleotid enthaltenden Verbindungen komplexiert sind, sowie Verfahren zur Herstellung der Komplexe und die Verwendung der 1,4- Dihydropyridin-Derivate zur Herstellung von Systemen zur Abgabe von Nukleotid enthaltenden Verbindungen, welche bei der Gentherapie und DNA-Imfpung nützlich sein sollen.

Das der vorliegenden Erfindung zugrundeliegende Problem liegt also in der Bereitstellung eines effizienten Herstellungsverfahrens von physiologisch geeigneten bzw. physiologisch kompatiblen Präkursoren und/oder Metaboliten von Acetoacetat bzw. letztendlich von 3-Hydroxybuttersäure (d. h. beta-Hydroxybuttersäure bzw. BHB bzw. 3-BHB) oder deren Salzen.

Ein solches Verfahren soll insbesondere Acetoacetat bzw. die betreffenden BHB-Präkursoren und/oder BHB-Metaboliten in effizienter Weise zugänglich machen, insbesondere auch in größeren Mengen und ohne nennenswerte Mengen an toxischen Nebenprodukten.

In vollkommen überraschender Weise hat die Anmelderin nunmehr herausgefunden, dass Polyglycerinester der 3-Oxobuttersäure einen effizienten und physiologisch wirksamen bzw. physiologisch kompatiblen Präkursor und/oder Metaboliten für den Ketokörper Acetoacetat bzw. für die unter physiologischen Bedingungen reduktiv hieraus erzeugte 3-Hydroxybuttersäure bzw. deren Salze darstellen, und hat in diesem Zusammenhang ein effizientes Herstellungsverfahren für diese Verbindungen auffinden bzw. entwickeln können, welches einen direkten und wirksamen, insbesondere ökonomischen wie auch großtechnisch umsetzbaren Zugang zu diesen Verbindungen ermöglicht.

Zur Lösung des zuvor geschilderten Problems schlägt die vorliegende Erfindung daher - gemäß einem **ersten** Aspekt der vorliegenden Erfindung - ein Verfahren zur Herstellung von Polyglycerinestern der 3-Oxobuttersäure (beta-Oxobuttersäure, 3-Oxobutansäure) gemäß Anspruch 1 vor; weitere, insbesondere besondere und/oder vorteilhafte Ausgestaltungen dieses erfindungsgemäßen Verfahrens sind Gegenstand der diesbezüglichen Verfahrensunteransprüche.

Des Weiteren betrifft die vorliegende Erfindung - gemäß einem **zweiten** Aspekt der vorliegenden Erfindung - ein Verfahren zur Herstellung von funktionalisierten, insbesondere fettsäurefunktionalisierten, Polyglycerinestern der 3-Oxobuttersäure (beta-Oxobuttersäure, 3-Oxobutansäure) gemäß Anspruch 6.

Weiterhin betrifft die vorliegende Erfindung - gemäß einem **dritten** Aspekt der vorliegenden Erfindung -einen gegebenenfalls funktionalisierten Polyglycerinester der 3-Oxobuttersäure (beta-Oxobuttersäure, 3-Oxobutansäure) bzw. diesbezügliche Gemische hiervon gemäß den diesbezüglichen Ansprüchen (Ansprüche 7 bis 13); weitere, insbesondere besondere und/oder vorteilhafte Ausgestaltungen dieses Erfindungsaspekts sind Gegenstand der diesbezüglichen Unteransprüche.

Gleichermaßen betrifft die vorliegende Erfindung - gemäß einem **vierten** Aspekt der vorliegenden Erfindung - eine pharmazeutische Zusammensetzung, insbesondere Arzneimittel oder Medikament, gemäß dem diesbezüglichen unabhängigen Anspruch (Anspruch 15); weitere, insbesondere besondere und/oder vorteilhafte Ausgestaltungen dieses Erfindungsaspekts sind Gegenstand des diesbezüglichen Unteranspruchs.

Des Weiteren betrifft die vorliegende Erfindung - gemäß einem **fünften** Aspekt der vorliegenden Erfindung - ein Nahrungsmittel und/oder Lebensmittelerzeugnis gemäß dem diesbezüglichen unabhängigen Anspruch (Anspruch 17).

Es versteht sich bei den nachfolgenden Ausführungen von selbst, dass Ausgestaltungen, Ausführungsformen, Vorteile und dergleichen, welche nachfolgend zu Zwecken der Vermeidung von Wiederholungen nur zu einem Erfindungsaspekt ausgeführt sind, selbstverständlich auch in Bezug auf die übrigen Erfindungsaspekte entsprechend gelten, ohne dass dies einer gesonderten Erwähnung bedarf.

Des Weiteren versteht es sich von selbst, dass einzelne Aspekte und Ausführungsformen der vorliegenden Erfindung auch in beliebiger Kombination mit anderen Aspekten und Ausführungsformen der vorliegenden Erfindung als offenbart gelten und insbesondere auch eine beliebige Kombination von Merkmalen und Ausführungsformen, wie sie sich aus den Rückbezügen aller Patentansprüche ergibt, umfangreich als offenbart gilt, und zwar im Hinblick auf alle sich ergebenden Kombinationsmöglichkeiten.

Bei allen nachstehend genannten relativen bzw. prozentualen gewichtsbezogenen Angaben, insbesondere relativen Mengen- oder Gewichtsangaben, ist weiterhin zu beachten, dass diese im Rahmen der vorliegenden Erfindung vom Fachmann derart auszuwählen sind, dass sie sich in der Summe unter Einbeziehung aller Komponenten bzw. Inhaltsstoffe, insbesondere wie nachfolgend definiert, stets zu 100 % bzw. 100 Gew.-% ergänzen bzw. addieren; dies versteht sich aber für den Fachmann von selbst.

Im Übrigen gilt, dass der Fachmann - anwendungsbezogen oder aber einzelfallbedingt - von den nachfolgend angeführten Bereichsangaben erforderlichenfalls abweichen kann, ohne dass er den Rahmen der vorliegenden Erfindung verlässt.

Zudem gilt, dass alle im Folgenden genannten Werte- bzw. Parameterangaben oder dergleichen grundsätzlich mit genormten bzw. standardisierten oder explizit angegebenen Bestimmungsverfahren oder andernfalls mit dem Fachmann auf diesem Gebiet an sich geläufigen Bestimmungs- bzw. Messmethoden ermittelt bzw. bestimmt werden können.

Dies vorausgeschickt, wird die vorliegende Erfindung nunmehr nachfolgend im Detail erläutert.

Gegenstand der vorliegenden Erfindung - gemäß einem **ersten** Aspekt der vorliegenden Erfindung - ist somit ein Verfahren zur Herstellung von Polyglycerinestern der 3-Oxobuttersäure (beta-Oxobuttersäure, 3-Oxobutansäure), wobei mindestens eine Verbindung der allgemeinen Formel (I)

CH₃- C(O) - CH₂ - C(O)OR¹ (I)

wobei in der allgemeinen Formel (I) der Rest R¹ ein C₁-C₄-Alkyl, insbesondere Methyl oder Ethyl, bevorzugt Ethyl, darstellt,
mit mindestens einem Polyglycerin der allgemeinen Formel (IIb)

   HO - CH₂ - CH(OH) - CH₂ - [O - CH₂ - CH(OH) - CH₂]ₚ - OH (IIb)
wobei in der allgemeinen Formel (IIb) die Variable p eine ganze Zahl von 1 bis 6, insbesondere 1 bis 4, vorzugsweise 1 oder 2, besonders bevorzugt 1, darstellt,
umgesetzt wird,
wobei die Umsetzung in Abwesenheit von Lösemitteln durchgeführt wird und
wobei die Umsetzung in Gegenwart eines Katalysators durchgeführt wird,
   so dass als Reaktionsprodukt ein oder mehrere 3-Oxobuttersäure-Polyglycerinester erhalten werden
wobei bei der Umsetzung gleichzeitig die Verbindung gemäß der allgemeinen Formel (IV)

   R¹ - OH (IV)
gebildet wird, wobei in der allgemeinen Formel (IV) der Rest R¹ ein C₁-C₄-Alkyl, insbesondere Methyl oder Ethyl, bevorzugt Ethyl, darstellt, wobei die Verbindung gemäß der allgemeinen Formel (IV) der Umsetzung kontinuierlich entzogen wird.

Wie zuvor ausgeführt, hat die Anmelderin nämlich vollkommen überraschend herausgefunden, dass die auf diese Weise hergestellten Polyglycerinester der 3-Oxobuttersäure (beta-Oxobuttersäure, 3-Oxobutansäure) - synonym auch als "3-Oxobuttersäure-Polyglycerinester" bezeichnet - effiziente, da physiologisch verträgliche Präkursoren und/oder Metabolite der 3-Hydroxybuttersäure bzw. deren Salzen darstellen, welche pharmazeutisch bzw. klinisch auch in größeren Mengen zum Einsatz kommen können, da sie physiologisch kompatibel sind.

Die vorgenannten Polyglycerinester der 3-Oxobuttersäure, welche durch das erfindungsgemäße Herstellungsverfahren erstmals in effizienter Weise zugänglich sind, stellen eine physiologisch und pharmakologisch relevante Alternative zu der freien 3-Hydroxybuttersäure bzw. deren Salzen dar.

Die Herstellung von Polyglycerinestern der 3-Oxobuttersäure mittels herkömmlicher organischer Synthese ist komplex und aufwendig, da 3-Oxobuttersäure instabil ist und zerfällt. Im Rahmen der vorliegenden Erfindung konnte erstmals ein effizient arbeitendes Herstellungsverfahren bereitgestellt werden, mit welchem sich Polyglycerinester der 3-Oxobuttersäure ohne unerwünschte Nebenreaktionen herstellen lassen, insbesondere einstufig.

Das erfindungsgemäße Verfahren ermöglicht somit erstmalig die Bereitstellung untoxischer Polyglycerinester der 3-Oxobuttersäure aus an sich bekannten, kommerziell verfügbaren und vor allem physiologisch unbedenklichen Komponenten bzw. Edukten (Ausgangsverbindungen). Die resultierenden Polyglycerinester der 3-Oxobuttersäure, können physiologisch, insbesondere im Magen und/oder im Darm, aufgespalten werden und das Zielmolekül "Acetoacetat (Acetatacetat)" und letztlich hieraus "3-Hydroxybuttersäure" bzw. deren Salze als Wirkstoff bzw. Wirkkomponente freisetzen bzw. generieren.

Darüber hinaus weisen die vorgenannten Polyglycerinester der 3-Oxobuttersäure auch einen akzeptablen Geschmack auf, um eine Kompatibilität auch bei oraler Verabreichung größerer Mengen über einen längeren Zeitraum zu gewährleisten (z. B. Verabreichung von 50 g Tagesdosis oder mehr).

Gleichermaßen ermöglicht es das erfindungsgemäße Herstellungsverfahren die Polyglycerinester der 3-Oxobuttersäure frei von toxischen Verunreinigungen bereitzustellen.

Darüber hinaus ist das erfindungsgemäße Herstellungsverfahren, einschließlich optionaler Weiterverarbeitungs- bzw. Aufreinigungsverfahrensschritte, wirtschaftlich bzw. ökonomisch betreibbar und auch großtechnisch umsetzbar.

Insbesondere verwendet das erfindungsgemäße Herstellungsverfahren kommerziell verfügbare Edukte, welche eine ökonomisch effiziente und darüber hinaus eine relativ einfache Verfahrensführung ermöglichen, auch bei großtechnischer Umsetzung.

Im Gegensatz zu herkömmlichen Herstellungsverfahren des Standes der Technik kommt das erfindungsgemäße Herstellungsverfahren ohne komplexe Edukte aus und verläuft nur einstufig. Dennoch werden im Rahmen des erfindungsgemäßen Herstellungsverfahrens exzellente Ausbeuten erzielt, wobei in gleicher Weise die Bildung von Nebenprodukten minimiert bzw. vermieden wird. Insbesondere kann durch die erfindungsgemäß eingesetzten Edukte zur Bildung eines 3-Hydroxybuttersäure Präkursoren bzw. Metaboliten die Dimerisierung verhindert werden.

Darüber hinaus ist das erfindungsgemäßen Verfahren einfach und wirtschaftlich. Das erfindungsgemäße Verfahren wird in Abwesenheit von Lösemitteln und/oder ohne jedwedes Lösemittel durchgeführt (d. h. also als Reaktion in Masse bzw. als Reaktion in Substanz bzw. als sogenannte *Bulk Reaction*); folglich sind die erhaltenen Reaktionsprodukte nicht mit Lösemittel verunreinigt und es muss kein Lösemittel nach Durchführung des Verfahrens bzw. der Reaktion aufwendig und energieträchtig entfernt und entsorgt bzw. rezykliert werden. Es werden zudem auch keine toxischen Nebenprodukte gebildet.

Das erfindungsgemäße Herstellungsverfahren führt üblicherweise zu einem Gemisch verschiedener Polyglycerinester der 3-Oxobuttersäure, d. h. zu einem Gemisch von mindestens zwei, insbesondere mindestens drei voneinander verschiedenen Polyglycerinestern der 3-Oxobuttersäure. Das resultierende Rohreaktionsprodukt bzw. Rohgemisch kann mit an sich bekannten Methoden aufgereinigt werden, insbesondere von gegebenenfalls noch vorhandenen Edukten und/oder gegebenenfalls vorhandenen Nebenprodukten befreit werden, und darüber hinaus - sofern gewünscht - mit ebenfalls an sich bekannten Methoden aufgespalten werden, insbesondere destillativ und/oder chromatographisch (z. B. Fraktionierung in die einzelnen Polyolester, d. h. Mono-, Di-, Tri- usw. Polyolester der 3-Oxobuttersäure, oder aber Fraktionierung in Fraktionen mit angereichertem und abgereichertem Anteil einzelner Fraktionen etc.).

Weiterhin benötigt das erfindungsgemäße Herstellungsverfahren keine enantioselektive Reaktionsführung, da die Bildung bzw. Freisetzung des Wirkstoffs 3-Hydroxybuttersäure im Magen/Darm-Trakt grundsätzlich in die physiologischrelevante Form des R-Enantionmers erfolgt.

Erfindungsgemäß bevorzugt ist es, wenn in der allgemeinen Formel (I) der Rest R¹ Ethyl darstellt.

Mit anderen Worten ist es erfindungsgemäß bevorzugt, dass als Verbindung der allgemeinen Formel (I) 3-Oxobuttersäureethylester (Ethyl-3-oxobutyrat) der Formel CH₃ - C(O) - CH₂ - C(O)OC₂H₅ eingesetzt wird.

Dies ermöglicht eine besonders effiziente Verfahrensführung und hohe Ausbeuten mit minimierter bzw. unterdrückter Nebenproduktbildung. Außerdem ist der 3-Oxobuttersäureethylester auch in großen Mengen kommerziell verfügbar und zudem, im Gegensatz zur freien Säure (d. h. 3-Oxobuttersäure), stabil und dadurch mit geringerem Aufwand verwendbar. Insbesondere kann der 3-Oxobuttersäureethylester als Ausgangsverbindung großtechnisch z. B. durch Claisen-Kondensation von Ethylacetat gewonnen werden.

Bei dem erfindungsgemäßen Verfahren wird die Umsetzung in Abwesenheit von Lösemitteln und/oder ohne jedwedes Lösemittel durchgeführt. D. h. die Umsetzung wird also als Reaktion in Masse bzw. als Reaktion in Substanz bzw. als sogenannte *Bulk Reaction* durchgeführt. Dies hat den Vorteil, dass die erhaltenen Reaktionsprodukte nicht mit Lösemittel verunreinigt sind und kein Lösemittel nach Durchführung des Verfahrens bzw. der Reaktion aufwendig und energieträchtig entfernt und entsorgt bzw. rezykliert werden muss. Überraschenderweise verläuft das Verfahren bzw. die Reaktion dennoch mit hohen Umsätzen und Ausbeuten und zumindest im Wesentlichen ohne signifikante Nebenproduktbildung.

Gemäß der vorliegenden Erfindung wird die Umsetzung in Gegenwart eines Katalysators, insbesondere eines Enzyms und/oder eines metallhaltigen und/oder metallbasierten sauren oder basischen Katalysators, durchgeführt. Bei dieser besonderen Ausführungsform ist es bevorzugt, wenn der Katalysator nach der Umsetzung rezykliert wird.

Wie zuvor ausgeführt, kann gemäß einer besonderen Ausführungsform des erfindungsgemäßen Herstellungsverfahrens die Umsetzung in Gegenwart eines Enzyms als Katalysator durchgeführt werden.

Dabei kann das Enzym insbesondere ausgewählt sein aus Synthetasen (Ligasen), Katalasen, Esterasen, Lipasen und deren Kombinationen. Erfindungsgemäß werden als Synthetasen (synonym Ligasen) insbesondere Enzyme aus der Klasse der Ligasen bezeichnet; Ligasen sind Enzyme, welche das Verknüpfen zweier oder mehrerer Moleküle durch eine kovalente Bindung katalysieren. Katalasen im Sinne der vorliegenden Erfindung sind insbesondere Enzyme, welche Wasserstoffperoxid zu Sauerstoff und Wasser umzusetzen imstande sind. Der Begriff der Esterasen bezeichnet insbesondere Enzyme, welche imstande sind, Ester hydrolytisch in Alkohol und Säure aufzuspalten (Verseifung); es handelt sich somit insbesondere um Hydrolasen, wobei fettspaltende Esterasen auch als Lipasen bezeichnet werden. Lipasen im Sinne der vorliegenden Erfindung sind insbesondere Enzyme, welche von Lipiden, wie Glyceriden, freie Fettsäuren abzuspalten imstande sind (Lipolyse).

Im Rahmen der vorliegenden Erfindung kann sich das als Katalysator eingesetzte Enzym insbesondere ableiten von *Candida antarctica, Mucor miehei (Rhizomucor miehei), Thermomyces lanuginosus, Candida rugosa, Aspergillus oryzae, Pseudomonas cepacia, Pseudomonas fluorescens, Rhizopus delemar* und *Pseudomonas* sp. sowie deren Kombinationen, vorzugsweise von *Candida antarctica, Mucor miehei (Rhizomucor miehei)* und *Thermomyces lanuginosus.*

Gemäß einer besonderen Ausführungsform kann das Enzym in immobilisierter Form, immobilisiert auf einem Träger, vorzugsweise auf einem polymeren Träger, bevorzugt auf einem polymeren organischen Träger, besonders bevorzugt mit hydrophoben Eigenschaften, ganz besonders bevorzugt auf einem poly(meth)acrylharzbasierten Träger, eingesetzt werden.

Wie zuvor im Zusammenhang mit der Verwendung eines Katalysators im Allgemeinen dargelegt, ist es bevorzugt, im Fall der Verwendung eines Enzyms als Katalysator das Enzym nach der Umsetzung zu rezyklieren.

Sofern die Umsetzung im Rahmen des erfindungsgemäßen Herstellungsverfahrens in Gegenwart eines Enzyms als Katalysator durchgeführt wird, ist es bevorzugt, wenn die Umsetzung bei Temperaturen im Bereich von 10 °C bis 80 °C, insbesondere im Bereich von 20 °C bis 80 °C, vorzugsweise im Bereich von 25 °C bis 75 °C, besonders bevorzugt im Bereich von 45 °C bis 75 °C, ganz besonders bevorzugt im Bereich von 50 °C bis 70 °C, durchgeführt wird.

Im Fall der Verwendung eines Enzyms als Katalysator kann die Menge des eingesetzten Enzyms in weiten Bereichen variieren. Insbesondere kann das Enzym in Mengen, bezogen auf die Gesamtmenge der Ausgangsverbindungen (I) und (IIb), im Bereich von 0,001 Gew.-% bis 20 Gew.-%, insbesondere im Bereich von 0,01 Gew.-% bis 15 Gew.-%, vorzugsweise im Bereich von 0,1 Gew.-% bis 15 Gew.-%, bevorzugt im Bereich von 0,5 Gew.-% bis 10 Gew.-%, eingesetzt werden. Dennoch kann es einzelfallbedingt oder anwendungsbezogen erforderlich sein, von den vorgenannten Mengen abzuweichen, ohne dass der Rahmen der vorliegenden Erfindung verlassen ist.

Wenn gemäß einer besonderen Ausführungsform der vorliegenden Erfindung die Umsetzung in Gegenwart eines Enzyms als Katalysator durchgeführt wird, kann auch der angewendete Druckbereich in weiten Bereichen variieren. Insbesondere kann bei Umsetzung in Gegenwart eines Enzyms als Katalysator die Umsetzung bei einem Druck im Bereich von 0,0001 bar bis 10 bar, insbesondere im Bereich von 0,001 bar bis 5 bar, vorzugsweise im Bereich von 0,01 bar bis 2 bar, besonders bevorzugt im Bereich von 0,05 bar bis 1 bar, ganz besonders bei etwa 1 bar, durchgeführt werden.

Gemäß einer alternativen Ausführungsform der vorliegenden Erfindung kann die Umsetzung in Gegenwart eines metallhaltigen und/oder metallbasierten, sauren oder basischen Katalysators durchgeführt werden.

Gemäß dieser alternativen Ausführungsform der vorliegenden Erfindung, wonach die Umsetzung in Gegenwart eines metallhaltigen und/oder metallbasierten, sauren oder basischen Katalysators durchgeführt wird, kann der Katalysator insbesondere ausgewählt sein aus (i) basischen Katalysatoren, insbesondere Alkali- oder Erdalkalihydroxyden und Alkali- oder Erdalkalialkoholaten, wie NaOH, KOH, LiOH, Ca(OH)₂, NaOMe, KOMe und Na(OBu-tert.), (ii) sauren Katalysatoren, insbesondere Mineralsäuren, und organischen Säuren, wie Schwefelsäure, Salzsäure, Phosphorsäure, Salpetersäure, Sulfonsäuren, Methansulfonsäure, para-Toluolsulfonsäure und Carbonsäuren, (iii) Lewis-Säuren, insbesondere Lewis-Säuren auf der Basis von Titan-, Zinn-, Zink- und Aluminiumverbindungen, wie Titantetrabutylat, Zinnsäuren, Zinkacetat, Aluminiumtrichlorid und Aluminiumtri-isopropyl und (iv) heterogenen Katalysatoren, insbesondere auf der Basis von mineralischen Silikaten, Germanaten, Carbonaten und Aluminiumoxiden, wie Zeolithen, Montmorilloniten, Mordeniten, Hydrotalciten und Tonerden, sowie deren Kombinationen.

Bei dieser Ausführungsform kann als Katalysator insbesondere ein Alkali- oder Erdalkalialkoholat eingesetzt werden.

Insbesondere ist es auch bei dieser Ausführungsform bevorzugt, wenn der Katalysator auf Basis des metallhaltigen und/oder metallbasierten sauren oder basischen Katalysators nach der Umsetzung rezykliert wird.

Wenn gemäß der besonderen Ausführungsform der vorliegenden Erfindung die Umsetzung in Gegenwart eines metallhaltigen und/oder metallbasierten, sauren oder basischen Katalysators erfolgt, können die Temperaturen in weiten Bereichen variiert werden. Insbesondere kann die Umsetzung in Gegenwart eines metallhaltigen und/oder metallbasierten sauren oder basischen Katalysators bei Temperaturen im Bereich von 20 °C bis 150 °C, insbesondere im Bereich von 50 °C bis 140 °C, vorzugsweise im Bereich von 70 °C bis 130 °C, besonders bevorzugt im Bereich von 80 °C bis 125 °C, ganz besonders bevorzugt im Bereich von 100 °C bis 120 °C, durchgeführt werden.

Weiterhin kann auch bei dieser Ausführungsform der Katalysator (d. h. der metallhaltige und/oder metallbasierte, saure oder basische Katalysator) in weiten Mengenbereichen variiert werden: So kann der Katalysator auf Basis eines metallhaltigen und/oder metallbasierten, sauren oder basischen Katalysators in Mengen, bezogen auf die Gesamtmenge der Ausgangsverbindungen (I) und (IIb), im Bereich von 0,01 Gew.-% bis 30 Gew.-%, insbesondere im Bereich von 0,05 Gew.-% bis 15 Gew.-%, vorzugsweise im Bereich von 0,1 Gew.-% bis 15 Gew.-%, bevorzugt im Bereich von 0,2 Gew.-% bis 10 Gew.-%, eingesetzt werden. Dennoch ist es anwendungsbezogen oder einzelfallbedingt möglich, von den vorgenannten Mengen abzuweichen, ohne dass der Rahmen der vorliegenden Erfindung verlassen ist.

Wenn gemäß dieser besonderen Ausführungsform der vorliegenden Erfindung die Umsetzung in Gegenwart eines metallhaltigen und/oder metallbasierten, sauren oder basischen Katalysators erfolgt, kann der Druckbereich gleichermaßen in einem weiten Regime variieren: Insbesondere kann die Umsetzung in Gegenwart eines metallhaltigen und/oder metallbasierten, sauren oder basischen Katalysators bei einem Druck im Bereich von 0,0001 bar bis 10 bar, insbesondere im Bereich von 0,001 bar bis 5 bar, vorzugsweise im Bereich von 0,01 bar bis 2 bar, besonders bevorzugt im Bereich von 0,05 bar bis 1 bar, ganz besonders bei etwa 1 bar, durchgeführt werden.

Was die Menge an Edukten bzw. Ausgangsverbindungen anbelangt, so kann auch diese in weiten Bereichen variiert werden.

Unter Berücksichtigung von Verfahrensökonomie und Optimierung des Verfahrensablaufs, insbesondere im Hinblick auf die Minimierung von Nebenprodukten, ist es vorteilhaft, wenn die Verbindung der allgemeinen Formel (I), bezogen auf die Hydroxylgruppen des Polyglycerins (IIb) in molaren Mengen in einem Bereich von äquimolarer Menge bis zu einem molaren Überschuss von 200 Mol-%, insbesondere in einem Bereich von äquimolarer Menge bis zu einem molaren Überschuss von 150 Mol-%, vorzugsweise in einem Bereich von äquimolarer Menge bis zu einem molaren Überschuss von 100 Mol-%, eingesetzt wird.

Gleichermaßen unter Berücksichtigung von Verfahrensökonomie und Optimierung des Verfahrensablaufs, insbesondere im Hinblick auf die Minimierung von Nebenprodukten, ist es vorteilhaft, wenn die Verbindung der allgemeinen Formel (I) und das Polyglycerin (IIb) in einem Molverhältnis von Verbindung der allgemeinen Formel (I) / Polyglycerin (IIb) in einem Bereich von 1 :1 bis 10 : 1, insbesondere in einem Bereich von 2 : 1 bis 8 : 1, vorzugsweise in einem Bereich von 3 : 1 bis 6 : 1, eingesetzt werden.

Gemäß dem erfindungsgemäßen Verfahren entspricht das Polyglycerin der allgemeinen Formel (IIb)

HO - CH₂ - CH(OH) - CH₂ - [O - CH₂ - CH(OH) - CH₂]ₚ - OH (IIb)

wobei in der allgemeinen Formel (IIb) die Variable p eine ganze Zahl von 1 bis 6, insbesondere 1 bis 4, vorzugsweise 1 oder 2, besonders bevorzugt 1.

Gemäß einer weiteren besonderen Ausführungsform des erfindungsgemäßen Verfahrens kann das Polyglycerin (IIb) ein Diglycerin der Formel (IIc)

HO - CH₂ - CH(OH) - CH₂ - O - CH₂ - CH(OH) - CH₂ - OH (IIc)

sein.

Gemäß einer bevorzugten Ausführungsform des erfindungsgemäßen Verfahrens ist das Polyglycerin (IIb) kein Propan-1,2,3-triol ist, d. h. das Polyglycerin (IIb) kein Glycerin ist.

Gemäß einer bevorzugten Ausführungsform der vorliegenden Erfindung betrifft die vorliegende Erfindung gemäß diesem Erfindungsaspekt ein Verfahren zur Herstellung von Polyglycerinestern der 3-Oxobuttersäure (beta-Oxobuttersäure, 3-Oxobutansäure), insbesondere ein wie zuvor beschriebenes Verfahren,
wobei mindestens eine Verbindung der allgemeinen Formel (I)

   CH₃ - C(O) - CH₂ - C(O)OR¹ (I)
wobei in der allgemeinen Formel (I) der Rest R¹ ein C₁-C₄-Alkyl, insbesondere Methyl oder Ethyl, bevorzugt Ethyl, darstellt,
mit mindestens einem Polyglycerinen der allgemeinen Formel (IIb)

   HO - CH₂ - CH(OH) - CH₂ - [O - CH₂ - CH(OH) - CH₂]ₚ - OH (IIb)
wobei in der allgemeinen Formel (IIb) die Variable p eine ganze Zahl von 1 bis 6, insbesondere 1 bis 4, vorzugsweise 1 oder 2, besonders bevorzugt 1, darstellt, umgesetzt wird,
so dass als Reaktionsprodukt ein oder mehrere 3-Oxobuttersäure-Polyglycerinester erhalten werden.

Gemäß einer weiteren bevorzugten Ausführungsform der vorliegenden Erfindung betrifft die vorliegende Erfindung gemäß diesem Erfindungsaspekt ein Verfahren zur Herstellung von Polyglycerinestern der 3-Oxobuttersäure (beta-Oxobuttersäure, 3-Oxobutansäure), insbesondere ein wie zuvor beschriebenes Verfahren,
wobei mindestens eine Verbindung der allgemeinen Formel (I)

   CH₃- CH(OH) - CH₂ - C(O)OR¹ (I)
wobei in der allgemeinen Formel (I) der Rest R¹ Ethyl darstellt,
mit mindestens einem Polyglycerinen der allgemeinen Formel (IIb)

   HO - CH₂ - CH(OH) - CH₂ - [O - CH₂ - CH(OH) - CH₂]ₚ - OH (IIb)
wobei in der allgemeinen Formel (IIb) die Variable p eine ganze Zahl von 1 bis 6, insbesondere 1 bis 4, vorzugsweise 1 oder 2, besonders bevorzugt 1, darstellt, umgesetzt wird,
so dass als Reaktionsprodukt ein oder mehrere 3-Oxobuttersäure-Polyglycerinester erhalten werden.

Eine erfindungsgemäß besonders bevorzugte Vorgehensweise wird durch das nachfolgende Reaktions- bzw. Syntheseschema veranschaulicht (wobei in Abhängigkeit von der Reaktionsführung entweder einzelne der Ester oder ein Gemisch von zwei oder mehreren hiervon erhalten werden):

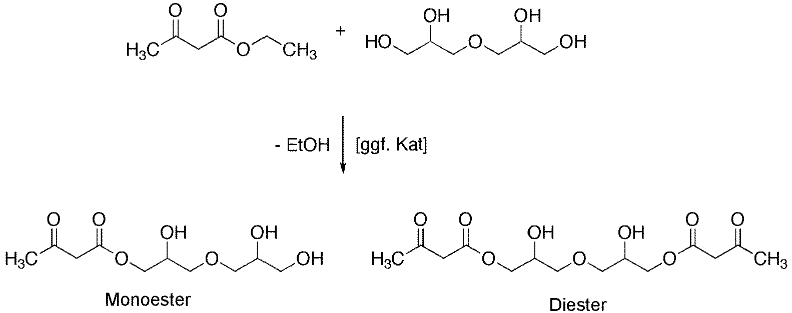

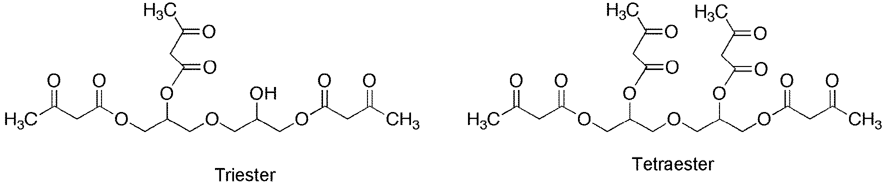

Im Rahmen des erfindungsgemäßen Verfahrens wird bei der Umsetzung gleichzeitig die Verbindung gemäß der allgemeinen Formel (IV)

R¹ - OH (IV)

gebildet wird, wobei in der allgemeinen Formel (IV) der Rest R¹ ein C₁-C₄-Alkyl, insbesondere Methyl oder Ethyl, bevorzugt Ethyl, darstellt.

In diesem Zusammenhang ist es vorgesehen, dass die Verbindung gemäß der allgemeinen Formel (IV) der Umsetzung kontinuierlich entzogen wird, insbesondere mittels vorzugsweise kontinuierlicher destillativer Entfernung. Auf diese Weise wird das Reaktionsgleichgewicht in effizienter Weise auf die Seite der Reaktionsprodukte verlagert. Auch wird auf diese Weise die Bildung von Nebenprodukten minimiert bzw. verhindert.

Im Rahmen des erfindungsgemäßen Herstellungsverfahrens kann das Reaktionsprodukt, insbesondere die Zusammensetzung des Reaktionsprodukts, insbesondere das Vorhandensein verschiedener 3-Oxobuttersäure-Polyglycerinester und deren Anteil im Fall eines Gemischs, mittels der Umsetzungsbedingungen kontrolliert und/oder gesteuert werden, insbesondere durch Auswahl der Umsetzungstemperatur (Reaktionstemperatur) und/oder Auswahl des Umsetzungsdrucks (Reaktionsdrucks) und/oder Vorsehen eines Katalysators und dessen Auswahl in Bezug auf Art und/oder Menge und/oder Auswahl der Mengen der Ausgangsverbindungen (Edukte) und/oder Vorsehen der Entfernung der Verbindung gemäß der allgemeinen Formel (IV), wie zuvor definiert.

Im Anschluss an die Umsetzung kann das erhaltene Reaktionsprodukt weiteren üblichen bzw. an sich bekannten Aufreinigungs- bzw. Aufarbeitungsschritten unterzogen werden.

In diesem Zusammenhang kann das erhaltene Reaktionsprodukt nach erfolgter Umsetzung fraktioniert werden, insbesondere destillativ fraktioniert werden.

Auch können nichtumgesetzte Ausgangsverbindungen (I) und/oder (IIb) aus dem Reaktionsprodukt abgetrennt und anschließend rezykliert werden.

Gemäß einer besonderen Ausführungsform des erfindungsgemäßen Herstellungsverfahrens kann insbesondere derart vorgegangen werden, dass im Reaktionsprodukt nach erfolgter Umsetzung noch vorhandene Hydroxylgruppen zumindest teilweise, vorzugsweise vollständig, funktionalisiert, insbesondere verestert werden. Insbesondere kann sich der Umsetzung eine teilweise, insbesondere vollständige Funktionalisierung, insbesondere Veresterung, noch vorhandener Hydroxylgruppen anschließen.

Bei dieser besonderen Ausführungsform des erfindungsgemäßen Verfahrens kann insbesondere die Funktionalisierung, insbesondere Veresterung, der Hydroxylgruppen im Reaktionsprodukt mit mindestens einem Carbonsäureanhydrid der allgemeinen Formel (V)

R³ - O - R³ (V)
wobei in der allgemeinen Formel (V) der Rest R³ jeweils unabhängig voneinander, gleich oder verschieden, einen Rest vom Typ lineares (geradkettiges) oder verzweigtes, gesättigtes oder ein- oder mehrfach ungesättigtes (C₁-C₃₃-Alkyl) - C(O) -, insbesondere (C₄-C₃₃-Alkyl) - C(O) -, bevorzugt (C₇-C₃₃-Alkyl) - C(O) -, darstellt, durchgeführt werden; und/oder
wobei in der allgemeinen Formel (V) der Rest R³ jeweils unabhängig voneinander, gleich oder verschieden, ein Fettsäurerest, insbesondere ein C₅-C₃₄-Fettsäurerest, bevorzugt ein C₈-C₃₄-Fettsäurerest, ist.

Weiterhin ist es bei dieser besonderen Ausführungsform des erfindungsgemäßen Verfahrens bevorzugt, wenn das Carbonsäureanhydrid der allgemeinen Formel (V) ein Fettsäureanhydrid, vorzugsweise ein C₅-C₃₄-Fettsäureanhydrid, bevorzugt ein C₈-C₃₄-Fettsäureanhydrid, ist.

Insbesondere ist es bei dabei bevorzugt, wenn als Carbonsäureanhydrid der allgemeinen Formel (V) eine Verbindung eingesetzt wird, bei welcher die Reste R³ identisch sind. Mit anderen Worte wird als Carbonsäureanhydrid der allgemeinen Formel (V) ein symmetrisches Carbonsäureanhydrid eingesetzt.

Gemäß einer alternativen Ausführungsform ist es bevorzugt, wenn als Carbonsäureanhydrid der allgemeinen Formel (V) eine Verbindung eingesetzt wird, bei welcher die Reste R³ voneinander verschieden sind. Mit anderen Worten wird als Carbonsäureanhydrid der allgemeinen Formel (V) ein unsymmetrisches Carbonsäureanhydrid eingesetzt.

Erfindungsgemäß kann die Funktionalisierung der nach erfolgter Umsetzung noch vorhandenen Hydroxylgruppen im Reaktionsprodukt mit dem mindestens einen Carbonsäureanhydrid der allgemeinen Formel (V) bei Temperaturen im Bereich von 60 bis 150 °C, insbesondere im Bereich von 70 bis 120 °C, vorzugsweise im Bereich von 80 bis 100 °C, durchgeführt werden.

Die erfindungsgemäße Funktionalisierung der nach erfolgter Umsetzung noch vorhandenen Hydroxylgruppen im Reaktionsprodukt mit dem mindestens einen Carbonsäureanhydrid der allgemeinen Formel (V) kann insbesondere bei einem Druck im Bereich von 0,0001 bar bis 10 bar, insbesondere im Bereich von 0,001 bar bis 5 bar, vorzugsweise im Bereich von 0,01 bar bis 2 bar, besonders bevorzugt im Bereich von 0,05 bar bis 1 bar, ganz besonders bei etwa 1 bar, durchgeführt werden.

Insbesondere ist es bevorzugt, wenn die Funktionalisierung der nach erfolgter Umsetzung noch vorhandenen Hydroxylgruppen im Reaktionsprodukt mit dem mindestens einen Carbonsäureanhydrid der allgemeinen Formel (V) in Abwesenheit von Lösemitteln und/oder ohne jedwedes Lösemittel durchgeführt wird. D. h. die Umsetzung wird also als Reaktion in Masse bzw. als Reaktion in Substanz bzw. als sogenannte *Bulk Reaction* durchgeführt. Dies hat den Vorteil, dass die erhaltenen Reaktionsprodukte nicht mit Lösemittel verunreinigt sind und kein Lösemittel nach Durchführung des Verfahrens bzw. der Reaktion aufwendig und energieträchtig entfernt und entsorgt bzw. rezykliert werden muss. Überraschenderweise verläuft das Verfahren bzw. die Reaktion dennoch mit hohen Umsätzen und Ausbeuten und zumindest im Wesentlichen ohne signifikante Nebenproduktbildung.

Im Rahmen der erfindungsgemäßen Funktionalisierung der nach erfolgter Umsetzung noch vorhandenen Hydroxylgruppen im erfindungsgemäßen Reaktionsprodukt mit dem mindestens einen Carbonsäureanhydrid der allgemeinen Formel (V) wird gleichzeitig eine Verbindung gemäß der allgemeinen Formel (VI)

R³ - OH (VI)

gebildet, wobei der Rest R³ die zuvor angegebene Bedeutung hat.

Insbesondere ist es in diesem Zusammenhang bevorzugt, wenn die Verbindung gemäß der allgemeinen Formel (VI) während oder nach erfolgter Umsetzung, insbesondere nach erfolgter Umsetzung, entfernt wird, vorzugsweise destillativ.

Im Anschluss an die erfindungsgemäßen Funktionalisierung der nach erfolgter Umsetzung noch vorhandene Hydroxylgruppen im Reaktionsprodukt mit dem mindestens einen Carbonsäureanhydrid der allgemeinen Formel (V) kann das erhaltene Produkt weiteren üblichen bzw. an sich bekannten Aufreinigungs- bzw. Aufarbeitungsschritten unterzogen werden.

In diesem Zusammenhang kann sich der Funktionalisierung insbesondere eine Destillation und/oder eine Chromatographie, vorzugsweise eine Destillation anschließen.

Insbesondere werden dabei gegebenenfalls noch vorhandene Edukte und Reaktionsnebenprodukte, insbesondere Verbindungen gemäß der allgemeinen Formel (VI), abdestilliert.

Im Rahmen der erfindungsgemäßen Funktionalisierung wird für den Fall, dass in der allgemeinen Formel (V) die Reste R³ voneinander verschieden sind, und/oder für den Fall, dass in der allgemeinen Formel (V) die Reste R³ jeweils einen Alkylrest mit mehr als zwei Kohlenstoffatomen darstellen, das Carbonsäureanhydrid der allgemeinen Formel (V) erhalten und/oder ist das Carbonsäureanhydrid der allgemeinen Formel (V) erhältlich durch die Umsetzung von Essigsäureanhydrid (Acetanhydrid) mit mindestens einer Carbonsäure, insbesondere Fettsäure, der allgemeinen Formel (VI)

R³ - OH (VI)

wobei der Rest R³ die zuvor angegebene Bedeutung hat.

In diesem Zusammenhang erfolgt die Umsetzung von Essigsäureanhydrid (Acetanhydrid) mit der mindestens einen Carbonsäure, insbesondere Fettsäure, der allgemeinen Formel (VI) insbesondere entsprechend der Reaktionsgleichung 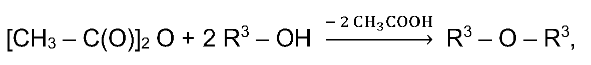 wobei der Rest R³ die zuvor angegebene Bedeutung hat, jedoch mit der Maßgabe, dass die Reste R³ voneinander verschieden sind und/oder dass die Reste R³ jeweils unabhängig voneinander, einen Alkylrest mit mehr als zwei Kohlenstoffatomen darstellen.

Gemäß einer besonderen Ausführungsform dieses erfindungsgemäßen Verfahrens wird ein symmetrisches Carbonsäureanhydrid der allgemeinen Formel (V) hergestellt. Mit anderen Worten sind in der allgemeinen Formel (V) die Reste R³ identisch und stellen einen Alkylrest mit mehr als zwei Kohlenstoffatomen dar.

Gemäß einer alternativen besonderen Ausführungsform dieses erfindungsgemäßen Verfahren wird ein unsymmetrisches Carbonsäureanhydrid der allgemeinen Formel (V) hergestellt. Somit sind in der allgemeinen Formel (V) die Reste R³ voneinander verschieden, vorzugsweise stellen in der allgemeinen Formel (V) die Reste R³ jeweils einen Alkylrest mit mehr als zwei Kohlenstoffatomen dar. Gemäß einer alternativen Ausführungsform der Funktionalisierung, insbesondere Veresterung, der nach erfolgter Umsetzung noch vorhandenen Hydroxylgruppen im Reaktionsprodukt kann diese insbesondere durch Reaktion mit mindestens einer Carbonsäure und/oder einem Carbonsäureester der allgemeinen Formel (VII)

R ³ - O - R⁴ (VII)

durchgeführt werden, wobei in der allgemeinen Formel (VII)
- der Rest R³ einen Rest vom Typ lineares (geradkettiges) oder verzweigtes, gesättigtes oder ein- oder mehrfach ungesättigtes (C₁-C₃₃-Alkyl) - C(O) -, insbesondere (C₄-C₃₃-Alkyl) - C(O) -, bevorzugt (C₇-C₃₃-Alkyl) - C(O) -, darstellt,
- der Rest R⁴ Wasserstoff oder ein C₁-C₄-Alkyl, insbesondere Methyl oder Ethyl, bevorzugt Wasserstoff, darstellt.

In diesem Zusammenhang ist es insbesondere bevorzugt, wenn die Carbonsäure und/oder der Carbonsäureester der allgemeinen Formel (VII) eine Fettsäure und/oder ein Fettsäureester, vorzugsweise eine C₅-C₃₄-Fettsäure und/oder einen C₅-C₃₄-Fettsäureester, bevorzugt eine C₈-C₃₄-Fettsäure und/oder einen C₈-C₃₄-Fettsäureester, darstellt.

Insbesondere wird bei dieser erfindungsgemäßen Ausführungsform der Funktionalisierung der nach erfolgter Umsetzung noch vorhandenen Hydroxylgruppen im Reaktionsprodukt mit mindestens einer Carbonsäure und/oder einem Carbonsäureester der allgemeinen Formel (VII) in Abwesenheit von Lösemitteln und/oder ohne jedwedes Lösemittel durchgeführt wird. D. h. die Umsetzung wird also als Reaktion in Masse bzw. als Reaktion in Substanz bzw. als sogenannte *Bulk Reaction* durchgeführt. Dies hat den Vorteil, dass die erhaltenen Reaktionsprodukte nicht mit Lösemittel verunreinigt sind und kein Lösemittel nach Durchführung des Verfahrens bzw. der Reaktion aufwendig und energieträchtig entfernt und entsorgt bzw. rezykliert werden muss. Überraschenderweise verläuft das Verfahren bzw. die Reaktion dennoch mit hohen Umsätzen und Ausbeuten und zumindest im Wesentlichen ohne signifikante Nebenproduktbildung.

Gemäß einer bevorzugten Ausführungsform wird die Funktionalisierung der nach erfolgter Umsetzung noch vorhandenen Hydroxylgruppen im Reaktionsprodukt mit mindestens einer Carbonsäure und/oder einem Carbonsäureester der allgemeinen Formel (VII) in Gegenwart eines Katalysators, insbesondere eines Enzyms und/oder eines metallhaltigen und/oder metallbasierten, sauren oder basischen Katalysators, durchgeführt.

In diesem Zusammenhang ist es insbesondere bevorzugt, wenn der Katalysators nach der Umsetzung rezykliert wird.

Wie zuvor ausgeführt, kann gemäß einer bevorzugten Ausführungsform die Funktionalisierung der nach erfolgter Umsetzung noch vorhandenen Hydroxylgruppen im Reaktionsprodukt mit mindestens einer Carbonsäure und/oder einem Carbonsäureester der allgemeinen Formel (VII) in Gegenwart eines Enzyms als Katalysator durchgeführt werden.

Dabei kann das Enzym insbesondere ausgewählt sein aus Synthetasen (Ligasen), Katalasen, Esterasen, Lipasen und deren Kombinationen. Erfindungsgemäß werden als Synthetasen (synonym Ligasen) insbesondere Enzyme aus der Klasse der Ligasen bezeichnet; Ligasen sind Enzyme, welche das Verknüpfen zweier oder mehrerer Moleküle durch eine kovalente Bindung katalysieren. Katalasen im Sinne der vorliegenden Erfindung sind insbesondere Enzyme, welche Wasserstoffperoxid zu Sauerstoff und Wasser umzusetzen imstande sind. Der Begriff der Esterasen bezeichnet insbesondere Enzyme, welche imstande sind, Ester hydrolytisch in Alkohol und Säure aufzuspalten (Verseifung); es handelt sich somit insbesondere um Hydrolasen, wobei fettspaltende Esterasen auch als Lipasen bezeichnet werden. Lipasen im Sinne der vorliegenden Erfindung sind insbesondere Enzyme, welche von Lipiden, wie Glyceriden, freie Fettsäuren abzuspalten imstande sind (Lipolyse).

Im Rahmen dieser besonderen Ausführungsform kann sich das als Katalysator eingesetzte Enzym ableitet von Candida antarctica, Mucor miehei (Rhizomucor miehei), Thermomyces lanuginosus, Candida rugosa, Aspergillus oryzae, Pseudomonas cepacia, Pseudomonas fluorescens, Rhizopus delemar und Pseudomonas sp. sowie deren Kombinationen, vorzugsweise von Candida antarctica, Mucor miehei (Rhizomucor miehei) und Thermomyces lanuginosus.

Dabei ist es insbesondere bevorzugt, wenn das Enzym in immobilisierter Form, insbesondere immobilisiert auf einem Träger, vorzugsweise auf einem polymeren Träger, bevorzugt auf einem polymeren organischen Träger, besonders bevorzugt mit hydrophoben Eigenschaften, ganz besonders bevorzugt auf einem poly(meth)acrylharzbasierten Träger, eingesetzt wird.

Wie bereits im Zusammenhang mit dem Katalysator im Allgemeinen dargelegt, ist es bevorzugt, wenn das Enzym nach der Umsetzung rezykliert wird.

Sofern die erfindungsgemäße Funktionalisierung der nach erfolgter Umsetzung noch vorhandener Hydroxylgruppen im Reaktionsprodukt mit mindestens einer Carbonsäure und/oder einem Carbonsäureester der allgemeinen Formel (VII) in Gegenwart eines Enzyms als Katalysator durchgeführt wird, ist es bevorzugt, wenn die Umsetzung bei Temperaturen im Bereich von 10 °C bis 80 °C, insbesondere im Bereich von 20 °C bis 80 °C, vorzugsweise im Bereich von 25 °C bis 75 °C, besonders bevorzugt im Bereich von 45 °C bis 75 °C, ganz besonders bevorzugt im Bereich von 50 °C bis 70 °C, durchgeführt wird.

Im Fall der Verwendung eines Enzyms als Katalysator kann die Menge des eingesetzten Enzyms in weiten Bereichen variieren. Insbesondere kann das Enzym in Mengen, bezogen auf die Gesamtmenge der Ausgangsverbindungen, im Bereich von 0,001 Gew.-% bis 20 Gew.-%, insbesondere im Bereich von 0,01 Gew.-% bis 15 Gew.-%, vorzugsweise im Bereich von 0,1 Gew.-% bis 15 Gew.-%, bevorzugt im Bereich von 0,5 Gew.-% bis 10 Gew.-%, eingesetzt werden. Dennoch kann es einzelfallbedingt oder anwendungsbezogen erforderlich sein, von den vorgenannten Mengen abzuweichen, ohne dass der Rahmen der vorliegenden Erfindung verlassen ist. In diesem Zusammenhang sind die Ausgangsverbindungen der 3-Oxobuttersäure-Polyolester (III) und die Carbonsäure und/oder der Carbonsäureester der allgemeinen Formel (VII).

Wenn gemäß einer besonderen Ausführungsform der vorliegenden Erfindung die Funktionalisierung der nach erfolgter Umsetzung noch vorhandener Hydroxylgruppen im Reaktionsprodukt mit mindestens einer Carbonsäure und/oder einem Carbonsäureester der allgemeinen Formel (VII) in Gegenwart eines Enzyms als Katalysator durchgeführt wird, kann auch der angewendete Druckbereich in weiten Bereichen variieren. Insbesondere kann bei Umsetzung in Gegenwart eines Enzyms als Katalysator die Umsetzung bei einem Druck im Bereich von 0,0001 bar bis 10 bar, insbesondere im Bereich von 0,001 bar bis 5 bar, vorzugsweise im Bereich von 0,01 bar bis 2 bar, besonders bevorzugt im Bereich von 0,05 bar bis 1 bar, ganz besonders bei etwa 1 bar, durchgeführt werden.

Gemäß einer alternativen Ausführungsform der erfindungsgemäßen Funktionalisierung der nach erfolgter Umsetzung noch vorhandener Hydroxylgruppen im Reaktionsprodukt mit mindestens einer Carbonsäure und/oder einem Carbonsäureester der allgemeinen Formel (VII) kann die Funktionalisierung in Gegenwart eines metallhaltigen und/oder metallbasierten, sauren oder basischen Katalysators durchgeführt werden.

Gemäß dieser alternativen Ausführungsform der erfindungsgemäßen Funktionalisierung, wonach die Funktionalisierung in Gegenwart eines metallhaltigen und/oder metallbasierten, sauren oder basischen Katalysators durchgeführt wird, kann der Katalysator insbesondere ausgewählt sein aus (i) basischen Katalysatoren, insbesondere Alkali- oder Erdalkalihydroxyden und Alkali- oder Erdalkalialkoholaten, wie NaOH, KOH, LiOH, Ca(OH)₂, NaOMe, KOMe und Na(OBu-tert.), (ii) sauren Katalysatoren, insbesondere Mineralsäuren, und organischen Säuren, wie Schwefelsäure, Salzsäure, Phosphorsäure, Salpetersäure, Sulfonsäuren, Methansulfonsäure, para-Toluolsulfonsäure und Carbonsäuren, (iii) Lewis-Säuren, insbesondere Lewis-Säuren auf der Basis von Titan-, Zinn-, Zink- und Aluminiumverbindungen, wie Titantetrabutylat, Zinnsäuren, Zinkacetat, Aluminiumtrichlorid und Aluminiumtri-isopropyl und (iv) heterogenen Katalysatoren, insbesondere auf der Basis von mineralischen Silikaten, Germanaten, Carbonaten und Aluminiumoxiden, wie Zeolithen, Montmorilloniten, Mordeniten, Hydrotalciten und Tonerden, sowie deren Kombinationen.

Bei dieser Ausführungsform kann als Katalysator insbesondere ein Alkali- oder Erdalkalialkoholat eingesetzt werden.

Insbesondere ist es auch bei dieser Ausführungsform der Funktionalisierung bevorzugt, wenn der Katalysator auf Basis des metallhaltigen und/oder metallbasierten sauren oder basischen Katalysators nach der Umsetzung rezykliert wird.

Wenn gemäß dieser besonderen Ausführungsform der vorliegenden Erfindung die Funktionalisierung der nach erfolgter Umsetzung noch vorhandenen Hydroxylgruppen im Reaktionsprodukt mit mindestens einer Carbonsäure und/oder einem Carbonsäureester der allgemeinen Formel (VII) in Gegenwart eines metallhaltigen und/oder metallbasierten, sauren oder basischen Katalysators erfolgt, können die Temperaturen in weiten Bereichen variiert werden. Insbesondere kann die Umsetzung in Gegenwart eines metallhaltigen und/oder metallbasierten sauren oder basischen Katalysators bei Temperaturen im Bereich von 20 °C bis 150 °C, insbesondere im Bereich von 50 °C bis 140 °C, vorzugsweise im Bereich von 70 °C bis 130 °C, besonders bevorzugt im Bereich von 80 °C bis 125 °C, ganz besonders bevorzugt im Bereich von 100 °C bis 120 °C, durchgeführt werden.

Weiterhin kann auch bei dieser Ausführungsform der Katalysator (d. h. der metallhaltige und/oder metallbasierte, saure oder basische Katalysator) in weiten Mengenbereichen variiert werden: So kann der Katalysator auf Basis eines metallhaltigen und/oder metallbasierten, sauren oder basischen Katalysators in Mengen, bezogen auf die Gesamtmenge der Ausgangsverbindungen, im Bereich von 0,01 Gew.-% bis 30 Gew.-%, insbesondere im Bereich von 0,05 Gew.-% bis 15 Gew.-%, vorzugsweise im Bereich von 0,1 Gew.-% bis 15 Gew.-%, bevorzugt im Bereich von 0,2 Gew.-% bis 10 Gew.-%, eingesetzt werden. Dennoch ist es anwendungsbezogen oder einzelfallbedingt möglich, von den vorgenannten Mengen abzuweichen, ohne dass der Rahmen der vorliegenden Erfindung verlassen ist. In diesem Zusammenhang sind die Ausgangsverbindungen der 3-Oxobuttersäure-Polyolester (III) und die Carbonsäure und/oder der Carbonsäureester der allgemeinen Formel (VII).

Wenn gemäß dieser besonderen Ausführungsform der vorliegenden Erfindung die Funktionalisierung der nach erfolgter Umsetzung noch vorhandenen Hydroxylgruppen im Reaktionsprodukt mit mindestens einer Carbonsäure und/oder einem Carbonsäureester der allgemeinen Formel (VII) in Gegenwart eines metallhaltigen und/oder metallbasierten, sauren oder basischen Katalysators erfolgt, kann der Druckbereich gleichermaßen in einem weiten Regime variieren: Insbesondere kann die Umsetzung in Gegenwart eines metallhaltigen und/oder metallbasierten, sauren oder basischen Katalysators bei einem Druck im Bereich von 0,0001 bar bis 10 bar, insbesondere im Bereich von 0,001 bar bis 5 bar, vorzugsweise im Bereich von 0,01 bar bis 2 bar, besonders bevorzugt im Bereich von 0,05 bar bis 1 bar, ganz besonders bei etwa 1 bar, durchgeführt werden.

Im Rahmen dieser besonderen Ausführungsform der Funktionalisierung der nach erfolgter Umsetzung noch vorhandenen Hydroxylgruppen im Reaktionsprodukt mit mindestens einer Carbonsäure und/oder einem Carbonsäureester der allgemeinen Formel (VII) wird gleichzeitig eine Verbindung der allgemeinen Formel (VIII)

R⁴ - OH (VIII)

gebildet, wobei der Rest R⁴ die zuvor angegebene Bedeutung hat.

Insbesondere ist es in diesem Zusammenhang bevorzugt, wenn die Verbindung der allgemeinen Formel (VIII) während oder nach erfolgter Umsetzung, insbesondere während der Umsetzung, entfernt wird, vorzugsweise destillativ. Auf diese Weise wird das Reaktionsgleichgewicht in effizienter Weise auf die Seite der Reaktionsprodukte verlagert. Auch wird auf diese Weise die Bildung von Nebenprodukten minimiert bzw. verhindert.

Gemäß einer besonderen Ausführungsform des erfindungsgemäßen Verfahrens können die in dem Reaktionsprodukt (III) durch das zuvor beschriebene Verfahren eingeführten Estergruppen einer teilweisen Umesterung mittels einer Verbindung der allgemeinen Formel (VII), wie zuvor definiert, unterzogen werden. Mit anderen Worten können die in dem Reaktionsprodukt (III) durch das zuvor beschriebene Verfahren eingeführten Estergruppen teilweise durch einen Rest R³ mit der zuvor angegebenen Bedeutung mittels Umesterung ausgetauscht werden.

In diesem Zusammenhang kann die Umesterung gemäß dieser besonderen Ausführungsform der vorliegenden Erfindung unter Reaktionsbedingungen, wie zuvor für die erfindungsgemäße Funktionalisierung der nach erfolgter Umsetzung noch vorhandenen Hydroxylgruppen im Reaktionsprodukt mit mindestens einer Carbonsäure und/oder einem Carbonsäureester, erfolgen.

Eine erfindungsgemäß besonders bevorzugte Vorgehensweise, welche eine Funktionalisierung, insbesondere Veresterung, noch vorhandener Hydroxylgruppen im Anschluss an die Umsetzung vorsieht, wird durch das nachfolgende Reaktions- bzw. Syntheseschema veranschaulicht (wobei in Abhängigkeit von der Reaktionsführung bei der Umsetzung entweder einzelne Ester oder ein Gemisch von zwei oder mehreren hiervon erhalten werden und wobei in dem nachfolgenden Reaktions- bzw. Syntheseschema der Rest R einen Rest der Formel CH₃ - (CH₂)_{x =} **₀₋₂₈** - C(O) - bezeichnet):

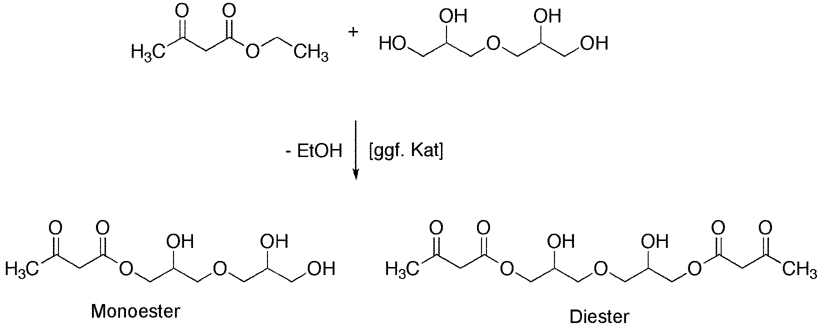

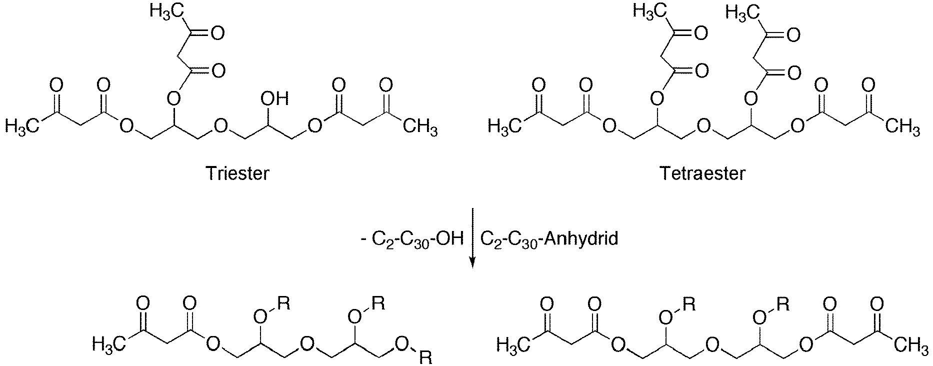

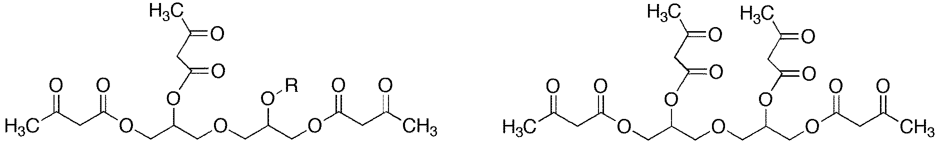

Des Weiteren betrifft die vorliegende Erfindung - gemäß einem **zweiten** Aspekt der vorliegenden Erfindung - ein Verfahren zur Herstellung von funktionalisierten, insbesondere fettsäurefunktionalisierten, Polyglycerinestern der 3-Oxobuttersäure (beta-Oxobuttersäure, 3-Oxobutansäure),
(A) wobei gemäß einer (ersten) Syntheseroute (A) zunächst in einem ersten Verfahrensschritt mindestens eine Verbindung der allgemeinen Formel (I)

   CH₃- C(O) - CH₂ - C(O)OR¹ (I)
   wobei in der allgemeinen Formel (I) der Rest R¹ ein C₁-C₄-Alkyl, insbesondere Methyl oder Ethyl, bevorzugt Ethyl, darstellt,
   mit mindestens einem Polyglycerin der allgemeinen Formel (IIb)

      HO - CH₂ - CH(OH) - CH₂ - [O - CH₂ - CH(OH) - CH₂]ₚ - OH (IIb)
   wobei in der allgemeinen Formel (IIb) die Variable p eine ganze Zahl von 1 bis 6, insbesondere 1 bis 4, vorzugsweise 1 oder 2, besonders bevorzugt 1, darstellt, umgesetzt wird,
   wobei die Umsetzung in Abwesenheit von Lösemitteln durchgeführt wird und
   wobei die Umsetzung in Gegenwart eines Katalysators durchgeführt wird,
   wobei bei der Umsetzung gleichzeitig die Verbindung gemäß der allgemeinen Formel (IV)

      R¹ - OH (IV)
   gebildet wird, wobei in der allgemeinen Formel (IV) der Rest R¹ ein C₁-C₄-Alkyl, insbesondere Methyl oder Ethyl, bevorzugt Ethyl, darstellt; wobei die Verbindung gemäß der allgemeinen Formel (IV) der Umsetzung kontinuierlich entzogen wird
   gefolgt von einem zweiten Verfahrensschritt, wobei der zweite Verfahrensschritt
      (i) eine zumindest teilweise Funktionalisierung, insbesondere eine zumindest teilweise Veresterung, noch vorhandener Hydroxylgruppen mittels mindestens einer Fettsäure und/oder deren Ester oder Anhydrid, insbesondere mittels mindestens einer C₅-C₃₄-Fettsäure und/oder deren Ester oder Anhydrid, vorzugsweise mittels mindestens einer C₈-C₃₄-Fettsäure und/oder deren Ester oder Anhydrid, und/oder
      (ii) eine teilweise Umesterung von im ersten Verfahrensschritt eingeführten Estergruppen mittels mindestens einer Fettsäure und/oder deren Ester, insbesondere mittels mindestens einer C₅-C₃₄-Fettsäure und/oder deren Ester, vorzugsweise mittels mindestens einer C₈-C₃₄-Fettsäure und/oder deren Ester,
   umfasst;
   oder aber
(B) wobei gemäß einer (zweiten, zu (A) alternativen) Syntheseroute (B) zunächst in einem ersten Verfahrensschritt mindestens einem Polyglycerin der allgemeinen Formel (IIb)

   HO - CH₂ - CH(OH) - CH₂ - [O - CH₂ - CH(OH) - CH₂]ₚ - OH (IIb)

   wobei in der allgemeinen Formel (IIb) die Variable p eine ganze Zahl von 1 bis 6, insbesondere 1 bis 4, vorzugsweise 1 oder 2, besonders bevorzugt 1, darstellt,
   mit mindestens einer Fettsäure und/oder deren Ester oder Anhydrid, insbesondere mit mindestens einer C₅-C₃₄-Fettsäure und/oder deren Ester oder Anhydrid, vorzugsweise mit mindestens einer C₈-C₃₄-Fettsäure und/oder deren Ester oder Anhydrid, umgesetzt wird,
   gefolgt von einem zweiten Verfahrensschritt, wobei der zweite Verfahrensschritt
      (i) eine zumindest teilweise Veresterung noch vorhandener Hydroxylgruppen mittels einer Verbindung der allgemeinen Formel (I), wie zuvor definiert, und/oder
      (ii) eine teilweisen Umesterung von im ersten Verfahrensschritt eingeführten Estergruppen mittels einer Verbindung der allgemeinen Formel (I), wie zuvor definiert,
   umfasst;
   so dass als Reaktionsprodukt jeweils ein oder mehrere funktionalisierte, insbesondere fettsäurefunktionalisierte, vorzugsweise C₅-C₃₄-fettsäurefunktionalisierte, bevorzugt C₈-C₃₄-fettsäurefunktionalisierte, 3-Oxobuttersäure-Polyglycerinester erhalten werden.

Insbesondere kann das erfindungsgemäße Herstellungsverfahren gemäß Syntheseroute (A) entsprechend dem zuvor beschriebenen erfindungsgemäßen Verfahren durchgeführt werden.

Entsprechend dieses Erfindungsaspekts ist es auch gemäß Syntheseroute (B) vorgesehen, dass das im Rahmen von Syntheseroute (B) des erfindungsgemäßen Verfahrens eingesetzte Polyglycerinen der allgemeinen Formel (IIb')

HO - CH₂ - CH(OH) - CH₂ - [O - CH₂ - CH(OH) - CH₂]_{q} - OH (IIb')

entspricht, wobei in der allgemeinen Formel (IIb') die Variable q eine ganze Zahl von 0 bis 6, insbesondere 0 bis 4, vorzugsweise 0 oder 1, besonders bevorzugt 1.

Gemäß einer weiteren besonderen Ausführungsform des erfindungsgemäßen Verfahrens gemäß Syntheseroute (B) kann das Polyglycerin (IIb) ein Diglycerin der Formel (IIc)

HO - CH₂ - CH(OH) - CH₂ - O - CH₂ - CH(OH) - CH₂ - OH (IIc)

sein.

Gemäß einer alternativen besonderen Ausführungsform des erfindungsgemäßen Verfahrens gemäß Syntheseroute (B) ist das Polyglycerin (IIb) kein Propan-1,2,3-triol (Glycerin).

Was die im Rahmen des erfindungsgemäßen Verfahrens gemäß Syntheseroute (B) einsetzbare Fettsäure und/oder Fettsäureester anbelangt, so ist es insbesondere bevorzugt, wenn die Fettsäure und/oder der Fettsäureester eine Carbonsäure und/oder ein Carbonsäureester der allgemeinen Formel (VII)

R³ - O - R⁴ (VII)

ist, wobei in der allgemeinen Formel (VII)
- der Rest R³ einen Rest vom Typ lineares (geradkettiges) oder verzweigtes, gesättigtes oder ein- oder mehrfach ungesättigtes (C₁-C₃₃-Alkyl) - C(O) -, insbesondere (C₄-C₃₃-Alkyl) - C(O) -, bevorzugt (C₇-C₃₃-Alkyl) - C(O) -, darstellt,
- der Rest R⁴ Wasserstoff oder ein C₁-C₄-Alkyl, insbesondere Wasserstoff, Methyl oder Ethyl, besonders bevorzugt Wasserstoff, darstellt.

Was weiterhin das im Rahmen des erfindungsgemäßen Verfahrens gemäß Syntheseroute (B) einsetzbare Fettsäureanhydrid anbelangt, so ist es insbesondere bevorzugt, wenn das Fettsäureanhydrid ein Carbonsäureanhydrid der allgemeinen Formel (V)

R³ - O - R³ (V)

ist, wobei in der allgemeinen Formel (V) der Rest R³ einen Rest vom Typ lineares (geradkettiges) oder verzweigtes, gesättigtes oder ein- oder mehrfach ungesättigtes (C₁-C₃₃-Alkyl) - C(O) -, insbesondere (C₄-C₃₃-Alkyl) - C(O) -, bevorzugt (C₇-C₃₃-Alkyl) - C(O) -, darstellt.

Insbesondere wird bei dem erfindungsgemäßen Verfahren gemäß Syntheseroute (B) im ersten Verfahrensschritt die Umsetzung in Abwesenheit von Lösemitteln und/oder ohne jedwedes Lösemittel durchgeführt. Wie zuvor ausgeführt heißt das, dass die Umsetzung wird also als Reaktion in Masse bzw. als Reaktion in Substanz bzw. als sogenannte *Bulk Reaction* durchgeführt. Dies hat den Vorteil, dass die erhaltenen Reaktionsprodukte nicht mit Lösemittel verunreinigt sind und kein Lösemittel nach Durchführung des Verfahrens bzw. der Reaktion aufwendig und energieträchtig entfernt und entsorgt bzw. rezykliert werden muss. Überraschenderweise verläuft das Verfahren bzw. die Reaktion dennoch mit hohen Umsätzen und Ausbeuten und zumindest im Wesentlichen ohne signifikante Nebenproduktbildung.

Gemäß einer besonderen Ausführungsform des erfindungsgemäßen Verfahrens gemäß Syntheseroute (B) kann im ersten Verfahrensschritt die Umsetzung im Fall der Verwendung von Fettsäure und/oder deren Ester als Edukt in Gegenwart eines Katalysators, insbesondere eines metallhaltigen und/oder metallbasierten, sauren oder basischen Katalysators, durchgeführt werden.

In diesem Zusammenhang ist es insbesondere bevorzugt, wenn der Katalysators nach der Umsetzung rezykliert wird.

Alternativ zu dieser besonderen Ausführungsform wird gemäß Syntheseroute (B) die Umsetzung im Fall der Verwendung von Fettsäureanhydrid als Edukt in Abwesenheit eines Katalysators und/oder ohne einen Katalysator durchgeführt wird.

Wie zuvor ausgeführt, kann gemäß einer besonderen Ausführungsform des erfindungsgemäßen Herstellungsverfahrens gemäß Syntheseroute (B) im ersten Verfahrensschritt die Umsetzung im Fall der Verwendung von Fettsäure und/oder deren Ester als Edukt in Gegenwart eines metallhaltigen und/oder metallbasierten, sauren oder basischen Katalysators durchgeführt werden.

Dabei kann der Katalysator ausgewählt sein aus (i) basischen Katalysatoren, insbesondere Alkali- oder Erdalkalihydroxyden und Alkali- oder Erdalkalialkoholaten, wie NaOH, KOH, LiOH, Ca(OH)₂, NaOMe, KOMe und Na(OBu-tert.), (ii) sauren Katalysatoren, insbesondere Mineralsäuren, und organischen Säuren, wie Schwefelsäure, Salzsäure, Phosphorsäure, Salpetersäure, Sulfonsäuren, Methansulfonsäure, para-Toluolsulfonsäure und Carbonsäuren, (iii) Lewis-Säuren, insbesondere Lewis-Säuren auf der Basis von Titan-, Zinn-, Zink- und Aluminiumverbindungen, wie Titantetrabutylat, Zinnsäuren, Zinkacetat, Aluminiumtrichlorid und Aluminiumtri-isopropyl und (iv) heterogenen Katalysatoren, insbesondere auf der Basis von mineralischen Silikaten, Germanaten, Carbonaten und Aluminiumoxiden, wie Zeolithen, Montmorilloniten, Mordeniten, Hydrotalciten und Tonerden, sowie deren Kombinationen.

Bei dieser Ausführungsform kann als Katalysator insbesondere ein Alkali- oder Erdalkalialkoholat eingesetzt werden.

Insbesondere ist es in diesem Zusammenhang, wie zuvor bereits erwähnt, bevorzugt, wenn der Katalysator nach der Umsetzung rezykliert wird.

Wenn gemäß der besonderen Ausführungsform die Umsetzung in Gegenwart eines metallhaltigen und/oder metallbasierten, sauren oder basischen Katalysators durchgeführt ist, ist es bevorzugt, wenn die Umsetzung bei Temperaturen im Bereich von 20 °C bis 150 °C, insbesondere im Bereich von 50 °C bis 140 °C, vorzugsweise im Bereich von 70 °C bis 130 °C, besonders bevorzugt im Bereich von 80 °C bis 125 °C, ganz besonders bevorzugt im Bereich von 100 °C bis 120 °C, durchgeführt wird.

Insbesondere ist es gemäß dieser Ausführungsform auch bevorzugt, wenn der Katalysator in Mengen, bezogen auf die Gesamtmenge der Ausgangsverbindungen, im Bereich von 0,01 Gew.-% bis 30 Gew.-%, insbesondere im Bereich von 0,05 Gew.-% bis 15 Gew.-%, vorzugsweise im Bereich von 0,1 Gew.-% bis 15 Gew.-%, bevorzugt im Bereich von 0,2 Gew.-% bis 10 Gew.-%, eingesetzt wird.

Weiterhin ist es gemäß dieser Ausführungsform bevorzugt, wenn die Umsetzung in Gegenwart eines metallhaltigen und/oder metallbasierten, sauren oder basischen Katalysators bei einem Druck im Bereich von 0,0001 bar bis 10 bar, insbesondere im Bereich von 0,001 bar bis 5 bar, vorzugsweise im Bereich von 0,01 bar bis 2 bar, besonders bevorzugt im Bereich von 0,05 bar bis 1 bar, ganz besonders bei etwa 1 bar, durchgeführt wird.

Im Rahmen des erfindungsgemäßen Herstellungsverfahrens gemäß Syntheseroute (B) wird im Fall der Verwendung von Fettsäure und/oder deren Ester als Edukt bei der Umsetzung gleichzeitig die Verbindung der allgemeinen Formel (VIII)

R⁴ - OH (VIII)

gebildet, wobei der Rest R⁴ die zuvor angegebene Bedeutung hat.

In diesem Zusammenhang ist es insbesondere bevorzugt, wenn die Verbindung der allgemeinen Formel (VIII) während oder nach erfolgter Umsetzung, insbesondere während der Umsetzung, entfernt wird, vorzugsweise destillativ.

Im Rahmen des erfindungsgemäßen Herstellungsverfahrens gemäß Syntheseroute (B) wird im Fall der Verwendung von Fettsäureanhydrid als Edukt bei der Umsetzung gleichzeitig die Verbindung der allgemeinen Formel (VI)

R³ - OH (VI)

gebildet, wobei der Rest R³ die zuvor angegebene Bedeutung hat.

Dabei ist es insbesondere bevorzugt, wenn die Verbindung der allgemeinen Formel (VI) während oder nach erfolgter Umsetzung, entfernt wird, vorzugsweise destillativ.

Insbesondere wird bei dem erfindungsgemäßen Verfahren gemäß Syntheseroute (B) auch der zweite Verfahrensschritt in Abwesenheit von Lösemitteln und/oder ohne jedwedes Lösemittel durchgeführt.

Weiterhin wird bei dem erfindungsgemäßen Verfahren gemäß Syntheseroute (B) auch der zweite Verfahrensschritt in Gegenwart eines Katalysators, insbesondere eines metallhaltigen und/oder metallbasierten, sauren oder basischen Katalysators, durchgeführt.

Auch in diesem Zusammenhang ist es bevorzugt, wenn der Katalysators nach der Umsetzung rezykliert wird.

Gemäß einer wiederum besonderen Ausführungsform der vorliegen Erfindung ist es bevorzugt, wenn das Herstellungsverfahren gemäß Syntheseroute (B) auch im zweiten Verfahrensschritt in Gegenwart eines metallhaltigen und/oder metallbasierten, sauren oder basischen Katalysators durchgeführt wird.

Insbesondere ist es auch gemäß dieser besonderen Ausführungsform bevorzugt, wenn der Katalysator ausgewählt ist aus (i) basischen Katalysatoren, insbesondere Alkali- oder Erdalkalihydroxyden und Alkali- oder Erdalkalialkoholaten, wie NaOH, KOH, LiOH, Ca(OH)₂, NaOMe, KOMe und Na(OBu-tert.), (ii) sauren Katalysatoren, insbesondere Mineralsäuren, und organischen Säuren, wie Schwefelsäure, Salzsäure, Phosphorsäure, Salpetersäure, Sulfonsäuren, Methansulfonsäure, para-Toluolsulfonsäure und Carbonsäuren, (iii) Lewis-Säuren, insbesondere Lewis-Säuren auf der Basis von Titan-, Zinn-, Zink- und Aluminiumverbindungen, wie Titantetrabutylat, Zinnsäuren, Zinkacetat, Aluminiumtrichlorid und Aluminiumtri-isopropyl und (iv) heterogenen Katalysatoren, insbesondere auf der Basis von mineralischen Silikaten, Germanaten, Carbonaten und Aluminiumoxiden, wie Zeolithen, Montmorilloniten, Mordeniten, Hydrotalciten und Tonerden, sowie deren Kombinationen.

In diesem Zusammenhang wird als Katalysator insbesondere ein Alkali- oder Erdalkalialkoholat eingesetzt.

Wie zuvor ausgeführt ist es insbesondere vorteilhaft, wenn der Katalysator nach der Veresterung und/oder Umesterung rezykliert wird.

Gemäß dieser besonderen Ausführungsform des erfindungsgemäßen Verfahrens können die Temperaturen in weiten Bereichen variieren. Insbesondere kann gemäß Syntheseroute (B) der zweiten Verfahrensschritt in Gegenwart eines metallhaltigen und/oder metallbasierten, sauren oder basischen Katalysators bei Temperaturen im Bereich von 20 °C bis 150 °C, insbesondere im Bereich von 50 °C bis 140 °C, vorzugsweise im Bereich von 70 °C bis 130 °C, besonders bevorzugt im Bereich von 80 °C bis 125 °C, ganz besonders bevorzugt im Bereich von 100 °C bis 120 °C, durchgeführt werden.

Weiterhin kann auch die Menge des Katalysator gemäß dieser besonderen Ausführungsform variiert werden: So kann der Katalysator in Mengen, bezogen auf die Gesamtmenge der Ausgangsverbindungen, im Bereich von 0,01 Gew.-% bis 30 Gew.-%, insbesondere im Bereich von 0,05 Gew.-% bis 15 Gew.-%, vorzugsweise im Bereich von 0,1 Gew.-% bis 15 Gew.-%, bevorzugt im Bereich von 0,2 Gew.-% bis 10 Gew.-%, eingesetzt werden.

Wenn gemäß dieser besonderen Ausführungsform der vorliegenden Erfindung gemäß Syntheseroute (B) der zweite Verfahrensschritt in Gegenwart eines metallhaltigen und/oder metallbasierten, sauren oder basischen Katalysators erfolgt, kann der Druckbereich gleichermaßen in einem weiten Regime variieren: Insbesondere kann der zweite Verfahrensschritt in Gegenwart eines metallhaltigen und/oder metallbasierten, sauren oder basischen Katalysators bei einem Druck im Bereich von 0,0001 bar bis 10 bar, insbesondere im Bereich von 0,001 bar bis 5 bar, vorzugsweise im Bereich von 0,01 bar bis 2 bar, besonders bevorzugt im Bereich von 0,05 bar bis 1 bar, ganz besonders bei etwa 1 bar, durchgeführt werden.

Im Rahmen dieser besonderen Ausführungsform des erfindungsgemäßen Verfahrens wird gemäß Syntheseroute (B) im zweiten Verfahrensschritt gleichzeitig die Verbindung der allgemeinen Formel (IX)

R¹ - O - R⁵ (IX)

gebildet, wobei in der allgemeinen Formel (IX)
- der Rest R¹ ein C₁-C₄-Alkyl, insbesondere Methyl oder Ethyl, bevorzugt Ethyl, darstellt,
- der Rest R⁵ jeweils unabhängig voneinander, gleich oder verschieden, Wasserstoff oder einen Rest R³, wie zuvor definiert, darstellt.

In diesem Zusammen ist es insbesondere vorgesehen, dass die Verbindung der allgemeinen Formel (IX) während oder nach erfolgter Umsetzung, insbesondere während der Umsetzung, entfernt wird, vorzugsweise destillativ.

Bei den erfindungsgemäßen Verfahren gemäß dem ersten und zweiten Erfindungsaspekt kann bei der Funktionalisierung bzw. Veresterung bzw. Umesterung insbesondere eine Fettsäure oder deren Derivat, insbesondere Ester oder Anhydrid, eingesetzt werden.

Erfindungsgemäß ist es insbesondere vorgesehen, dass die Fettsäure, vorzugsweise die C₅-C₃₄-Fettsäure, insbesondere die C₈-C₃₄-Fettsäure, insbesondere in freier Form oder in Form von deren Ester oder Anhydrid, ausgewählt ist aus der Gruppe von Caprylsäure, Pelargonsäure, Caprinsäure, Undecansäure, Laurinsäure, Tridecansäure, Myristinsäure, Pentadecansäure, Palmitinsäure, Margarinsäure, Stearinsäure, Nonadecansäure, Arachinsäure, Heneicosansäure, Behensäure, Lignocerinsäure, Cerotinsäure, Montansäure, Melissinsäure, Laccersäure, Geddinsäure, Undecylsäure, Myristoleinsäure, Palmitoleinsäure, Margaroleinsäure, Petroselinsäure, Ölsäure, Elaidinsäure, Vaccensäure, Gadoleinsäure, Cetoleinsäure, Erucasäure, Nervonsäure, Linolsäure, Linolensäuren, Calendulasäure, Punicinsäure, Eleostearinsäuren, Stearidonsäure, Arachidonsäure, Eicosapentaensäure, Docosadiensäure, Docosatetraensäure, Docosapentaensäure, Docosahexaensäure, und Tetracosahexaensäure, sowie deren Mischungen.

Gemäß einer besonderen Ausführungsform der vorliegenden Erfindung ist es bevorzugt, wenn die Fettsäure, vorzugsweise die C₅-C₃₄-Fettsäure, insbesondere die C₈-C₃₄-Fettsäure, insbesondere in freier Form oder in Form von deren Ester oder Anhydrid, ausgewählt ist aus der Gruppe von Myristinsäure, Pentadecansäure, Palmitoleinsäure, Cetoleinsäure, Ölsäure, Gadoleinsäure, Cetoleinsäure, Erucasäure, Arachidonsäure, Eicosapentaensäure, Docosadiensäure, Docosatetraensäure, Docosapentaensäure, Docosahexaensäure, Tetracosahexaensäure, sowie deren Mischungen, vorzugsweise Eicosapentaensäure und Docosahexaensäure, sowie deren Mischungen.

Gemäß einer weiteren besonderen Ausführungsform ist die Fettsäure, vorzugsweise die C₅-C₃₄-Fettsäure, insbesondere die C₈-C₃₄-Fettsäure, insbesondere in freier Form oder in Form von deren Ester oder Anhydrid, ausgewählt ist aus der Gruppe von fischölbasierten und/oder in Fischölen vorkommenden Fettsäuren, insbesondere Eicosapentaensäure, Docosadiensäure, Docosatetraensäure, Docosapentaensäure, Docosahexaensäure und Tetracosahexaensäure, sowie deren Mischungen, vorzugsweise Eicosapentaensäure, Docosahexaensäure, sowie deren Mischungen.

Im Rahmen des bzw. der erfindungsgemäßen Verfahren gemäß den beiden vorgenannten Erfindungsaspekten lassen sich als Reaktionsprodukte ein oder mehrere gegebenenfalls funktionalisierte, vorzugsweise gegebenenfalls fettsäurefunktionalisierte, bevorzugt gegebenenfalls C₅-C₃₄-fettsäurefunktionalisierte, vorzugsweise gegebenenfalls C₈-C₃₄-fettsäurefunktionalisierte, 3-Oxobuttersäurepolyglycerinester erhalten.

Insbesondere lassen sich im Rahmen des erfindungsgemäßen Verfahrens gemäß dem ersten Erfindungsaspekt als Reaktionsprodukt ein oder mehrere 3-Oxobuttersäurepolyglycerinester erhalten.

Des Weiteren lassen sich im Rahmen des erfindungsgemäßen Verfahrens gemäß dem ersten oder zweiten Erfindungsaspekt als Reaktionsprodukt ein oder mehrere funktionalisierte, insbesondere fettsäurefunktionalisierte, bevorzugt C₅-C₃₄-fettsäurefunktionalisierte, besonders bevorzugt C₈-C₃₄-fettsäurefunktionalisierte, 3-Oxobuttersäurepolyglycerinester erhalten.

Weiterhin betrifft die vorliegende Erfindung - gemäß einem **dritten** Aspekt der vorliegenden Erfindung - ein gegebenenfalls funktionalisierter 3-Oxobuttersäure-Polyglcerinester oder Gemisch von mindestens zwei voneinander verschiedenen gegebenenfalls funktionalisierten 3-Oxobuttersäure-Polyglcerinestern, erhältlich gemäß dem Verfahren nach der vorliegenden Erfindung. Insbesondere handelt es sich dabei um ein oder mehrere, gegebenenfalls funktionalisierte, insbesondere gegebenenfalls fettsäurefunktionalisierte, vorzugsweise gegebenenfalls C₅-C₃₄-fettsäurefunktionalisierte, bevorzugt gegebenenfalls C₈-C₃₄-fettsäurefunktionalisierte, Polyglycerinester der 3-Oxobuttersäure (beta-Oxobuttersäure bzw. 3-Oxobutansäure) bzw. diesbezügliche Gemische hiervon.

Gemäß einer wiederum besonderen Ausführungsform der vorliegenden Erfindung kann das Reaktionsprodukt ein oder mehrere gegebenenfalls funktionalisierte, vorzugsweise gegebenenfalls fettsäurefunktionalisierte, insbesondere gegebenenfalls C₅-C₃₄-fettsäurefunktionalisierte, bevorzugt gegebenenfalls C₈-C₃₄-fettsäurefunktionalisierte, 3-Oxobuttersäurepolyglycerinester der allgemeinen Formel (IIIb₁")

R⁶O - CH₂ - CH(OR⁶) - CH₂ - [O - CH₂ - CH(OR⁶) - CH₂]ₚ - OR⁶ (IIIb₁")

umfassen,
wobei in der allgemeinen Formel (IIIb₁")
- die Variable p eine ganze Zahl von 1 bis 6, insbesondere 1 bis 4, vorzugsweise 1 oder 2, besonders bevorzugt 1, darstellt,
- der Rest R⁶ jeweils unabhängig voneinander, gleich oder verschieden, darstellt: Wasserstoff, CH₃ - C(O) - CH₂ - C(O) - oder einen Rest R³, wobei der Rest R³ einen Rest vom Typ lineares (geradkettiges) oder verzweigtes, gesättigtes oder ein- oder mehrfach ungesättigtes (C₁-C₃₃-Alkyl) - C(O) -, insbesondere (C₄-C₃₃-Alkyl) - C(O) -, bevorzugt (C₇-C₃₃-Alkyl) - C(O) -, darstellt, jedoch mit der Maßgabe, dass mindestens ein Rest R⁶, insbesondere mindestens zwei Reste R⁶, keinen Wasserstoff darstellt, und mit der Maßgabe, dass mindestens ein Rest R⁶, insbesondere mindestens zwei Reste R⁶, CH₃- C(O) - CH₂ - C(O) - darstellt.

Gemäß einer wiederum weiteren besonderen Ausführungsform der vorliegenden Erfindung kann das Reaktionsprodukt ein oder mehrere gegebenenfalls funktionalisierte, vorzugsweise gegebenenfalls fettsäurefunktionalisierte, insbesondere gegebenenfalls C₅-C₃₄-fettsäurefunktionalisierte, bevorzugt gegebenenfalls C₈-C₃₄-fettsäurefunktionalisierte, 3-Oxobuttersäurepolyglycerinester der allgemeinen Formel (IIIc")

R⁶O - CH₂ - CH(OR⁶) - CH₂ - O - CH₂ - CH(OR⁶) - CH₂ - OR⁶ (IIIc")

umfassen,
wobei in der allgemeinen Formel (IIIc") der Rest R⁶ jeweils unabhängig voneinander, gleich oder verschieden, darstellt: Wasserstoff, CH₃ - C(O) - CH₂ - C(O) - oder einen Rest R³, wobei der Rest R³ einen Rest vom Typ lineares (geradkettiges) oder verzweigtes, gesättigtes oder ein- oder mehrfach ungesättigtes (C₁-C₃₃-Alkyl) - C(O) -, insbesondere (C₄-C₃₃-Alkyl) - C(O) -, bevorzugt (C₇-C₃₃-Alkyl) - C(O) -, darstellt, jedoch mit der Maßgabe, dass mindestens ein Rest R⁶, insbesondere mindestens zwei Reste R⁶, keinen Wasserstoff darstellt, und mit der Maßgabe, dass mindestens ein Rest R⁶, insbesondere mindestens zwei Reste R⁶, CH₃ - C(O) - CH₂ - C(O) - darstellt.

Gemäß einer besonderen Ausführungsform der vorliegenden Erfindung kann das Reaktionsprodukt insbesondere ein Gemisch von mindestens zwei voneinander verschiedenen gegebenenfalls funktionalisierten, vorzugweise gegebenenfalls fettsäurefunktionalisierten, insbesondere gegebenenfalls C₅-C₃₄-fettsäurefunktionalisierten, bevorzugt gegebenenfalls C₈-C₃₄-fettsäurefunktionalisierten, 3-Oxobuttersäure-Polyglycerinestern, insbesondere wie zuvor definiert, umfassen.

Gemäß einer weiteren besonderen Ausführungsform der vorliegenden Erfindung kann das Reaktionsprodukt insbesondere ein Gemisch von mindestens drei voneinander verschiedenen gegebenenfalls funktionalisierten, vorzugsweise gegebenenfalls fettsäurefunktionalisierten, insbesondere gegebenenfalls C₅-C₃₄-fettsäurefunktionalisierten, bevorzugt gegebenenfalls C₈-C₃₄-fettsäurefunktionalisierten, 3-Oxobuttersäure-Polyglycerinestern, insbesondere wie zuvor definiert, umfassen.

Für den Fall, dass keine Funktionalisierung bzw. Veresterung bzw. Umesterung durchgeführt wird, werden als Reaktionsprodukt (d. h. (chemisches) Produkt oder Produktgemisch) ein oder mehrere 3-Oxobuttersäurepolyglycerinester erhalten.

Insbesondere kann das Reaktionsprodukt ein oder mehrere 3-Oxobuttersäure-polyglycerinester der allgemeinen Formel (IIIb)

R²O - CH₂ - CH(OR²) - CH₂ - [O - CH₂ - CH(OR²) - CH₂]ₚ - OR² (IIIb)

umfassen,
wobei in der allgemeinen Formel (IIIb)
- die Variable p eine ganze Zahl von 1 bis 6, insbesondere 1 bis 4, vorzugsweise 1 oder 2, besonders bevorzugt 1, darstellt,
- der Rest R² jeweils unabhängig voneinander, gleich oder verschieden, darstellt: Wasserstoff oder CH₃ - C(O) - CH₂ - C(O) -, jedoch mit der Maßgabe, dass mindestens ein Rest R², insbesondere mindestens zwei Reste R², keinen Wasserstoff darstellt.

Gemäß einer besonderen Ausführungsform der vorliegenden Erfindung kann das Reaktionsprodukt ein oder mehrere 3-Oxobuttersäurepolyglycerinester der allgemeinen Formel (IIIc)

R²O - CH₂ - CH(OR²) - CH₂ - O - CH₂ - CH(OR²) - CH₂ - OR² (IIIc)

umfassen,
wobei in der allgemeinen Formel (IIIc) der Rest R² jeweils unabhängig voneinander, gleich oder verschieden, Wasserstoff oder CH₃ - C(O) - CH₂ -C(O) -, darstellt, jedoch mit der Maßgabe, dass mindestens ein Rest R², insbesondere mindestens zwei Reste R², keinen Wasserstoff darstellt.

Gemäß einer besonderen Ausführungsform kann das Reaktionsprodukt insbesondere ein Gemisch von mindestens zwei voneinander verschiedenen 3-Oxobuttersäure-Polyglycerinestern, insbesondere wie zuvor definiert, umfassen.

Insbesondere kann das Gemisch ein gewichtsbezogenes Verhältnis von monoglyceridischen Estern/di- und höherglyceridischen Estern im Bereich von 15-70 / 30-85, insbesondere im Bereich von 20-65 / 35-80, aufweisen.

Gemäß einer weiteren besonderen Ausführungsform kann das Reaktionsprodukt insbesondere ein Gemisch von mindestens drei voneinander verschiedenen 3-Oxobuttersäure-Polyglycerinestern, insbesondere wie zuvor definiert, umfassen.

Insbesondere kann das Gemisch ein gewichtsbezogenes Verhältnis von monoglyceridischen Estern/diglyceridischen Estern/drei- und höherglyceridischen Estern im Bereich von 15-50 / 35-60 / 1-35, insbesondere im Bereich von 20-45 / 40-55 / 3-30, aufweisen.

Für den Fall, dass eine Funktionalisierung bzw. Veresterung bzw. Umesterung durchgeführt wird, werden als Reaktionsprodukt (d. h. (chemisches) Produkt oder Produktgemisch) ein oder mehrere funktionalisierte, insbesondere fettsäurefunktionalisierte, bevorzugt C₅-C₃₄-fettsäurefunktionalisierte, besonders bevorzugt C₈-C₃₄-fettsäurefunktionalisierte, 3-Oxobuttersäurepolyglycerinester erhalten. Gemäß einer besonderen Ausführungsform der vorliegenden Erfindung kann das Reaktionsprodukt ein oder mehrere funktionalisierte, insbesondere fettsäurefunktionalisierte, bevorzugt C₅-C₃₄-fettsäurefunktionalisierte, besonders bevorzugt C₈-C₃₄-fettsäurefunktionalisierte, 3-Oxobuttersäurepolyglycerinester der allgemeinen Formel (IIIb₁')

R⁶O - CH₂ - CH(OR⁶) - CH₂ - [O - CH₂ - CH(OR⁶) - CH₂]ₚ - OR⁶ (IIIb₁')

umfassen,
wobei in der allgemeinen Formel (IIIb₁')
- die Variable p eine ganze Zahl von 1 bis 6, insbesondere 1 bis 4, vorzugsweise 1 oder 2, besonders bevorzugt 1, darstellt,
- der Rest R⁶ jeweils unabhängig voneinander, gleich oder verschieden, darstellt: Wasserstoff, CH₃- C(O) - CH₂ - C(O) - oder einen Rest R³, wie zuvor definiert, jedoch mit der Maßgabe, dass mindestens zwei Reste R⁶ keinen Wasserstoff darstellen, und mit der Maßgabe, dass mindestens ein Rest R⁶, insbesondere mindestens zwei Reste R⁶, CH₃ - C(O) - CH₂ - C(O) - darstellt, und mit der Maßgabe, dass mindestens ein Rest R⁶, insbesondere mindestens zwei Reste R⁶, einen Rest R³, wie zuvor definiert, darstellt.

Insbesondere kann in der allgemeinen Formel (IIIb₁') der Rest R⁶ jeweils unabhängig voneinander, gleich oder verschieden, darstellen: CH₃- C(O) - CH₂ - C(O) - oder einen Rest R³, wie zuvor definiert, jedoch mit der Maßgabe, dass mindestens ein Rest R⁶, insbesondere mindestens zwei Reste R⁶, CH₃- C(O) - CH₂ - C(O) - darstellt, und mit der Maßgabe, dass mindestens ein Rest R⁶ einen Rest R³, wie zuvor definiert, darstellt.

Dabei ist es insbesondere bevorzugt, wenn in der allgemeinen Formel (IIIb₁') der Rest R⁶ jeweils unabhängig voneinander, gleich oder verschieden, keinen Wasserstoff darstellt.

Gemäß einer weiteren besonderen Ausführungsform der vorliegenden Erfindung kann das Reaktionsprodukt ein oder mehrere funktionalisierte, insbesondere fettsäurefunktionalisierte, bevorzugt C₅-C₃₄-fettsäurefunktionalisierte, besonders bevorzugt C₈-C₃₄-fettsäurefunktionalisierte, 3-Oxobuttersäurepolyglycerinester der allgemeinen Formel (IIIc')

R⁶O - CH₂ - CH(OR⁶) - CH₂ - O - CH₂ - CH(OR⁶) - CH₂ - OR⁶ (IIIc')

umfassen,
wobei in der allgemeinen Formel (IIIc') der Rest R⁶ jeweils unabhängig voneinander, gleich oder verschieden, darstellt: Wasserstoff, CH₃ - C(O) - CH₂ - C(O) - oder einen Rest R³, wie zuvor definiert, jedoch mit der Maßgabe, dass mindestens zwei Reste R⁶ keinen Wasserstoff darstellen, und mit der Maßgabe, dass mindestens ein Rest R⁶, insbesondere mindestens zwei Reste R⁶, CH₃-C(O) - CH₂ - C(O) - darstellt, und mit der Maßgabe, dass mindestens ein Rest R⁶, insbesondere mindestens zwei Reste R⁶, einen Rest R³, wie zuvor definiert, darstellt.

Insbesondere kann in der allgemeinen Formel (IIIc') der Rest R⁶ jeweils unabhängig voneinander, gleich oder verschieden, darstellen: CH₃ - C(O) - CH₂ -C(O) - oder einen Rest R³, wie zuvor definiert, jedoch mit der Maßgabe, dass mindestens ein Rest R⁶, insbesondere mindestens zwei Reste R⁶, CH₃ - C(O) - CH₂ - C(O) - darstellt, und mit der Maßgabe, dass mindestens ein Rest R⁶ einen Rest R³, wie zuvor definiert, darstellt.

Dabei ist es insbesondere bevorzugt, wenn in der allgemeinen Formel (IIIc') der Rest R⁶ jeweils unabhängig voneinander, gleich oder verschieden, keinen Wasserstoff darstellt.

Gemäß einer besonderen Ausführungsform kann das Reaktionsprodukt insbesondere ein Gemisch von mindestens zwei voneinander verschiedenen funktionalisierten, insbesondere fettsäurefunktionalisierte, bevorzugt C₅-C₃₄-fettsäurefunktionalisierte, besonders bevorzugt C₈-C₃₄-fettsäurefunktionalisierten, 3-Oxobuttersäure-Polyglycerinestern, insbesondere wie zuvor definiert, umfassen.

Gemäß einer weiteren besonderen Ausführungsform kann das Reaktionsprodukt insbesondere ein Gemisch von mindestens drei voneinander verschiedenen funktionalisierte, insbesondere fettsäurefunktionalisierte, bevorzugt C₅-C₃₄-fettsäurefunktionalisierte, besonders bevorzugt C₈-C₃₄-fettsäurefunktionalisierte, 3-Oxobuttersäure-Polyglycerinestern, insbesondere wie zuvor definiert, umfassen.

Wiederum weiterer Gegenstand der vorliegenden Erfindung ist ebenfalls ein gegebenenfalls funktionalisierter 3-Oxobuttersäure-Polyolester (III"), insbesondere wie zuvor definiert,
wobei der gegebenenfalls funktionalisierte, vorzugsweise gegebenenfalls fettsäurefunktionalisierte, insbesondere gegebenenfalls C₅-C₃₄-fettsäurefunktionalisierte, bevorzugt gegebenenfalls C₈-C₃₄-fettsäurefunktionalisierte, 3-Oxobuttersäurepolyglycerinester der allgemeinen Formel (IIIb₁")

   R⁶O - CH₂ - CH(OR⁶) - CH₂ - [O - CH₂ - CH(OR⁶) - CH₂]ₚ - OR⁶ (IIIb₁")
entspricht,
wobei in der allgemeinen Formel (IIIb₁")

- die Variable p eine ganze Zahl von 1 bis 6, insbesondere 1 bis 4, vorzugsweise 1 oder 2, besonders bevorzugt 1, darstellt,
- der Rest R⁶ jeweils unabhängig voneinander, gleich oder verschieden, darstellt: Wasserstoff, CH₃ - C(O) - CH₂ - C(O) - oder einen Rest R³, wobei der Rest R³ einen Rest vom Typ lineares (geradkettiges) oder verzweigtes, gesättigtes oder ein- oder mehrfach ungesättigtes (C₁-C₃₃-Alkyl) - C(O) -, insbesondere (C₄-C₃₃-Alkyl) - C(O) -, bevorzugt (C₇-C₃₃-Alkyl) - C(O) -, darstellt, jedoch mit der Maßgabe, dass mindestens ein Rest R⁶, insbesondere mindestens zwei Reste R⁶, keinen Wasserstoff darstellt, und mit der Maßgabe, dass mindestens ein Rest R⁶, insbesondere mindestens zwei Reste R⁶, CH₃ - C(O) - CH₂ - C(O) - darstellt.

Weiterer Gegenstand der vorliegenden Erfindung ist ebenfalls ein gegebenenfalls funktionalisierter 3-Oxobuttersäure-Polyolester (III"), insbesondere wie zuvor definiert,
wobei der gegebenenfalls funktionalisierte, vorzugsweise gegebenenfalls fettsäurefunktionalisierte, insbesondere gegebenenfalls C₅-C₃₄-fettsäurefunktionalisierte, bevorzugt gegebenenfalls C₈-C₃₄-fettsäurefunktionalisierte, 3-Oxobuttersäurepolyglycerinester der allgemeinen Formel (IIIc")

   R⁶O - CH₂ - CH(OR⁶) - CH₂ - O - CH₂ - CH(OR⁶) - CH₂ - OR⁶ (IIIc")
entspricht,
wobei in der allgemeinen Formel (IIIc") der Rest R⁶ jeweils unabhängig voneinander, gleich oder verschieden, darstellt: Wasserstoff, CH₃ - C(O) - CH₂ - C(O) - oder einen Rest R³, wobei der Rest R³ einen Rest vom Typ lineares (geradkettiges) oder verzweigtes, gesättigtes oder ein- oder mehrfach ungesättigtes (C₁-C₃₃-Alkyl) - C(O) -, insbesondere (C₄-C₃₃-Alkyl) - C(O) -, bevorzugt (C₇-C₃₃-Alkyl) - C(O) -, darstellt, jedoch mit der Maßgabe, dass mindestens ein Rest R⁶, insbesondere mindestens zwei Reste R⁶, keinen Wasserstoff darstellt, und mit der Maßgabe, dass mindestens ein Rest R⁶, insbesondere mindestens zwei Reste R⁶, CH₃ - C(O) - CH₂ - C(O) - darstellt.

Weiterer Gegenstand der vorliegenden Erfindung gemäß diesem Erfindungsaspekt ist entsprechend einer besonderen Ausführungsform ein Gemisch, welches mindestens zwei voneinander verschiedene gegebenenfalls funktionalisierte, vorzugweise gegebenenfalls fettsäurefunktionalisierte, insbesondere gegebenenfalls C₅-C₃₄-fettsäurefunktionalisierte, bevorzugt gegebenenfalls C₃-C₃₄-fettsäurefunktionalisierte, 3-Oxobuttersäure-Polyglycerinester, insbesondere wie zuvor definiert, umfasst.

Wiederum weiterer Gegenstand der vorliegenden Erfindung gemäß diesem Erfindungsaspekt ist entsprechend einer besonderen Ausführungsform ein Gemisch, welches mindestens drei voneinander verschiedene gegebenenfalls funktionalisierte, vorzugsweise gegebenenfalls fettsäurefunktionalisierte, insbesondere gegebenenfalls C₅-C₃₄-fettsäurefunktionalisierte, bevorzugt gegebenenfalls C₈-C₃₄-fettsäurefunktionalisierte, 3-Oxobuttersäure-Polyglycerinester, insbesondere wie zuvor definiert, umfasst.

Gemäß einer weiteren Ausführungsform ist ein weiterer Gegenstand der vorliegenden Erfindung ein 3-Oxobuttersäure-Polyglycerinester, insbesondere wie zuvor definiert,
wobei der 3-Oxobuttersäurepolyglycerinester der allgemeinen Formel (IIIb)

   R²O - CH₂ - CH(OR²) - CH₂ - [O - CH₂ - CH(OR²) - CH₂]ₚ - OR² (IIIb)
entspricht,
wobei in der allgemeinen Formel (IIIb)
   • die Variable p eine ganze Zahl von 1 bis 6, insbesondere 1 bis 4, vorzugsweise 1 oder 2, besonders bevorzugt 1, darstellt,
   • der Rest R² jeweils unabhängig voneinander, gleich oder verschieden, darstellt: Wasserstoff oder CH₃ - C(O) - CH₂ - C(O) -, jedoch mit der Maßgabe, dass mindestens ein Rest R², insbesondere mindestens zwei Reste R², keinen Wasserstoff darstellt.

Gemäß dieser Ausführungsform ist ein wiederum weiterer Gegenstand der vorliegenden Erfindung ein 3-Oxobuttersäure-Polyglycerinester, insbesondere wie zuvor definiert,
wobei der 3-Oxobuttersäurepolyglycerinester der allgemeinen Formel (IIIc)

   R²O - CH₂ - CH(OR²) - CH₂ - O - CH₂ - CH(OR²) - CH₂ - OR² (IIIc)
entspricht,
wobei in der allgemeinen Formel (IIIc) der Rest R² jeweils unabhängig voneinander, gleich oder verschieden, Wasserstoff oder CH₃ - C(O) - CH₂ - C(O) -, darstellt, jedoch mit der Maßgabe, dass mindestens ein Rest R², insbesondere mindestens zwei Reste R², keinen Wasserstoff darstellt.

Ein weiterer Gegenstand der vorliegenden Erfindung gemäß diesem Erfindungsaspekt entsprechend einer weiteren besonderen Ausführungsform ist ein Gemisch, welches mindestens zwei voneinander verschiedene 3-Oxobuttersäure-Polyglycerinester, insbesondere wie zuvor definiert, umfasst.

Insbesondere kann das Gemisch ein gewichtsbezogenes Verhältnis von monoglyceridischen Estern / di- und höherglyceridischen Estern im Bereich von 15-70 / 30-85, insbesondere im Bereich von 20-65 / 35-80, aufweisen.

Ein wiederum weiterer Gegenstand der vorliegenden Erfindung gemäß diesem Erfindungsaspekt entsprechend einer weiteren besonderen Ausführungsform ist ein Gemisch, welches mindestens drei voneinander verschiedene 3-Oxobuttersäure-Polyglycerinester, insbesondere wie zuvor definiert, umfasst.

Insbesondere kann das Gemisch ein gewichtsbezogenes Verhältnis von monoglyceridischen Estern / di- und höherglyceridischen Estern im Bereich von 15-50 / 35-60 / 1-35, insbesondere im Bereich von 20-45 / 40-55 / 3-30, aufweisen.

Gemäß einer weiteren Ausführungsform ist ein wiederum weiterer Gegenstand der vorliegenden Erfindung ein funktionalisierter 3-Oxobuttersäure-Polyglcerinester, insbesondere wie zuvor definiert,
wobei der funktionalisierte, insbesondere fettsäurefunktionalisierte, bevorzugt C₅-C₃₄-fettsäurefunktionalisierte, besonders bevorzugt C₈-C₃₄-fettsäurefunktionalisierte, 3-Oxobuttersäurepolyglycerinester der allgemeinen Formel (IIIb₁')

   R⁶O - CH₂ - CH(OR⁶) - CH₂ - [O - CH₂ - CH(OR⁶) - CH₂]ₚ - OR⁶ (IIIb₁')
entspricht,
wobei in der allgemeinen Formel (IIIb₁')
• die Variable p eine ganze Zahl von 1 bis 6, insbesondere 1 bis 4, vorzugsweise 1 oder 2, besonders bevorzugt 1, darstellt,
• der Rest R⁶ jeweils unabhängig voneinander, gleich oder verschieden, darstellt: Wasserstoff, CH₃- C(O) - CH₂ - C(O) - oder einen Rest R³, wie zuvor definiert, jedoch mit der Maßgabe, dass mindestens zwei Reste R⁶ keinen Wasserstoff darstellen, und mit der Maßgabe, dass mindestens ein Rest R⁶, insbesondere mindestens zwei Reste R⁶, CH₃- C(O) - CH₂ - C(O) - darstellt, und mit der Maßgabe, dass mindestens ein Rest R⁶, insbesondere mindestens zwei Reste R⁶, einen Rest R³, wie zuvor definiert, darstellt.

Insbesondere kann in der allgemeinen Formel (IIIb₁') der Rest R⁶ jeweils unabhängig voneinander, gleich oder verschieden, darstellen: CH₃- C(O) - CH₂ - C(O) - oder einen Rest R³, wie zuvor definiert, jedoch mit der Maßgabe, dass mindestens ein Rest R⁶, insbesondere mindestens zwei Reste R⁶, CH₃- C(O) - CH₂ - C(O) - darstellt, und mit der Maßgabe, dass mindestens ein Rest R⁶ einen Rest R³, wie zuvor definiert, darstellt.

Bei dieser besonderen Ausführungsform ist es bevorzugt, wenn in der allgemeinen Formel (IIIb₁') der Rest R⁶ jeweils unabhängig voneinander, gleich oder verschieden, keinen Wasserstoff darstellt.

Gemäß dieser besonderen Ausführungsform ist ein weiterer Gegenstand der vorliegenden Erfindung ein funktionalisierter 3-Oxobuttersäure-Polyglycerinester, insbesondere wie zuvor definiert,
wobei der funktionalisierte, insbesondere fettsäurefunktionalisierte, bevorzugt C₅-C₃₄-fettsäurefunktionalisierte, besonders bevorzugt C₈-C₃₄-fettsäurefunktionalisierte, 3-Oxobuttersäurepolyglycerinester der allgemeinen Formel (IIIc')

   R⁶O - CH₂ - CH(OR⁶) - CH₂ - O - CH₂ - CH(OR⁶) - CH₂ - OR⁶ (IIIc')
entspricht,
wobei in der allgemeinen Formel (IIIc') der Rest R⁶ jeweils unabhängig voneinander, gleich oder verschieden, darstellt: Wasserstoff, CH₃-C(O)-CH₂-C(O)- oder einen Rest R³, wie zuvor definiert, jedoch mit der Maßgabe, dass mindestens zwei Reste R⁶ keinen Wasserstoff darstellen, und mit der Maßgabe, dass mindestens ein Rest R⁶, insbesondere mindestens zwei Reste R⁶, CH₃-C(O)-CH₂-C(O)- darstellt, und mit der Maßgabe, dass mindestens ein Rest R⁶, insbesondere mindestens zwei Reste R⁶, einen Rest R³, wie zuvor definiert, darstellt.

Insbesondere kann in der allgemeinen Formel (IIIc') der Rest R⁶ jeweils unabhängig voneinander, gleich oder verschieden, darstellen: CH₃-C(O)-CH₂-C(O) - oder einen Rest R³, wie zuvor definiert, jedoch mit der Maßgabe, dass mindestens ein Rest R⁶, insbesondere mindestens zwei Reste R⁶, CH₃-C(O)-CH₂-C(O)- darstellt, und mit der Maßgabe, dass mindestens ein Rest R⁶ einen Rest R³, wie zuvor definiert, darstellt.

Bei dieser besonderen Ausführungsform ist es bevorzugt, wenn in der allgemeinen Formel (IIIc') der Rest R⁶ jeweils unabhängig voneinander, gleich oder verschieden, keinen Wasserstoff darstellt.

Weiterer Gegenstand der vorliegenden Erfindung gemäß diesem Erfindungsaspekt ist entsprechend einer weiteren besonderen Ausführungsform ein Gemisch, welches mindestens zwei voneinander verschiedene funktionalisierte, insbesondere fettsäurefunktionalisierte, bevorzugt C₅-C₃₄-fettsäurefunktionalisierte, besonders bevorzugt C₈-C₃₄-fettsäurefunktionalisierte, 3-Oxobuttersäure-Polyglycerinester, insbesondere wie zuvor definiert, umfasst.

Wiederum weiterer Gegenstand der vorliegenden Erfindung gemäß diesem Erfindungsaspekt ist entsprechend einer weiteren besonderen Ausführungsform ein Gemisch, welches mindestens drei voneinander verschiedene funktionalisierte, insbesondere fettsäurefunktionalisierte, bevorzugt C₅-C₃₄-fettsäurefunktionalisierte, besonders bevorzugt C₈-C₃₄-fettsäurefunktionalisierte, 3-Oxobuttersäure-Polyglycerinester, insbesondere wie zuvor definiert, umfasst.

Wie die Anmelderin überraschend herausgefunden hat, eignet sich der oder die nach dem erfindungsgemäßen Herstellungsverfahren erhältliche bzw. erfindungsgemäße gegebenenfalls funktionalisierte 3-Oxobuttersäure-Polyglycerinester, wie zuvor definiert, insbesondere als Präkursor bzw. Metabolit der Ketokörper Acetoacetat (Acetatacetat) und letztlich 3-Hydroxybuttersäure bzw. deren Salzen, da dieser Präkursor bzw. Metabolit einerseits physiologisch, insbesondere im Magen/Darm-Trakt, zu Acetoacetat (Acetatacetat) und letztlich zu 3-Hydroxybuttersäure bzw. deren Salzen umgesetzt wird und andererseits gleichzeitig eine gute physiologische Kompatibilität bzw. Verträglichkeit aufweist, insbesondere im Hinblick auf Nichttoxizität und akzeptable organoleptische Eigenschaften. Insbesondere die retardierte Freisetzung des physiologisch wirksamen Stoffes (d. h. Acetoacetat und letztlich 3-Hydroxybuttersäure) im Magen/Darm-Trakt ist im medizinischen Bereich vorteilhaft, da der Wirkstoff 3-Hydroxybuttersäure so über einen längeren Zeitraum zur Verfügung gestellt werden kann und somit eine Ketosetherapie ermöglicht wird. 3-Oxobuttersäure-Polyglycerinester weisen einen stärker retardierenden Effekt bzw. Wirkeintritt als 3-Hydroxybuttersäure-Polyolester auf, wodurch der Zeitraum der Wirkstofffreigabe in einer entsprechenden Therapie im Vergleich zur Verwendung von 3-Hydroxybuttersäure-Polyolestern weiterführend verlängert ist.

Darüber hinaus ist der oder die nach dem erfindungsgemäßen Herstellungsverfahren erhältliche bzw. erfindungsgemäße gegebenenfalls funktionalisierte 3-Oxobuttersäure-Polyglycerinester, wie zuvor definiert, ohne Weiteres auf synthetischem Wege auch in großtechnischem Maßstab zugänglich bzw. verfügbar, und zwar auch mit der erforderlichen pharmazeutischen bzw. pharmakologischen Qualität.

Der oder die nach dem erfindungsgemäßen Herstellungsverfahren erhältliche bzw. erfindungsgemäße gegebenenfalls funktionalisierte 3-Oxobuttersäure-Polyglycerinester, wie zuvor definiert, stellt somit ein effizientes pharmakologisches Wirkstoff-Target im Rahmen einer Ketokörper-Therapie des menschlichen oder tierischen Körpers dar.

Nachfolgend werden noch die übrigen Erfindungsaspekte weiterführende erläutert und im Detail beschrieben.

Gleichermaßen betrifft die vorliegende Erfindung - gemäß einem **vierten** Aspekt der vorliegenden Erfindung - eine pharmazeutische Zusammensetzung, insbesondere ein Arzneimittel oder Medikament, welche(s) einen oder mehrere nach dem erfindungsgemäßen Herstellungsverfahren erhältliche und/oder erfindungsgemäße gegebenenfalls funktionalisierte 3-Oxobuttersäure-Polyglycerinester, wie zuvor definiert, umfasst.

Insbesondere betrifft die vorliegende Erfindung gemäß diesem Erfindungsaspekt eine pharmazeutische Zusammensetzung zur prophylaktischen und/oder therapeutischen Behandlung bzw. zur Verwendung bei der prophylaktischen und/oder therapeutischen Behandlung von Erkrankungen des menschlichen oder tierischen Körpers. Hierbei kann es sich insbesondere Erkrankungen im Zusammenhang mit einer Störung des Energiestoffwechsels, insbesondere Ketokörperstoffwechsels, wie insbesondere Schädel-Hirn-Trauma, Schlaganfall, Hypoxien, kardiovaskuläre Erkrankungen wie Myokardinfarkt, Refeeding-Syndrom, Anorexien, Epilepsie, neurodegenerative Erkrankungen wie Demenz, Morbus Alzheimer, Morbus Parkinson, Multiple Sklerose und Amyotrophe Lateralsklerose, Fettstoffwechselerkrankungen wie Glucosetransporter-Defekt (GLUT1-Defekt), VL-FAOD und Mitochondriopathien wie mitochondrialer Thiolase-Defekt, Chorea Huntington, Krebserkrankungen wie T-Zell-Lymphome, Astrozytome und Glioblastome, HIV, rheumatische Erkrankungen wie rheumatoide Arthritis und Arthritis urica, Erkrankungen des Gastrointestinaltrakts wie chronisch entzündliche Darmerkrankungen, insbesondere Colitis ulcerosa und Morbus Crohn, lyosomale Speicherkrankheiten wie Sphingolipidosen, insbesondere Niemann-Pick-Erkrankung, Diabetes mellitus und Auswirkungen oder Nebenwirkungen von Chemotherapien, handeln.

Darüber hinaus eignet sich der nach dem erfindungsgemäßen Herstellungsverfahren erhältliche und/oder erfindungsgemäße gegebenenfalls funktionalisierte 3-Oxobuttersäure-Polyglycerinester, wie zuvor definiert, zur prophylaktischen und/oder therapeutischen Behandlung bzw. zur Verwendung bei der prophylaktischen und/oder therapeutischen Behandlung von Erkrankungen des menschlichen oder tierischen Körpers, insbesondere Erkrankungen im Zusammenhang mit einer Störung des Energiestoffwechsels, insbesondere Ketokörperstoffwechsels, wie insbesondere Schädel-Hirn-Trauma, Schlaganfall, Hypoxien, kardiovaskuläre Erkrankungen wie Myokardinfarkt, Refeeding-Syndrom, Anorexien, Epilepsie, neurodegenerative Erkrankungen wie Demenz, Morbus Alzheimer, Morbus Parkinson, Multiple Sklerose und Amyotrophe Lateralsklerose, Fettstoffwechselerkrankungen wie Glucosetransporter-Defekt (GLUT1-Defekt), VL-FAOD und Mitochondriopathien wie mitochondrialer Thiolase-Defekt, Chorea Huntington, Krebserkrankungen wie T-Zell-Lymphome, Astrozytome und Glioblastome, HIV, rheumatische Erkrankungen wie rheumatoide Arthritis und Arthritis urica, Erkrankungen des Gastrointestinaltrakts wie chronisch entzündliche Darmerkrankungen, insbesondere Colitis ulcerosa und Morbus Crohn, lyosomale Speicherkrankheiten wie Sphingolipidosen, insbesondere Niemann-Pick-Erkrankung, Diabetes mellitus und Auswirkungen oder Nebenwirkungen von Chemotherapien.

Des Weiteren kann die Verwendung ein oder mehrere nach dem erfindungsgemäßen Herstellungsverfahren erhältliche bzw. erfindungsgemäße gegebenenfalls funktionalisierter 3-Oxobuttersäure-Polyglycerinester, wie zuvor definiert, zur prophylaktischen und/oder therapeutischen Behandlung bzw. zur Herstellung eines Arzneimittels zur prophylaktischen und/oder therapeutischen Behandlung von Erkrankungen des menschlichen oder tierischen Körpers, insbesondere Erkrankungen im Zusammenhang mit einer Störung des Energiestoffwechsels, insbesondere Ketokörperstoffwechsels, wie insbesondere Schädel-Hirn-Trauma, Schlaganfall, Hypoxien, kardiovaskuläre Erkrankungen wie Myokardinfarkt, Refeeding-Syndrom, Anorexien, Epilepsie, neurodegenerative Erkrankungen wie Demenz, Morbus Alzheimer, Morbus Parkinson, Multiple Sklerose und Amyotrophe Lateralsklerose, Fettstoffwechselerkrankungen wie Glucosetransporter-Defekt (GLUT1-Defekt), VL-FAOD und Mitochondriopathien wie mitochondrialer Thiolase-Defekt, Chorea Huntington, Krebserkrankungen wie T-Zell-Lymphome, Astrozytome und Glioblastome, HIV, rheumatische Erkrankungen wie rheumatoide Arthritis und Arthritis urica, Erkrankungen des Gastrointestinaltrakts wie chronisch entzündliche Darmerkrankungen, insbesondere Colitis ulcerosa und Morbus Crohn, lyosomale Speicherkrankheiten wie Sphingolipidosen, insbesondere Niemann-Pick-Erkrankung, Diabetes mellitus und Auswirkungen oder Nebenwirkungen von Chemotherapien, vorgesehen sein.

Darüber hinaus kann die Verwendung eines oder mehrerer nach dem erfindungsgemäßen Herstellungsverfahren erhältlichen bzw. erfindungsgemäßen gegebenenfalls funktionalisierten 3-Oxobuttersäure-Polyglycerinester, wie zuvor definiert, zur prophylaktischen und/oder therapeutischen Behandlung bzw. zur Herstellung eines Arzneimittels zur prophylaktischen und/oder therapeutischen Behandlung bzw. zur Anwendung von/bei katabolen Stoffwechsellagen, wie Hunger, Diäten oder kohlenhydratarmer Ernährung, vorgesehen sein.

Des Weiteren betrifft die vorliegende Erfindung - gemäß einem **fünften** Aspekt der vorliegenden Erfindung - ein Nahrungsmittel und/oder Lebensmittelerzeugnis, welches einen oder mehrere nach dem erfindungsgemäßen Herstellungsverfahren bzw. erfindungsgemäßen gegebenenfalls funktionalisierte 3-Oxobuttersäure-Polyglycerinester, wie zuvor definiert, umfasst.

Gemäß einer besonderen Ausführungsform kann das Nahrungsmittel- und/oder Lebensmittelerzeugnis insbesondere ein Nahrungsergänzungsmittel, ein funktionelles Lebensmittel (*Functional Food*), ein *Novel Food,* ein Lebensmittelzusatzstoff, ein Nahrungszusatz, ein diätetisches Lebensmittel, ein Power-Snack, ein Appetitzügler oder ein Kraft- und/oder Ausdauersport-Supplement sein.

Schließlich kann die Verwendung eines oder mehrerer nach dem erfindungsgemäßen Herstellungsverfahren erhältlichen bzw. erfindungsgemäßen gegebenenfalls funktionalisierten 3-Oxobuttersäure-Polyglycerinester, wie zuvor definiert, in einem Nahrungsmittel- und/oder Lebensmittelerzeugnis, vorgesehen sein.

Dabei kann das Nahrungsmittel- und/oder Lebensmittelerzeugnis ein Nahrungsergänzungsmittel, ein funktionelles Lebensmittel (*Functional Food*)*,* ein *Novel Food,* ein Lebensmittelzusatzstoff, ein Nahrungszusatz, ein diätetisches Lebensmittel, ein Power-Snack, ein Appetitzügler oder ein Kraft- und/oder Ausdauersport-Supplement sein.

Weitere Ausgestaltungen, Abwandlungen und Variationen der vorliegenden Erfindung sind für den Fachmann beim Lesen der Beschreibung ohne Weiteres erkennbar oder realisierbar, ohne dass er dabei den Rahmen der vorliegenden Erfindung verlässt.

Die vorliegende Erfindung wir anhand der nachfolgenden Ausführungsbeispiele veranschaulicht, welche die vorliegende Erfindung jedoch keinesfalls beschränken sollen, sondern leidglich die beispielhafte und nichtlimitierende Durchführungsweise und Ausgestaltung der vorliegenden Erfindung erläutern sollen.

### AUSFÜHRUNGSBEISPIELE:

### Verwendete Abkürzungen

- PG(2) = Diglycerin: HO-CH₂-CH(OH)-CH₂-O-CH₂-CH(OH)-CH₂-OH
- PG(3) = Polyglycerin: HO-CH₂-CH(OH)-CH₂-[O-CH₂-CH(OH)-CH₂]₂-OH

### Herstellungsbeispiele

Das erfindungsgemäße Herstellungsverfahren wird anhand der nachfolgenden Ausführungsbeispiele veranschaulicht. Das diesbezügliche allgemeine Reaktionsschema hierzu ist im allgemeinen Beschreibungsteil dargestellt und erläutert.

### Herstellung von 3-Oxobuttersäure-Diglycerinester-Gemischen

In einem 100-ml-Mehrhalskolben mit Dephlegmator (Partialkondensator) und Destillationsbrücke werden 25 g 3-Oxobuttersäureethylester (Ethylacetoacetat bzw. Acetessigester) und 6,5 g Diglycerin vorgelegt.

Bei einer Temperatur von 50 °C werden 0,35 g immobilisiertes Enzym (CALB-Lipase auf Polymerträger, abgeleitet von *Candida antarctica,* z. B. Novozym^{®} 435) zugegeben. Das Reaktionsgemisch wird bei 50 °C für 24 h gerührt. Anschließend wir das Enzym abfiltriert und der überschüssige 3-Oxobuttersäureethylester unter Vakuum abdestilliert.

Das erhaltene Reaktionsprodukt ist ein 3-Oxobuttersäure-Diglycerin-Ester und besteht nach analytischer Untersuchung aus folgender Zusammensetzung: Mono-3-Oxobuttersäure-Diglycerinester 45 %, Di-3-Oxobuttersäure-Diglycerinester 48 %, Tri-3-Oxobuttersäure-Diglycerinester 7 %. Die Charakterisierung erfolgt mittels GC, GPC und GC-MS.

Im Rahmen der Aufreinigung werden Edukte und Reaktionsnebenprodukte entfernt, so dass ein Reingemisch erhalten wird. Ein Teil des Gemischs wird einer Auftrennung mittels Chromatographie unterzogen, so dass die verschiedenen Diglycerinester jeweils als Reinstoffe erhalten werden (d. h. jeweils reiner Mono-3-Oxobuttersäure-Diglycerinester, reiner Di-3-Oxobuttersäure-Diglycerinester und reiner Tri-3-Oxobuttersäure-Diglycerinester). Ein anderer Teil des Gemischs wird einer Auftrennung mittels fraktionierter Destillation unterzogen.

### Weitere Herstellung von 3-Oxobuttersäure-Diglycerinester-Gemischen

In einem 250-ml-Mehrhalskolben mit Dephlegmator (Partialkondensator) und Destillationsbrücke werden 106 g 3-Oxobuttersäureethylester (Ethyl-Acetoacetat bzw. Acetessigester) und 29 g Diglycerin vorgelegt.

Bei einer Temperatur von 100 °C werden 1,4 g 30 %-ige methanolische NaOMe-Lösung unter Rühren zugegeben. Das bei der Reaktion gebildete Ethanol wird kontinuierlich abdestilliert. Nach einer Reaktionszeit von 5 h wird das Reaktionsgemisch abgekühlt und mit NaCl-Lösung gewaschen. Die Rohestermischung wird anschließend getrocknet und der überschüssige 3-Oxobuttersäure-ethylester unter Vakuum abdestilliert.

Das Reaktionsprodukt ist ein 3-Oxobuttersäure-Diglycerin-Ester mit folgender Zusammensetzung: Mono-3-Oxobuttersäure-Diglycerinester 26 %, Di-3-Oxobuttersäure-Diglycerinester 51 %, Tri-3-Oxobuttersäure-Diglycerinester 22 %, Tetra-3-Oxobuttersäure-Diglycerinester 1%. Die Charakterisierung erfolgt mittels GC, GPC und GC-MS.

Im Rahmen der Aufreinigung werden Edukte und Reaktionsnebenprodukte entfernt, so dass ein Reingemisch erhalten wird. Ein Teil des Gemischs wird einer Auftrennung mittels Chromatographie unterzogen, so dass die verschiedenen Diglycerinester jeweils als Reinstoffe erhalten werden (d. h. jeweils reiner Mono-3-Oxobuttersäure-Diglycerinester, reiner Di-3-Oxobuttersäure-Diglycerinester, reiner Tri-3-Oxobuttersäure-Diglycerinester etc.). Ein anderer Teil des Gemischs wird einer Auftrennung mittels fraktionierter Destillation unterzogen.

### Weitere Herstellung von 3-Oxobuttersäure-Diglycerinester-Gemischen

Die vorangehenden Versuche werden jeweils (mit Enzym und mit NaOMe als Katalysator) wiederholt, jedoch mit anderen Polyolen (nämlich mit Glycerin (nicht erfindungsgemäß), Polyglycerin PG(3) und 1,2-Pentandiol (nicht erfindungsgemäß)). Es werden vergleichbare Ergebnisse erhalten. Aufreinigung bzw. Fraktionierung erfolgen in gleicher Weise.

### Weitere Herstellungsbeispiele

Es werden verschiedene Polyol-Komponenten auf Basis mehrwertiger Alkohole (Polyole) mit 3-Oxobuttersäureethylester (Ethyl-Acetoacetat bzw. Acetessigester) enzymatisch umgesetzt.

Als Polyole werden 1,2-Pentandiol (nicht erfindungsgemäß) und Diglycerin PG(2) eingesetzt. Die jeweiligen Polyole werden bei 70 °C für 24 h mit immobilisiertem Enzym (CALB-Lipase auf Polymerträger, abgeleitet von *Candida antarctica,* z. B. Novozym^{®} 435 von der Fa. Sigma-Aldrich bzw. Merck) umgesetzt (jeweils 1 Gew.-% Enzym und jeweils 40 Mol-% Überschuss 3-Oxobuttersäureethylester). Die vorgenannten Polyole 1,2-Pentandiol und Diglycerin PG(2) werden jeweils mit dem zuvor genannten Enzym (Novozym^{®} 435) effizient zu den gewünschten Produkten umgesetzt. Es werden vergleichbare Ergebnisse zu den vorangehenden Versuchen erhalten. Aufreinigung und Trennung bzw. Fraktionierung erfolgen in gleicher Weise.

Die Versuche werden mit Natriummethanolat (NaOMe) als Katalysator anstelle des Enzyms und bei Temperaturen zwischen 100 und 120 °C wiederholt. Es werden vergleichbare Ergebnisse erhalten. Aufreinigung und Trennung bzw. Fraktionierung erfolgen in gleicher Weise.

Da insbesondere die 3-Oxobuttersäure-PG(2)-Ester ein nur wenig bitteren Geschmack aufweisen, sind vor allem diese Ester eine effiziente Produktgruppe für eine therapeutische Anwendung. Daher wird der vorangehende Versuch mit Enzym und Diglycerin PG(2) als Polyol in einem größeren Maßstab (2 bis 4 kg) durchgeführt.

Zunächst werden in einem Maßstab von 2 kg die stöchiometrischen Reaktionsbedingungen der vorangehenden Versuche angewendet (40 Mol-% Überschuss 3-Oxobuttersäureethylester, 1 Gew.-% Enzym). Nach 15 h wird ein Teil der Reaktionsmischung (ca. 200 g) für eine weitere Untersuchung entnommen. Hierbei handelt es sich um ein Mono/Di-PG(2)-Ester-Gemisch. Danach werden weitere ca. 1 kg 3-Oxobuttersäureethylester hinzugegeben. Das Ziel ist die Herstellung eines Voll-Esters. Es ist zu erkennen, dass sich nach etwa 20 bis 30 h ein konstanter Gehalt an Di-PG(2)-Ester einstellt; der Mono-PG(2)-Ester Anteil fällt ab und der Tri-PG(2)-Ester-Anteil steigt. Weitere Analysen (GPC) zeigen, dass sich auch ein Tetra-PG(2)-Ester gebildet hat.

Nach Abdestillieren von überschüssigem 3-Oxobuttersäure-Ethylester weist das zunächst erhaltene (niedrigsiedende) Mono/Di-PG(2)-Ester-Gemisch nur einen leicht bitteren Geschmack auf, während das höhere (höhersiedende) Di-/Tri-/Tetra-PG(2)-Ester-Gemisch einen etwas stärker bitteren Geschmack aufweist. Beide Gemische sind jedoch organoleptisch akzeptabel und kompatibel.

Nach weitergehender Aufreinigung unter Entfernung restlicher Edukte und Reaktionsnebenprodukte wird ein Reingemisch mit signifikant verbesserten organoleptischen Eigenschaften erhalten.

### Funktionalisierungsversuche

1. Herstellung des Anhydrids
   In einem 2.000-ml-Mehrhalskolben mit Dephlegmator (Partialkondensator) und Destillationsbrücke werden 860 g Heptansäure vorgelegt und bei 90 °C werden 445 g Essigsäureanhydrid unter Rühren zugetropft. Das Reaktionsgemisch wird bei 130 °C unter Rückfluss für 6 h gerührt. Anschließend wird die entstandene Essigsäure, sowie das überschüssige Essigsäureanhydrid unter Vakuum abdestilliert. Es wird ein Heptansäure/Heptansäureanhydrid-Gemisch mit folgender Zusammensetzung erhalten: 15 % Heptansäure, 85 % Heptansäureanhydrid. Die Charakterisierung erfolgt mittels GC, GPC und GC-MS.
2. Funktionalisierung
   In einem 100-ml-Mehrhalskolben mit Dephlegmator (Partialkondensator) und Destillationsbrücke werden 25 g des Heptansäureanhydrids und 5 g eines erfindungsgemäß hergestellten 3-Oxobuttersäure-Mono-, Di-, Tri-, Tetra-Diglycerin-Gemischs vorgelegt. Das Reaktionsgemisch wird bei 70 °C für 24 h gerührt. Anschließend werden das überschüssige Heptansäureanhydrid und die entstandene Heptansäure mittels einer Kurzweg-Destillation abdestilliert. Es wird ein 3-Oxobuttersäure-Heptansäure-Diglycerinester-Gemisch erhalten.

Vergleichbare Funktionalisierungsversuche werden auch mit Fettsäuren und alternativ Fettsäureanhydriden und wiederum alternativ Fettsäureestern (jeweils mit Eicosapentaensäure/Docosahexaensäure-Gemisch und deren Anhydriden und Estern sowie mit Ölsäure und deren Anhydrid und Ester) durchgeführt und führen jeweils zu analogen Ergebnissen (d. h. Veresterung der freien OH-Gruppen), wie durch entsprechende Analytik bestätigt.

Die Versuche zeigen, dass die beabsichtigte Funktionalisierung durch Umsetzung mit Carbonsäureanhydriden zu den gewünschten Produkten führt (d. h. Veresterung der freien OH-Gruppen), wie durch entsprechende Analytik bestätigt.

### Weitere Synthesen funktionalisierter 3-Oxobuttersäurepolyolester

Als Polyole werden 1,2-Pentandiol (nicht erfindungsgemäß) und Diglycerin PG(2) eingesetzt. Die jeweiligen Polyole werden zunächst mit Natriummethanolat (NaOMe) als Katalysator und bei Temperaturen zwischen 100 und 120 °C mit Fettsäuren und alternativ Fettsäureanhydriden und wiederum alternativ Fettsäureestern (jeweils mit Eicosapentaensäure- und Docosahexaensäure und deren Anhydriden und Estern) umgesetzt; es resultieren die entsprechenden fettsäureveresterten Polyole, welche in einem zweiten, nachfolgenden Verfahrensschritt weiter mit 3-Oxobuttersäureethylester umgesetzt werden. Es resultieren die entsprechenden 3-Oxobuttersäure/Fettsäure-Polyolester-Gemische. Vergleichbare Ergebnisse werden bei umgekehrter Vorgehensweise erhalten (d. h. zunächst Umsetzung der Polyole mit 3-Oxobuttersäureethylester, gefolgt von einer weiteren Umsetzung mit den vorgenannten Fettsäuren bzw. alternativ deren Anhydriden und Estern).

### Physiologische Anwendungsversuche: in-vitro-Verdauversuche Verdauversuche (Spalt- bzw. Spaltungsversuche) von erfindungsgemäßen 3-Oxobuttersäure-PG(2)-Ester-Gemischen

Mittels Spaltungsversuchen wird gezeigt, dass erfindungsgemäß hergestellte 3-Oxobuttersäure-PG(2)-Ester bzw. deren Gemische, einschließlich der Reaktionsnebenprodukte, im menschlichen gastrointestinalen Trakt gespalten werden können.

Als Ausgangsgemisch wird einerseits ein aufgereinigtes, nach dem erfindungsgemäßen Verfahren erhaltenes Gemisch aus Mono-3-Oxobuttersäure-Diglycerinester, Di-3-Oxobuttersäure-Diglycerinester, Tri-3-Oxobuttersäure-Diglycerinester und Tetra-3-Oxobuttersäure-Diglycerinester und andererseits ein aufgereinigtes, nach dem erfindungsgemäßen Verfahren erhaltenes Gemisch aus funktionalisiertem Mono-3-Oxobuttersäure-Diglycerinester, funktionalisiertem Di-3-Oxobuttersäure-Diglycerinester und funktionalisiertem Tri-3-Oxobuttersäure-Diglycerinester eingesetzt (vgl. Produkte aus den vorstehend beschriebenen Funktionalisierungsversuchen).

Für die Spaltungsversuche unter köpernahen Bedingungen werden zwei Medien untersucht:
- FaSSGF, welches den Magen simuliert
- FaSSIF, welches den Darmtrakt simuliert

Beide Medien stammen von der Firma Biorelevant^{®}, Ltd., Großbritannien. Zusätzlich wird in einigen Experimenten beiden Medien jeweils Schweine-Pankrease zugesetzt (Panzytrat^{®} 40.000, Fa. Allergan).

Die Ergebnisse der Spaltungsversuche in einem FaSSGF- bzw. FaSSIF-Medium mit Panzytrat^{®} und ohne Panzytrat^{®} (jeweils 35 °C, 24 h) zeigen, dass die Proben unter FaSSGF-Bedingungen mit Panzytrat^{®} und ohne Panzytrat^{®} hydrolysieren; dies liegt hauptsächlich am niedrigen pH-Wert (pH = 1,6) des Mediums. Bei FaSSIF-Bedingungen findet eine geringere Umsetzung unter Verwendung von Panzytrat^{®} statt.

Der Versuch belegt, dass das erfindungsgemäße Ausgangsgemisch einen geeigneten physiologischen Präkursor für die Ketokörper Acetoacetat und letztendlich 3-Hydroxybuttersäure zur Verwendung in den entsprechenden Ketokörpertherapien darstellt.

### Weitere Verdauversuche (Spaltungsversuche) von erfindungsgemäßen 3-Oxobuttersäure-PG(2)-Ester-Gemischen

### Spaltungsversuche mit Pankreatin

2 g eines wie zuvor beschrieben hergestellten Gemischs auf Basis von Mono-3-Oxobuttersäure-Diglycerinester, Di-3-Oxobuttersäure-Diglycerinester, Tri-3-Oxobuttersäure-Diglycerinester und Tetra-3-Oxobuttersäure-Diglycerinester werden in 50 g Wasser gelöst und mit 0,5 g (1 Gew.-%) Pankreatin versetzt. Das Pankreatin wird in Form des kommerziell verfügbaren Produkts Panzytrat^{®} 40.000 von der Fa. Allergan eingesetzt. Das Ganze wird auf einer Heizplatte bei 50 °C gerührt; der Reaktionsverlauf wird mittels kontinuierlicher Erfassung der Säurezahl über die Zeit ermittelt und verfolgt. Die Säurezahl steigt über den Beobachtungszeitraum an (Spaltung des 3-Oxobuttersäure-Diglycerinester-Gemischs). Der Umsatz/Zeit-Verlauf der wässrigen Spaltung des erfindungsgemäßen Estergemischs mittels Pankreatin, einschließlich Zunahme der Säurezahl über die Zeit, belegt die gewünschte Zersetzung des Eduktgemischs. Dies wird durch entsprechende Analytik bestätigt. Auch dieser Versuch belegt, dass das erfindungsgemäße Ausgangsgemisch einen geeigneten physiologischen Präkursor für die Ketokörper Acetoacetat und letztendlich 3-Hydroxybuttersäure zur Verwendung in den entsprechenden Ketokörpertherapien darstellt. Der Versuch wird jeweils anhand der einzelnen Ester in Reinform wiederholt und verifiziert. Es werden vergleichbare Ergebnisse erhalten, d. h. sowohl der Mono-3-Oxobuttersäure-Diglycerinester als auch der Di-3-Oxobuttersäure-Diglycerinester sowie auch der Tri-3-Oxobuttersäure-Diglycerinester und der Tetra-3-Oxobuttersäure-Diglycerinester werden durch Pankreatin jeweils gespalten.

### Weitere Verdauversuche (Spaltungsversuche) von weiteren erfindungsgemäßen 3-Oxobuttersäure-Polyolester-Gemischen

Darüber hinaus werden auch die übrigen erfindungsgemäß hergestellten Polyolestergemische der 3-Oxobuttersäure, wie zuvor beschrieben, in entsprechender Weise Verdauversuchen unterzogen und liefern analog Ergebnisse.

Die zuvor geschilderten Spaltungsversuche belegen, dass die Polyolester, insbesondere Polyglycerinester, der 3-Oxobuttersäure effiziente Präkursoren bzw. Metabolite für die Ketokörper Acetoacetat und letztendlich 3-Hydroxybuttersäure zur Verwendung in den entsprechenden Ketokörpertherapien darstellen, insbesondere im Hinblick auf ihre beabsichtigte Wirkung, welche zudem in physiologisch verträglicher bzw. physiologisch kompatibler Form vorliegen.

## Patentansprüche

1. Verfahren zur Herstellung von Polyglycerinestern der 3-Oxobuttersäure (beta-Oxobuttersäure, 3-Oxobutansäure),
wobei mindestens eine Verbindung der allgemeinen Formel (I)
CH₃-C(O)-CH₂-C(O)OR¹ (I)
wobei in der allgemeinen Formel (I) der Rest R¹ ein C₁-C₄-Alkyl, insbesondere Methyl oder Ethyl, bevorzugt Ethyl, darstellt,
mit mindestens einem Polyglycerin der allgemeinen Formel (IIb)
HO-CH₂-CH(OH)-CH₂-[O-CH₂-CH(OH)-CH₂]ₚ-OH (IIb)
wobei in der allgemeinen Formel (IIb) die Variable p eine ganze Zahl von 1 bis 6, insbesondere 1 bis 4, vorzugsweise 1 oder 2, besonders bevorzugt 1, darstellt,
umgesetzt wird,
wobei die Umsetzung in Abwesenheit von Lösemitteln durchgeführt wird und
wobei die Umsetzung in Gegenwart eines Katalysators durchgeführt wird,
so dass als Reaktionsprodukt ein oder mehrere 3-Oxobuttersäure-Polyglycerinester erhalten werden
wobei bei der Umsetzung gleichzeitig die Verbindung gemäß der allgemeinen Formel (IV)
R¹- OH (IV)
gebildet wird, wobei in der allgemeinen Formel (IV) der Rest R¹ ein C₁-C₄-Alkyl, insbesondere Methyl oder Ethyl, bevorzugt Ethyl, darstellt, wobei die Verbindung gemäß der allgemeinen Formel (IV) der Umsetzung kontinuierlich entzogen wird.

2. Verfahren nach Anspruch 1,
wobei die Umsetzung in Gegenwart eines Katalysators, insbesondere eines Enzyms und/oder eines metallhaltigen , sauren oder basischen Katalysators, durchgeführt wird; und/oder
wobei der Katalysators nach der Umsetzung rezykliert wird.

3. Verfahren nach Anspruch 1 oder Anspruch 2,
wobei im Reaktionsprodukt nach erfolgter Umsetzung noch vorhandene Hydroxylgruppen zumindest teilweise, vorzugsweise vollständig, funktionalisiert, insbesondere verestert, werden;
insbesondere wobei die Funktionalisierung, insbesondere Veresterung, der nach erfolgter Umsetzung noch vorhandenen Hydroxylgruppen im Reaktionsprodukt mit mindestens einem Carbonsäureanhydrid der allgemeinen Formel (V)
R³-O-R³ (V)
wobei in der allgemeinen Formel (V) der Rest R³ jeweils unabhängig voneinander, gleich oder verschieden, einen Rest vom Typ lineares (geradkettiges) oder verzweigtes, gesättigtes oder ein- oder mehrfach ungesättigtes (C₁-C₃₃-Alkyl) - C(O) -, insbesondere (C₄-C₃₃-Alkyl) - C(O) -, bevorzugt (C₇-C₃₃-Alkyl) - C(O) -, darstellt, durchgeführt wird .

4. Verfahren nach Anspruch 3,
wobei die Funktionalisierung, insbesondere Veresterung, der nach erfolgter Umsetzung noch vorhandenen Hydroxylgruppen im Reaktionsprodukt durch Reaktion mit mindestens einer Carbonsäure und/oder einem Carbonsäureester der allgemeinen Formel (VII)
R³-O-R⁴ (VII)
wobei in der allgemeinen Formel (VII)
• der Rest R³ einen Rest vom Typ lineares (geradkettiges) oder verzweigtes, gesättigtes oder ein- oder mehrfach ungesättigtes (C₁-C₃₃-Alkyl) -C(O)-, insbesondere (C₄-C₃₃-Alkyl) - C(O) -, bevorzugt (C₇-C₃₃-Alkyl) - C(O) -, darstellt,
• der Rest R⁴ Wasserstoff oder ein C₁-C₄-Alkyl, insbesondere Methyl oder Ethyl, bevorzugt Wasserstoff, darstellt,
durchgeführt wird .

5. Verfahren nach Anspruch 1 oder Anspruch 2,
wobei die in dem Reaktionsprodukt (III) durch das Verfahren gemäß einem Anspruch 1 oder Anspruch 2 eingeführten Estergruppen einer teilweisen Umesterung mittels einer Verbindung der allgemeinen Formel (VII), wie zuvor definiert, unterzogen werden .

6. Verfahren zur Herstellung von funktionalisierten, insbesondere fettsäurefunktionalisierten, Polyglycerinestern der 3-Oxobuttersäure (beta-Oxobuttersäure, 3-Oxobutansäure),
(A) wobei gemäß einer (ersten) Syntheseroute (A) zunächst in einem ersten Verfahrensschritt mindestens eine Verbindung der allgemeinen Formel (I)
CH₃-C(O)-CH₂-C(O)OR¹ (I)
wobei in der allgemeinen Formel (I) der Rest R¹ ein C₁-C₄-Alkyl, insbesondere Methyl oder Ethyl, bevorzugt Ethyl, darstellt,
mit mindestens einem Polyglycerin der allgemeinen Formel (IIb)
HO-CH₂-CH(OH)-CH₂-[O-CH₂-CH(OH)-CH₂]ₚ-OH (IIb)
wobei in der allgemeinen Formel (IIb) die Variable p eine ganze Zahl von 1 bis 6, insbesondere 1 bis 4, vorzugsweise 1 oder 2, besonders bevorzugt 1, darstellt, umgesetzt wird,
wobei die Umsetzung in Abwesenheit von Lösemitteln durchgeführt wird und wobei die Umsetzung in Gegenwart eines Katalysators durchgeführt wird,
wobei bei der Umsetzung gleichzeitig die Verbindung gemäß der allgemeinen Formel (IV)
R¹-OH (IV)
gebildet wird, wobei in der allgemeinen Formel (IV) der Rest R¹ ein C₁-C₄-Alkyl, insbesondere Methyl oder Ethyl, bevorzugt Ethyl, darstellt; wobei die Verbindung gemäß der allgemeinen Formel (IV) der Umsetzung kontinuierlich entzogen wird gefolgt von einem zweiten Verfahrensschritt, wobei der zweite Verfahrensschritt
(i) eine zumindest teilweise Funktionalisierung, insbesondere eine zumindest teilweise Veresterung, noch vorhandener Hydroxylgruppen mittels mindestens einer Fettsäure und/oder deren Ester oder Anhydrid, insbesondere mittels mindestens einer C₅-C₃₄-Fettsäure und/oder deren Ester oder Anhydrid, vorzugsweise mittels mindestens einer C₈-C₃₄-Fettsäure und/oder deren Ester oder Anhydrid, und/oder
(ii) eine teilweise Umesterung von im ersten Verfahrensschritt eingeführten Estergruppen mittels mindestens einer Fettsäure und/oder deren Ester, insbesondere mittels mindestens einer C₅-C₃₄-Fettsäure und/oder deren Ester, vorzugsweise mittels mindestens einer C₈-C₃₄-Fettsäure und/oder deren Ester,
umfasst;
oder aber
(B) wobei gemäß einer (zweiten, zu (A) alternativen) Syntheseroute (B) zunächst in einem ersten Verfahrensschritt mindestens einem Polyglycerin der allgemeinen Formel (IIb)
HO-CH₂-CH(OH)-CH₂-[O-CH₂-CH(OH)-CH₂]ₚ-OH (IIb)
wobei in der allgemeinen Formel (IIb) die Variable p eine ganze Zahl von 1 bis 6, insbesondere 1 bis 4, vorzugsweise 1 oder 2, besonders bevorzugt 1, darstellt,
mit mindestens einer Fettsäure und/oder deren Ester oder Anhydrid, insbesondere mit mindestens einer C₅-C₃₄-Fettsäure und/oder deren Ester oder Anhydrid, vorzugsweise mit mindestens einer C₈-C₃₄-Fettsäure und/oder deren Ester oder Anhydrid, umgesetzt wird,
gefolgt von einem zweiten Verfahrensschritt, wobei der zweite Verfahrensschritt
(i) eine zumindest teilweise Veresterung noch vorhandener Hydroxylgruppen mittels einer Verbindung der allgemeinen Formel (I), wie zuvor definiert, und/oder
(ii) eine teilweisen Umesterung von im ersten Verfahrensschritt eingeführten Estergruppen mittels einer Verbindung der allgemeinen Formel (I), wie zuvor definiert,
umfasst;
so dass als Reaktionsprodukt jeweils ein oder mehrere funktionalisierte, insbesondere fettsäurefunktionalisierte, vorzugsweise C₅-C₃₄-fettsäurefunktionalisierte, bevorzugt C₈-C₃₄-fettsäurefunktionalisierte, 3-Oxobuttersäure-Polyglycerinester erhalten werden.

7. Gegebenenfalls funktionalisierter 3-Oxobuttersäure-Polyglcerinester oder Gemisch von mindestens zwei voneinander verschiedenen gegebenenfalls funktionalisierten 3-Oxobuttersäure-Polyglcerinestern, erhältlich gemäß dem Verfahren nach einem der vorangehenden Ansprüche.

8. Gegebenenfalls funktionalisierter 3-Oxobuttersäure-Polyglcerinester,
wobei der gegebenenfalls funktionalisierte, vorzugsweise gegebenenfalls fettsäurefunktionalisierte, bevorzugt gegebenenfalls C₅-C₃₄-fettsäurefunktionalisierte, insbesondere gegebenenfalls C₈-C₃₄-fettsäurefunktionalisierte, 3-Oxobuttersäure-polyglycerinester der allgemeinen Formel (IIIb₁")
R⁶O-CH₂-CH(OR⁶)-CH₂-[O-CH₂-CH(OR⁶)-CH₂]ₚ-OR⁶ (IIIb₁")
entspricht,
wobei in der allgemeinen Formel (IIIb₁")
• die Variable p eine ganze Zahl von 1 bis 6, insbesondere 1 bis 4, vorzugsweise 1 oder 2, besonders bevorzugt 1, darstellt,
• der Rest R⁶ jeweils unabhängig voneinander, gleich oder verschieden, darstellt: Wasserstoff, CH₃-C(O)-CH₂-C(O)- oder einen Rest R³, wobei der Rest R³ einen Rest vom Typ lineares (geradkettiges) oder verzweigtes, gesättigtes oder ein- oder mehrfach ungesättigtes (C₁-C₃₃-Alkyl) - C(O) -, insbesondere (C₄-C₃₃-Alkyl) - C(O) -, bevorzugt (C₇-C₃₃-Alkyl) - C(O) -, darstellt, jedoch mit der Maßgabe, dass mindestens ein Rest R⁶, insbesondere mindestens zwei Reste R⁶, keinen Wasserstoff darstellt, und mit der Maßgabe, dass mindestens ein Rest R⁶, insbesondere mindestens zwei Reste R⁶, CH₃-C(O)-CH₂-C(O)- darstellt.

9. Gegebenenfalls funktionalisierter 3-Oxobuttersäure-Polyglcerinester nach Anspruch 8, wobei der gegebenenfalls funktionalisierte, vorzugsweise gegebenenfalls fettsäurefunktionalisierte, bevorzugt gegebenenfalls C₅-C₃₄-fettsäurefunktionalisierte, insbesondere gegebenenfalls C₈-C₃₄-fettsäurefunktionalisierte, 3-Oxobuttersäure-polyglycerinester der allgemeinen Formel (IIIc")
R⁶O-CH₂-CH(OR⁶) -CH₂-O-CH₂-CH(OR⁶)-CH₂-OR⁶ (IIIc")
entspricht,
wobei in der allgemeinen Formel (IIIc") der Rest R⁶ jeweils unabhängig voneinander, gleich oder verschieden, darstellt: Wasserstoff, CH₃-C(O)-CH₂-C(O)- oder einen Rest R³, wobei der Rest R³ einen Rest vom Typ lineares (geradkettiges) oder verzweigtes, gesättigtes oder ein- oder mehrfach ungesättigtes (C₁-C₃₃-Alkyl) - C(O) -, insbesondere (C₄-C₃₃-Alkyl) - C(O) -, bevorzugt (C₇-C₃₃-Alkyl) - C(O) -, darstellt, jedoch mit der Maßgabe, dass mindestens ein Rest R⁶, insbesondere mindestens zwei Reste R⁶, keinen Wasserstoff darstellt, und mit der Maßgabe, dass mindestens ein Rest R⁶, insbesondere mindestens zwei Reste R⁶, CH₃-C(O)-CH₂-C(O)- darstellt.

10. Gegebenenfalls funktionalisierter 3-Oxobuttersäure-Polyglcerinester nach Anspruch 8, wobei der gegebenenfalls funktionalisierte 3-Oxobuttersäurepolyglycerinester der allgemeinen Formel (IIIb)
R²O-CH₂-CH(OR²)-CH₂-[O-CH₂-CH(OR²)-CH₂]ₚ-OR² (IIIb)
entspricht,
wobei in der allgemeinen Formel (IIIb)
• die Variable p eine ganze Zahl von 1 bis 6, insbesondere 1 bis 4, vorzugsweise 1 oder 2, besonders bevorzugt 1, darstellt,
• der Rest R² jeweils unabhängig voneinander, gleich oder verschieden, darstellt: Wasserstoff oder CH₃-C(O)-CH₂-C(O)-, jedoch mit der Maßgabe, dass mindestens ein Rest R², insbesondere mindestens zwei Reste R², keinen Wasserstoff darstellt.

11. Gegebenenfalls funktionalisierter 3-Oxobuttersäure-Polyglcerinester nach Anspruch 8 oder Anspruch 10, 3-Oxobuttersäure-Polyolester (III) nach Anspruch 10,
wobei der gegebenenfalls funktionalisierte 3-Oxobuttersäurepolyglycerinester der allgemeinen Formel (IIIc)
R²O-CH₂-CH(OR²)-CH₂-O-CH₂-CH(OR²)-CH₂-OR² (IIIc)
entspricht,
wobei in der allgemeinen Formel (IIIc) der Rest R² jeweils unabhängig voneinander, gleich oder verschieden, Wasserstoff oder CH₃-C(O)-CH₂-C(O)-, darstellt, jedoch mit der Maßgabe, dass mindestens ein Rest R², insbesondere mindestens zwei Reste R², keinen Wasserstoff darstellt.

12. Gegebenenfalls funktionalisierter 3-Oxobuttersäure-Polyglcerinester nach Anspruch 8, wobei der gegebenenfalls funktionalisierte 3-Oxobuttersäurepolyglycerinester der allgemeinen Formel (IIIb₁')
R⁶O-CH₂-CH(OR⁶)-CH₂-[O-CH₂-CH(OR⁶)-CH₂]ₚ -OR⁶ (IIIb₁')
entspricht,
wobei in der allgemeinen Formel (IIIb₁')
• die Variable p eine ganze Zahl von 1 bis 6, insbesondere 1 bis 4, vorzugsweise 1 oder 2, besonders bevorzugt 1, darstellt,
• der Rest R⁶ jeweils unabhängig voneinander, gleich oder verschieden, darstellt: Wasserstoff, CH₃-C(O)-CH₂-C(O) - oder einen Rest R³, wie zuvor definiert, jedoch mit der Maßgabe, dass mindestens zwei Reste R⁶ keinen Wasserstoff darstellen, und mit der Maßgabe, dass mindestens ein Rest R⁶, insbesondere mindestens zwei Reste R⁶, CH₃-C(O)-CH₂-C(O)- darstellt, und mit der Maßgabe, dass mindestens ein Rest R⁶, insbesondere mindestens zwei Reste R⁶, einen Rest R³, wie zuvor definiert, darstellt.

13. Gegebenenfalls funktionalisierter 3-Oxobuttersäure-Polyglcerinester nach Anspruch 8 oder Anspruch 12,
wobei der gegebenenfalls funktionalisierte, insbesondere fettsäurefunktionalisierte, bevorzugt C₅-C₃₄-fettsäurefunktionalisierte, besonders bevorzugt C₈-C₃₄-fettsäurefunktionalisierte, 3-Oxobuttersäurepolyglycerinester der allgemeinen Formel (IIIc')
R⁶O-CH₂-CH(OR⁶)-CH₂-O-CH₂-CH(OR⁶)-CH₂-OR⁶ (IIIc')
entspricht,
wobei in der allgemeinen Formel (IIIc') der Rest R⁶ jeweils unabhängig voneinander, gleich oder verschieden, darstellt: Wasserstoff, CH₃-C(O)-CH₂-C(O)- oder einen Rest R³, wie zuvor definiert, jedoch mit der Maßgabe, dass mindestens zwei Reste R⁶ keinen Wasserstoff darstellen, und mit der Maßgabe, dass mindestens ein Rest R⁶, insbesondere mindestens zwei Reste R⁶, CH₃-C(O)-CH₂-C(O)- darstellt, und mit der Maßgabe, dass mindestens ein Rest R⁶, insbesondere mindestens zwei Reste R⁶, einen Rest R³, wie zuvor definiert, darstellt.

14. Gemisch, umfassend mindestens zwei, insbesondere mindestens drei, voneinander verschiedene gegebenenfalls funktionalisierte, vorzugweise gegebenenfalls fettsäurefunktionalisierte, bevorzugt gegebenenfalls C₅-C₃₄-fettsäurefunktionalisierte, insbesondere gegebenenfalls C₈-C₃₄-fettsäurefunktionalisierte, 3-Oxobuttersäure-Polyglycerinester, insbesondere wie zuvor definiert.

15. Pharmazeutische Zusammensetzung, insbesondere Arzneimittel oder Medikament, umfassend einen oder mehrere gegebenenfalls funktionalisierte 3-Oxobuttersäure-Polyglycerinester gemäß einem der Ansprüche 7 bis 14.

16. Pharmazeutische Zusammensetzung nach Anspruch 15 zur Verwendung bei der prophylaktischen und/oder therapeutischen Behandlung von Erkrankungen des menschlichen oder tierischen Körpers, insbesondere Erkrankungen im Zusammenhang mit einer Störung des Energiestoffwechsels, insbesondere Ketokörperstoffwechsels, wie insbesondere Schädel-Hirn-Trauma, Schlaganfall, Hypoxien, kardiovaskuläre Erkrankungen wie Myokardinfarkt, Refeeding-Syndrom, Anorexien, Epilepsie, neurodegenerative Erkrankungen wie Demenz, Morbus Alzheimer, Morbus Parkinson, Multiple Sklerose und Amyotrophe Lateralsklerose, Fettstoffwechselerkrankungen wie Glucosetransporter-Defekt (GLUT1-Defekt), VL-FAOD und Mitochondriopathien wie mitochondrialer Thiolase-Defekt, Chorea Huntington, Krebserkrankungen wie T-Zell-Lymphome, Astrozytome und Glioblastome, HIV, rheumatische Erkrankungen wie rheumatoide Arthritis und Arthritis urica, Erkrankungen des Gastrointestinaltrakts wie chronisch entzündliche Darmerkrankungen, insbesondere Colitis ulcerosa und Morbus Crohn, lyosomale Speicherkrankheiten wie Sphingolipidosen, insbesondere Niemann-Pick-Erkrankung, Diabetes mellitus und Auswirkungen oder Nebenwirkungen von Chemotherapien.

17. Nahrungsmittel- und/oder Lebensmittelerzeugnis, umfassend einen oder mehrere gegebenenfalls funktionalisierte 3-Oxobuttersäure-Polyglycerinester gemäß einem der Ansprüche 7 bis 14.

## Claims

1. Method for the preparation of polyglycerol esters of 3-oxobutyric acid (beta-oxobutyric acid, 3-oxobutanoic acid),
wherein at least one compound of the general formula (I)
CH₃-C(O)-CH₂-C(O)OR¹ (I)
wherein, in the general formula (I), the residue R¹ represents a C₁-C₄-alkyl, in particular methyl or ethyl, preferably ethyl,
with at least one polyglycerol of the general formula (IIb)
HO-CH₂-CH(OH)-CH₂-[O-CH₂-CH(OH)-CH₂]ₚ-OH (IIb)
wherein, in the general formula (IIb), the variable p represents an integer from 1 to 6, in particular from 1 to 4, preferably 1 or 2, and especially preferably 1,
is reacted,
wherein the reaction is carried out in the absence of solvents and
wherein the reaction is carried out in the presence of a catalyst,
so that one or more 3-oxobutyric acid polyglycerol esters are obtained as reaction products wherein, during the reaction, the compound according to the general formula (IV)
R¹-OH (IV)
is formed, wherein in the general formula (IV) the residue R¹ represents a C₁-C₄-alkyl, in particular methyl or ethyl, preferably ethyl, wherein the compound according to the general formula (IV) is continuously removed from the reaction.

2. Method according to claim 1,
wherein the reaction is carried out in the presence of a catalyst, in particular an enzyme and/or a metal-containing, acidic or basic catalyst; and/or
wherein the catalyst is recycled after the reaction.

3. Method according to claim 1 or claim 2,
wherein hydroxyl groups still present in the reaction product after the reaction are at least partially, preferably completely, functionalized, in particular esterified;
in particular wherein the functionalization, in particular esterification, of the hydroxyl groups still present in the reaction product after the reaction is carried out with at least one carboxylic acid anhydride of the general formula (V)
R³-O-R³ (V)
wherein, in the general formula (V), the residue R³ is, independently of one another, identical or different, a residue of the type linear (straight-chain) or branched, saturated or mono- or polyunsaturated (C₁-C₃₃-alkyl) - C(O) -, in particular (C₄-C₃₃-alkyl) - C(O) -, preferably (C₇-C₃₃-alkyl) - C(O) -.

4. Method according to claim 3,
wherein the functionalization, in particular esterification, of the hydroxyl groups still present in the reaction product after the reaction is carried out by reaction with at least one carboxylic acid and/or a carboxylic acid ester of the general formula (VII)
R³-O-R⁴ (VII)
wherein, in the general formula (VII)
• the residue R³ represents a residue of the type linear (straight-chain) or branched, saturated or mono- or polyunsaturated (C₁-C₃₃-alkyl) - C(O) -, in particular (C₄-C₃₃-alkyl) - C(O) -, preferably (C₇-C₃₃-alkyl) - C(O) -,
• the residue R⁴ represents hydrogen or a C₁-C₄-alkyl, in particular methyl or ethyl, preferably hydrogen.

5. Method according to claim 1 or claim 2,
wherein the ester groups introduced into the reaction product (III) by the method according to claim 1 or claim 2 are subjected to partial transesterification using a compound of the general formula (VII) as defined above.

6. Method for the preparation of functionalized, in particular fatty acid-functionalized, polyglycerol esters of 3-oxobutyric acid (beta-oxobutyric acid, 3-oxobutanoic acid),
(A) wherein, according to a (first) synthesis route (A), in a first step of the method at least one compound of the general formula (I)
CH₃-C(O)-CH₂-C(O)OR¹ (I)
wherein, in the general formula (I), the residue R¹ represents a C₁-C₄-alkyl, in particular methyl or ethyl, preferably ethyl,
with at least one polyglycerol of the general formula (IIb)
HO-CH₂-CH(OH)-CH₂-[O-CH₂-CH(OH)-CH₂]ₚ-OH (IIb)
wherein, in the general formula (IIb), the variable p represents an integer from 1 to 6, in particular from 1 to 4, preferably 1 or 2, and especially preferably 1,
is reacted,
wherein the reaction is carried out in the absence of solvents and
wherein the reaction is carried out in the presence of a catalyst,
wherein the compound according to the general formula (IV)
R¹-OH (IV)
is formed, wherein in the general formula (IV) the residue R¹ represents a C₁-C₄-alkyl, in particular methyl or ethyl, preferably ethyl; wherein the compound according to the general formula (IV) is continuously removed from the reaction
followed by a second step of the method, wherein the second step of the method comprises
(i) at least partial functionalization, in particular at least partial esterification, of any remaining hydroxyl groups using at least one fatty acid and/or its ester or anhydride, in particular using at least one C₅-C₃₄-fatty acid and/or its ester or anhydride, preferably by means of at least one C₈-C₃₄-fatty acid and/or its ester or anhydride, and/or
(ii) partial transesterification of ester groups introduced in the first step of the method using at least one fatty acid and/or its ester, in particular using at least one C₅-C₃₄-fatty acid and/or its ester, preferably using at least one C₈-C₃₄-fatty acid and/or its ester;
or
(B) wherein, according to a (second, alternative to (A)) synthesis route (B), in a first step of the method at least one polyglycerol of the general formula (IIb)
HO-CH₂-CH(OH)-CH₂-[O-CH₂-CH(OH)-CH₂]ₚ-OH (IIb)
wherein, in the general formula (IIb), the variable p represents an integer from 1 to 6, in particular from 1 to 4, preferably 1 or 2, and especially preferably 1,
with at least one fatty acid and/or its ester or anhydride, in particular with at least one C₅-C₃₄-fatty acid and/or its ester or anhydride, preferably with at least one C₈-C₃₄-fatty acid and/or its ester or anhydride, is reacted,
followed by a second step of the method, wherein the second step of the method comprises
(i) at least partial esterification of any remaining hydroxyl groups using a compound of the general formula (I) as defined above, and/or
(ii) partial transesterification of ester groups introduced in the first step of the method using a compound of the general formula (I) as defined above;
so that one or more functionalized, in particular fatty acid-functionalized, preferably C₅-C₃₄-fatty acid-functionalized, preferably C₈-C₃₄-fatty acid-functionalized, 3-oxobutyric acid polyglycerol esters are obtained as reaction products.

7. Optionally functionalized 3-oxobutyric acid polyglycerol ester or mixture of at least two different, optionally functionalized 3-oxobutyric acid polyglycerol esters, obtainable according to the method according to one of the preceding claims.

8. Optionally functionalized 3-oxobutyric acid polyglycerol ester,
wherein the optionally functionalized, preferably optionally fatty acid-functionalized, preferably optionally C₅-C₃₄-fatty acid-functionalized, in particular optionally C₈-C₃₄-fatty acid-functionalized, 3-oxobutyric acid polyglycerol ester corresponds to the general formula (IIIb₁")
R⁶O-CH₂-CH(OR⁶)-CH₂-[O-CH₂-CH(OR⁶)-CH₂]ₚ-OR⁶ (IIIb₁")
wherein, in the general formula (IIIb₁")
• the variable p represents an integer from 1 to 6, in particular 1 to 4, preferably 1 or 2, especially preferably 1,
• the residue R⁶ independently of one another, identically or differently, represents: hydrogen, CH₃-C(O)-CH₂-C(O) - or a residue R³, wherein the radical R³ represents a residue of the type linear (straight-chain) or branched, saturated or mono- or polyunsaturated (C₁-C₃₃-alkyl) - C(O) -, in particular (C₄-C₃₃-alkyl) - C(O) -, preferably (C₇-C₃₃-alkyl) - C(O) -, but with the proviso that at least one residue R^{6,}in particular at least two residues R⁶,does not represent hydrogen, and with the proviso that at least one residue R⁶, in particular at least two residues R⁶, represents CH₃-C(O)-CH₂-C(O)-.

9. Optionally functionalized 3-oxobutyric acid polyglycerol ester according to claim 8, wherein the optionally functionalized, preferably optionally fatty acid-functionalized, preferably optionally C₅-C₃₄-fatty acid-functionalized, in particular optionally C₈-C₃₄-fatty acid-functionalized, 3-oxobutyric acid polyglycerol ester corresponds to the general formula (IIIc")
R⁶O- CH₂-CH(OR⁶)-CH₂-O-CH₂-CH(OR⁶) -CH₂-OR⁶ (IIIc")
wherein, in the general formula (IIIc"), the residue R⁶ represents, independently of one another, the same or different: hydrogen, CH₃ - C(O) - CH₂ - C(O) - or a residue R³, wherein the residue R³ is a residue of the type linear (straight-chain) or branched, saturated or mono- or polyunsaturated (C₁-C₃₃-alkyl) - C(O) -, in particular (C₄-C₃₃-alkyl) - C(O) -, preferably (C₇-C₃₃-alkyl) - C(O) -, but with the proviso that at least one residue R⁶, in particular at least two residues R⁶, does not represent hydrogen, and with the proviso that at least one residue R⁶, in particular at least two residues R⁶, represents CH(₃₎- C(O) - CH₂- C(O)-.

10. Optionally functionalized 3-oxobutyric acid polyglycerol ester according to claim 8,
wherein the optionally functionalized 3-oxobutyric acid polyglycerol ester corresponds to the general formula (IIIb)
R²O-CH₂-CH(OR²)-CH₂-[O-CH₂-CH(OR²)-CH₂]ₚ-OR². (IIIb)
wherein, in the general formula (IIIb),
• the variable p represents an integer from 1 to 6, in particular 1 to 4, preferably 1 or 2, especially preferably 1,
• the residue R² represents, independently of one another, the same or different: hydrogen or CH₃ - C(O) - CH₂ - C(O) -, but with the proviso that at least one residue R², in particular at least two residues R², does not represent hydrogen.

11. Optionally functionalized 3-oxobutyric acid polyglycerol ester according to claim 8 or claim 10, 3-oxobutyric acid polyol ester (III) according to claim 10,
wherein the optionally functionalized 3-oxobutyric acid polyglycerol ester corresponds to the general formula (IIIc)
R²O-CH₂-CH(OR²)-CH₂-O-CH₂-CH(OR²)-CH₂-OR² (IIIc)
wherein, in the general formula (IIIc), the residue R² represents, independently of one another, the same or different, hydrogen or CH₃-C(O)-CH₂-C(O) -, but with the proviso that at least one residue R², in particular at least two residues R², does not represent hydrogen.

12. Optionally functionalized 3-oxobutyric acid polyglycerol ester according to claim 8,
wherein the optionally functionalized 3-oxobutyric acid polyglycerol ester corresponds to the general formula (IIIb₁')
R⁶O-CH₂-CH(OR⁶)-CH₂-[O-CH₂-CH(OR⁶)-CH₂]ₚ-OR⁶ (IIIb₁')
wherein, in the general formula (IIIb₁'),
• the variable p represents an integer from 1 to 6, in particular 1 to 4, preferably 1 or 2, especially preferably 1,
• the residue R⁶ represents, independently of one another, the same or different: hydrogen, CH₃ - C(O) - CH₂ - C(O) - or a residue R³ as defined above, but with the proviso that at least two residues R⁶ do not represent hydrogen, and with the proviso that at least one residue R⁶, in particular at least two residues R⁶, represents CH₃ - C(O) - CH₂ - C(O) -, and with the proviso that at least one residue R⁶, in particular at least two residues R⁶, represents a residue R³ as defined above.

13. Optionally functionalized 3-oxobutyric acid polyglycerol ester according to claim 8 or claim 12,
wherein the optionally functionalized, in particular fatty acid-functionalized, preferably C₅-C₃₄-fatty acid-functionalized, particularly preferably C₈ -C₃₄ -fatty acid-functionalized, 3-oxobutyric acid polyglycerol ester corresponds to the general formula (IIIc')
R⁶O-CH₂-CH(OR⁶)-CH₂-O-CH₂-CH(OR⁶)-CH₂-OR^{6.} (IIIc')
wherein, in the general formula (IIIc'), the residue R⁶ represents, independently of one another, the same or different: hydrogen, CH₃-C(O) - CH(2) - C(O) - or a residue R³ as defined above, but with the proviso that at least two residues R⁶ do not represent hydrogen, and with the proviso that at least one residue R⁶, in particular at least two residues R⁶, represents CH₃ - C(O) - CH₂ - C(O) -, and provided that at least one residue R⁶, in particular at least two residues R⁶, represents a residue R³ as defined above.

14. Mixture comprising at least two, in particular at least three, mutually different, optionally functionalized, preferably optionally fatty acid-functionalized, preferably optionally C₅-C₃₄-fatty acid-functionalized, in particular optionally C₈-C₃₄-fatty acid-functionalized, 3-oxobutyric acid polyglycerol esters, in particular as defined above.

15. Pharmaceutical composition, in particular a drug or medication, comprising one or more optionally functionalized 3-oxobutyric acid polyglycerol esters according to any of claims 7 to 14.

16. Pharmaceutical composition according to claim 15 for use in the prophylactic and/or therapeutic treatment of diseases of the human or animal body, in particular diseases associated with a disturbance of energy metabolism, in particular ketone body metabolism, such as in particular traumatic brain injury, stroke, hypoxia, cardiovascular diseases such as myocardial infarction, refeeding syndrome, anorexia, epilepsy, neurodegenerative diseases such as dementia, Alzheimer's disease, Parkinson's disease, multiple sclerosis, and amyotrophic lateral sclerosis, lipid metabolism disorders such as glucose transporter defect (GLUT1 defect), VL-FAOD, and mitochondriopathies such as mitochondrial thiolase defect, Huntington's disease, cancers such as T-cell lymphomas, astrocytomas and glioblastomas, HIV, rheumatic diseases such as rheumatoid arthritis and gouty arthritis, diseases of the gastrointestinal tract such as chronic inflammatory bowel diseases, in particular ulcerative colitis and Crohn's disease, lysosomal storage diseases such as sphingolipidoses, in particular Niemann-Pick disease, diabetes mellitus, and effects or side effects of chemotherapy.

17. Food and/or food product comprising one or more optionally functionalized 3-oxobutyric acid polyglycerol esters according to any of claims 7 to 14.

## Revendications

1. Procédé de préparation d'esters polyglycériques de l'acide 3-oxobutyrique (acide bêta-oxobutyrique, acide 3-oxobutanoïque),
où au moins un composé de formule générale (I)
CH₃-C(O)-CH₂-C(O)OR¹ (I)
où, dans la formule générale (I), le radical R¹ représente un groupe alkyle en C₁ à C₄, en particulier un groupe méthyle ou éthyle, de préférence un groupe éthyle,
est converti avec au moins un polyglycérol de formule générale (IIb)
HO-CH₂-CH(OH)-CH₂-[O-CH₂-CH(OH)-CH₂]ₚ-OH (IIb)
où, dans la formule générale (IIb), la variable p représente un nombre entier de 1 à 6, en particulier de 1 à 4, de préférence 1 ou 2, de manière particulièrement préférée 1,
la réaction étant effectuée en l'absence de solvants et
la réaction étant effectuée en présence d'un catalyseur,
de sorte qu'on obtient comme produit de réaction un ou plusieurs esters polyglycériques de l'acide 3-oxobutyrique
la réaction produisant simultanément le composé de formule générale (IV)
R¹- OH (IV)
étant formé, le radical R¹ dans la formule générale (IV) représentant un groupe alkyle en C₁ à C₄, en particulier un groupe méthyle ou éthyle, de préférence un groupe éthyle, le composé selon la formule générale (IV) étant retiré en continu de la réaction.

2. Procédé selon la revendication 1,
la réaction étant effectuée en présence d'un catalyseur, en particulier d'une enzyme et/ou d'un catalyseur métallique, acide ou basique; et/ou
le catalyseur étant recyclé après la réaction.

3. Procédé selon la revendication 1 ou la revendication 2,
où les groupes hydroxyles encore présents dans le produit de réaction après la réaction sont au moins partiellement, de préférence complètement, fonctionnalisés, en particulier estérifiés;
en particulier la fonctionnalisation, en particulier l'estérification, des groupes hydroxyles encore présents dans le produit de réaction après la réaction étant effectuée avec au moins un anhydride d'acide carboxylique de formule générale (V)
R³-O-R³ (V)
où, dans la formule générale (V), le radical R³ représente, indépendamment les uns des autres, un radical de type linéaire (à chaîne droite) ou ramifié, saturé ou mono- ou polyinsaturé (alkyle en C₁ -C₃₃) - C(O) -, en particulier (alkyle en C₄ -C₃₃) - C(O) -, de préférence (alkyle en C₇ -C₃₃) - C(O) -, représente.

4. Procédé selon la revendication 3,
où la fonctionnalisation, en particulier l'estérification, des groupes hydroxyle encore présents dans le produit de réaction après la réaction est effectuée par réaction avec au moins un acide carboxylique et/ou un ester d'acide carboxylique de formule générale (VII)
R³-O-R⁴ (VII)
où, dans la formule générale (VII),
• le radical R⁽³⁾ représente un radical de type linéaire (à chaîne droite) ou ramifié, saturé ou mono- ou polyinsaturé (alkyle en C₁ -C₃₃ - C(O) -, en particulier (alkyle en C₄ -C₃₃) - C(O) -, de préférence (alkyle en C₇ -C₃₃ ) - C(O) -,
• le radical R⁴ représente un atome d'hydrogène ou un groupe alkyle en C₁ -C₄, en particulier méthyle ou éthyle, de préférence un atome d'hydrogène.

5. Procédé selon la revendication 1 ou la revendication 2,
où les groupes esters introduits dans le produit de réaction (III) par le procédé selon la revendication 1 ou la revendication 2 sont soumis à une transestérification partielle au moyen d'un composé de formule générale (VII) telle que définie précédemment.

6. Procédé de préparation d'esters polyglycériques fonctionnalisés, en particulier fonctionnalisés par des acides gras, de l'acide 3-oxobutyrique (acide bêta-oxobutyrique, acide 3-oxobutanoïque),
(A) où, selon une (première) voie de synthèse (A), au moins un composé de formule générale (I)
CH₃-C(O)-CH₂-C(O)OR¹ (I)
où, dans la formule générale (I), le radical R¹ représente un groupe alkyle en C₁ à C₄, en particulier méthyle ou éthyle, de préférence éthyle,
est converti avec au moins un polyglycérol de formule générale (Ilb)
HO-CH₂-CH(OH)-CH₂-[O-CH₂-CH(OH)-CH₂]ₚ -OH (IIb)
où, dans la formule générale (IIb), la variable p représente un nombre entier de 1 à 6, en particulier de 1 à 4, de préférence 1 ou 2, de manière particulièrement préférée 1,
la réaction étant effectuée en l'absence de solvants et
la réaction étant effectuée en présence d'un catalyseur,
la réaction étant effectuée simultanément avec le composé selon la formule générale (IV)
R¹- OH (IV)
étant formé, le radical R¹ dans la formule générale (IV) représentant un groupe alkyle en C₁ à C₄, en particulier un groupe méthyle ou éthyle, de préférence un groupe éthyle; le composé selon la formule générale (IV) étant retiré en continu de la réaction
suivie d'une deuxième étape de procédé, la deuxième étape de procédé
(i) une fonctionnalisation au moins partielle, en particulier une estérification au moins partielle, des groupes hydroxyles encore présents au moyen d'au moins un acide gras et/ou son ester ou anhydride, en particulier au moyen d'au moins un acide gras en C₅ à C₃₄ et/ou de son ester ou anhydride, de préférence au moyen d'au moins un acide gras en C₈ à C₃₄ et/ou de son ester ou anhydride, et/ou
(ii) une transestérification partielle des groupes esters introduits lors de la première étape du procédé au moyen d'au moins un acide gras et/ou de ses esters, en particulier au moyen d'au moins un acide gras en C₅ -C₃₄ et/ou de ses esters, de préférence au moyen d'au moins un acide gras en C₈ -C₃₄ et/ou de ses esters;
ou bien
(B) selon une voie de synthèse (B) (alternative à (A)), où, dans une première étape du procédé, au moins un polyglycérol de formule générale (IIb)
HO-CH₂-CH(OH)-CH₂-[O-CH₂-CH(OH)-CH₂]ₚ -OH (IIb)
où, dans la formule générale (IIb), la variable p représente un nombre entier de 1 à 6, en particulier de 1 à 4, de préférence 1 ou 2, de manière particulièrement préférée 1,
est mis à réagir avec au moins un acide gras et/ou son ester ou anhydride, en particulier avec au moins un acide gras en C₅ -C₃₄ et/ou son ester ou anhydride, de préférence avec au moins un acide gras en C₈ -C₃₄ et/ou son ester ou anhydride,
suivie d'une deuxième étape de procédé, la deuxième étape de procédé
(i) une estérification au moins partielle des groupes hydroxyle encore présents au moyen d'un composé de formule générale (I) telle que définie précédemment et/ou
(ii) une transestérification partielle des groupes esters introduits lors de la première étape du procédé au moyen d'un composé de formule générale (I) telle que définie précédemment;
de sorte que le produit de réaction obtenu est un ou plusieurs esters polyglycériques d'acide 3-oxobutyrique fonctionnalisés, en particulier fonctionnalisés par des acides gras, de préférence fonctionnalisés par des acides gras en C₅ à C₃₄, de préférence en C₈ à C₃₄.

7. Ester polyglycérique d'acide 3-oxobutyrique éventuellement fonctionnalisé ou mélange d'au moins deux esters polyglycériques d'acide 3-oxobutyrique éventuellement fonctionnalisés différents les uns des autres, pouvant être obtenus selon le procédé selon l'une des revendications précédentes.

8. Ester polyglycérique d'acide 3-oxobutyrique éventuellement fonctionnalisé,
où l'ester de polyglycérol d'acide 3-oxobutyrique éventuellement fonctionnalisé, de préférence éventuellement fonctionnalisé par un acide gras, de préférence éventuellement fonctionnalisé par un acide gras en C₅ - C₃₄, correspond à la formule générale (IIIb₁")
R⁶O-CH₂-CH(OR⁶)-CH₂-[O-CH₂-CH(OR⁶)-CH₂]ₚ-OR⁶ (IIIb₁")
où, dans la formule générale (IIIb₁")
• la variable p représente un nombre entier compris entre 1 et 6, en particulier entre 1 et 4, de préférence 1 ou 2, et de manière particulièrement préférée 1,
• le radical R⁶ représente, indépendamment les uns des autres, de manière identique ou différente: un atome d'hydrogène, CH₃-C(O)-CH₂-C(O)- ou un radical R³, le radical R³ représentant un radical de type linéaire (à chaîne droite) ou ramifié, saturé ou mono- ou polyinsaturé (alkyle en C₁ -C₃₃ ) - C(O) -, en particulier (alkyle en C₄ - C₃₃) - C(O) -, de préférence (alkyle en C₇ -C₃₃) - C(O) -, à condition toutefois qu'au moins un radical R(⁶⁾, en particulier au moins deux radicaux R(^{6) ,}ne représente pas un atome d'hydrogène, et à condition qu'au moins un radical R(⁶⁾, en particulier au moins deux radicaux R(⁶⁾, représente CH₃ - C(O) - CH(₂₎ - C(O) -.

9. Ester de polyglycérol de l'acide 3-oxobutyrique éventuellement fonctionnalisé selon la revendication 8,
où l'ester de polyglycérol d'acide 3-oxobutyrique éventuellement fonctionnalisé, de préférence éventuellement fonctionnalisé par un acide gras, de préférence éventuellement fonctionnalisé par un acide gras en C₅ - C₃₄, correspond à la formule générale (IIIc")
R⁶O-CH₂-CH(OR⁶)-CH₂-O-CH₂-CH(OR⁶)-CH₂-OR⁶ (IIIc")
où, dans la formule générale (IIIc"), le radical R⁶ représente, indépendamment les uns des autres, de manière identique ou différente : un atome d'hydrogène, CH₃ - C(O) - CH₂ -C(O) - ou un radical R³, le radical R³ représentant un radical de type linéaire (à chaîne droite) ou ramifié, saturé ou mono- ou polyinsaturé (alkyle en C₁ -C₃₃) - C(O) -, en particulier (alkyle en C₄ -C₃₃) - C(O) -, de préférence (alkyle en C₇ -C₃₃) - C(O) -, à condition toutefois qu'au moins un radical R⁶ , en particulier au moins deux radicaux R⁶ , ne représente pas un atome d'hydrogène, et à condition qu'au moins un radical R(^{6) ,}en particulier au moins deux radicaux R⁶, représente CH₃ - C(O) - CH(₂₎ - C(O) -.

10. Ester polyglycérol de l'acide 3-oxobutyrique éventuellement fonctionnalisé selon la revendication 8,
où l'ester polyglycérique d'acide 3-oxobutyrique éventuellement fonctionnalisé répond à la formule générale (IIIb)
R²O-CH₂-CH(OR²)-CH₂-[O-CH₂-CH(OR²)-CH₂]ₚ-OR² (IIIb)
dans la formule générale (IIIb)
• la variable p représente un nombre entier de 1 à 6, en particulier de 1 à 4, de préférence 1 ou 2, de manière particulièrement préférée 1,
• le radical R² représente, indépendamment les uns des autres, de manière identique ou différente: un atome d'hydrogène ou CH₃ - C(O) - CH₂ - C(O)-, à condition toutefois qu'au moins un radical R², en particulier au moins deux radicaux R², ne représente pas un atome d'hydrogène.

11. Ester polyglycérique d'acide 3-oxobutyrique éventuellement fonctionnalisé selon la revendication 8 ou la revendication 10, ester polyolique d'acide 3-oxobutyrique (III) selon la revendication 10,
l'ester polyglycérique d'acide 3-oxobutyrique éventuellement fonctionnalisé répondant à la formule générale (IIIc)
R²O-CH₂-CH(OR²)-CH₂-O-CH₂-CH(OR²)-CH₂-OR² (IIIc)
dans la formule générale (IIIc), le radical R² représente, indépendamment les uns des autres, de manière identique ou différente, un atome d'hydrogène ou CH₃ - C(O) - CH₂ -C(O) -, à condition toutefois qu'au moins un radical R², en particulier au moins deux radicaux R², ne représente pas un atome d'hydrogène.

12. Ester polyglycérol de l'acide 3-oxobutyrique éventuellement fonctionnalisé selon la revendication 8,
où l'ester polyglycérique d'acide 3-oxobutyrique éventuellement fonctionnalisé correspond à la formule générale (IIIb₁')
R⁶O-CH₂-CH(OR⁶)-CH₂-[O-CH₂-CH(OR⁶)-CH₂]ₚ-OR⁶ (IIIb₁')
dans la formule générale (IIIb₁')
• la variable p représente un nombre entier compris entre 1 et 6, en particulier entre 1 et 4, de préférence 1 ou 2, et de manière particulièrement préférée 1,
• le radical R⁶ représente, indépendamment les uns des autres, de manière identique ou différente: un atome d'hydrogène, CH₃ - C(O) - CH₂ - C(O) - ou un radical R³ tel que défini précédemment, à condition toutefois qu'au moins deux radicaux R⁶ ne représentent pas un atome d'hydrogène, et à condition qu'au moins un radical R⁶, en particulier au moins deux radicaux R⁶, représente CH₃ - C(O) - CH₂ - C(O)-, et à condition qu'au moins un radical R⁶, en particulier au moins deux radicaux R⁶, représente un radical R³ tel que défini précédemment.

13. Ester de polyglycérol de l'acide 3-oxobutyrique éventuellement fonctionnalisé selon la revendication 8 ou la revendication 12,
où l'ester de polyglycérol d'acide 3-oxobutyrique éventuellement fonctionnalisé, de préférence éventuellement fonctionnalisé par un acide gras, de préférence éventuellement fonctionnalisé par un acide gras en C₅ - C₃₄ , correspond à la formule générale (IIIc')
R⁶O-CH₂-CH(OR⁶)-CH₂-O-CH₂-CH(OR⁶)-CH₂-OR⁶ (IIIc')
dans la formule générale (IIIc'), le radical R⁶ représente, indépendamment les uns des autres, de manière identique ou différente: un atome d'hydrogène, CH₃- C(O) - CH(2)-C(O) - ou un radical R³ tel que défini précédemment, à condition toutefois qu'au moins deux radicaux R⁶ ne représentent pas un atome d'hydrogène, et à condition qu'au moins un radical R⁶ en particulier au moins deux radicaux R⁶, représentent CH_{3 -} C(O) - CH₂ -C(O) -, et à condition qu'au moins un radical R⁶, en particulier au moins deux radicaux R⁶, représente un radical R³ tel que défini précédemment.

14. Mélange comprenant au moins deux, en particulier au moins trois, différents esters de polyglycérol de l'acide 3-oxobutyrique, éventuellement fonctionnalisés, de préférence éventuellement fonctionnalisés par un acide gras, de préférence éventuellement fonctionnalisés par un acide gras en C₅ -C₃₄ , en particulier tels que définis précédemment.

15. Composition pharmaceutique, en particulier médicament ou remède, comprenant un ou plusieurs esters polyglycériques d'acide 3-oxobutyrique éventuellement fonctionnalisés selon l'une des revendications 7 à 14.

16. Composition pharmaceutique selon la revendication 15 destinée à être utilisée dans le traitement prophylactique et/ou thérapeutique de maladies du corps humain ou animal, en particulier de maladies liées à un trouble du métabolisme énergétique, en particulier du métabolisme des corps cétoniques, telles que notamment les traumatismes crâniens, les accidents vasculaires cérébraux, les hypoxies, maladies cardiovasculaires telles que l'infarctus du myocarde, le syndrome de realimentation, les anorexies, l'épilepsie, les maladies neurodégénératives telles que la démence, la maladie d'Alzheimer, la maladie de Parkinson, la sclérose en plaques et la sclérose latérale amyotrophique, les maladies du métabolisme lipidique telles que le défaut du transporteur de glucose (défaut GLUT1), VL-FAOD et mitochondriopathies telles que le déficit en thiolase mitochondriale, la chorée de Huntington, cancers tels que les lymphomes à cellules T, astrocytomes et glioblastomes, VIH, maladies rhumatismales telles que la polyarthrite rhumatoïde et l'arthrite urique, maladies du tractus gastro-intestinal telles que les maladies inflammatoires chroniques de l'intestin, en particulier la colite ulcéreuse et la maladie de Crohn, maladies lysosomales telles que les sphingolipidoses, en particulier la maladie de Niemann-Pick, le diabète sucré et les effets ou effets secondaires des chimiothérapies.

17. Produit alimentaire et/ou denrée alimentaire comprenant un ou plusieurs esters polyglycériques d'acide 3-oxobutyrique éventuellement fonctionnalisés selon l'une des revendications 7 à 14.
